# EUROPÄISCHE PATENTSCHRIFT

(11) **EP 0 617 026 B1**
(45) Veröffentlichungstag und Bekanntmachung des Hinweises auf die Patenterteilung: **30.07.1997**
(21) Anmeldenummer: 94103880.4
(22) Anmeldetag: 14.03.1994
(51) Int. Cl.: C07D 249/14, C07D 401/12, C07D 405/12, C07D 409/12, A01N 43/653

(54) **Substituierte 1-Aryltriazolinone**
Substituted 1-aryltriazolinones
1-aryltriazolinones substitués

(30) Priorität: 26.03.1993 DE 4309966
(43) Veröffentlichungstag der Anmeldung: 28.09.1994
(73) Patentinhaber: BAYER AG, 51368 Leverkusen (DE)
(72) Erfinder: Linker, Karl-Heinz, D-51377 Leverkusen (DE); Findeisen, Kurt, Prof. Dr., D-51375 Leverkusen (DE); Haas, Wilhelm, Dr., D-50259 Pulheim (DE); Schallner, Otto, Dr., D-40789 Monheim (DE); König, Klaus, Dr., D-51519 Odenthal (DE); Santel, Hans-Joachim, Dr., D-51371 Leverkusen (DE); Dollinger, Markus, Dr., D-51381 Leverkusen (DE)

(56) Entgegenhaltungen:
- WO-A-86/04481
- DE-A- 3 131 982
- DE-A- 3 839 480
- Z. NATURFORSCHUNG, TEIL C Bd. 29C, Nr. 5/6 , 1974 Seiten 232 - 235 A.TREBST ET AL 'Herbicidal N-Alkylated Ureas and Ring closed N-Acylamides as Inhibitors of Photosystem II'

## Beschreibung

Die Erfindung betrifft neue substituierte 1-Aryltriazolinone, mehrere Verfahren zu ihrer Herstellung, mehrere neue Zwischenprodukte und ihre Verwendung als Herbizide.

Es ist bekannt, daß bestimmte substituierte Triazolinone wie beispielsweise die Verbindung 3-Methyl-4-propargyl-1-(2,5-difluor-4-cyano-phenyl)-1,2,4-triazolin-5-on herbizide Eigenschaften besitzen (vergl. z.B. DE 38 39 480).

Desweiteren werden in der WO 86/00111 1-Aryl-3-haloalkyl-4,5-dihydro-1,2,4-triazol-5(1H)-one und deren herbizide Wirksamkeit beschrieben. Die DE 31 31 982 beschreibt 4-Amino-2-aryl-1,2,4-triazol-3-one als Zwischenprodukte zur Synthese von Pflanzenschutzmitteln und pharmazeutischen Wirkstoffen.

Schließlich wird in der Zeitschrift für Naturforschung Teil C, Vol.29 (5-6) 1974, 232-5, die Einzelverbindung 3-Methyl-4-methylamino-1-phenyl-1,2,4-triazol-5-on offenbart.

Die herbizide Wirksamkeit dieser vorbekannten Verbindungen gegenüber Problemunkräutern ist jedoch ebenso wie ihre Verträglichkeit gegenüber wichtigen Kulturpflanzen nicht in allen Anwendungsgebieten völlig zufriedenstellend.

Es wurden neue substituierte 1-Aryltriazolinone der allgemeinen Formel (I), 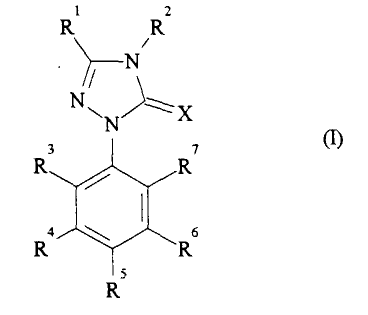 in welcher
- R¹: für Wasserstoff oder für jeweils geradkettiges oder verzweigtes Alkyl, Alkoxy, Alkylthio oder Alkylsulfonyl mit jeweils 1 bis 8 Kohlenstoffatomen steht, außerdem für geradkettiges oder verzweigtes Halogenalkyl mit 1 bis 8 Kohlenstoffatomen und 1 bis 17 gleichen oder verschiedenen Halogenatomen steht oder für Cycloalkyl mit 3 bis 8 Kohlenstoffatomen steht,
- R²: für einen Rest der Formel -NR⁸R⁹steht,
- R³, R⁶: jeweils für Wasserstoff stehen,
- R⁷: für Wasserstoff, Fluor, Chlor, Brom, Iod, Amino oder Nitro steht,
- R⁴: für Wasserstoff, Fluor, Chlor, Brom, Iod, Cyano, Nitro, oder für einen der Reste -R¹⁰, -O-R¹⁰, -S-R¹⁰, -S(O)-R¹⁰, -SO₂-R¹⁰, -SO₂-OR¹⁰, -SO₂-NR¹¹R¹⁰, -CO-OR¹⁰, -CO-NR¹¹R¹⁰, -O-SO₂-R¹⁰, -N(R¹¹)-SO₂-R¹⁰, -NR¹¹R¹⁰, -NH-P(O)(R¹¹)(OR¹⁰) oder -NH-P(O)(OR¹¹)(OR¹⁰) steht,
- R⁵: für Cyano steht und
- X: für Sauerstoff oder Schwefel steht, wobei
- R⁸: für Wasserstoff, für geradkettiges oder verzweigtes Alkyl mit 1 bis 8 Kohlenstoffatomen oder für geradkettiges oder verzweigtes Halogenalkyl mit 1 bis 8 Kohlenstoffatomen und 1 bis 17 gleichen oder verschiedenen Halogenatomen steht und außerdem für einen Rest der Formel -CO-R¹² oder für einen Rest der Formel -S(O)ₙ-R¹² steht,
- R⁹: für geradkettiges oder verzweigtes Alkyl mit 1 bis 8 Kohlenstoffatomen oder für geradkettiges oder verzweigtes Halogenalkyl mit 1 bis 8 Kohlenstoffatomen und 1 bis 17 gleichen oder verschiedenen Halogenatomen steht und außerdem für einen Rest der Formel -CO-R¹² oder für einen Rest der Formel -S(O)ₙ-R¹² steht,
- R¹⁰: für Wasserstoff steht;
- R¹⁰: außerdem für gegebenenfalls einfach oder mehrfach, gleich oder verschieden substituiertes, geradkettiges oder verzweigtes Alkyl mit 1 bis 14 Kohlenstoffatomen steht, wobei als Substituenten infrage kommen:
Halogen - insbesondere Fluor, Chlor, Brom und/oder Iod -, Cyano, Carboxyl, Carbamoyl, jeweils geradkettiges oder verzweigtes Alkoxy, Alkoxyalkoxy, Alkylthio, Alkylsulfinyl, Alkylsulfonyl, Alkoxycarbonyl, N-Alkylaminocarbonyl, N,N-Dialkylaminocarbonyl oder Alkylsulfonylaminocarbonyl mit jeweils 1 bis 8 Kohlenstoffatomen in den einzelnen Alkylteilen oder Heterocyclyl, wobei als Heterocyclylrest ein fünf- bis siebengliedriger, gegebenenfalls benzannellierter, gesättigter oder ungesättigter Heterocyclus mit 1 bis 3 gleichen oder verschiedenen Heteroatomen - insbesondere Stickstoff, Sauerstoff und/oder Schwefel - steht;
- R¹⁰: außerdem für jeweils gegebenenfalls einfach oder mehrfach, gleich oder verschieden durch Halogen - insbesondere Fluor, Chlor, Brom und/oder Iod - substituiertes Alkenyl oder Alkinyl mit jeweils 2 bis 8 Kohlenstoffatomen steht;
- R¹⁰: außerdem für gegebenenfalls einfach oder mehrfach, gleich oder verschieden durch Halogen - insbesondere Fluor, Chlor, Brom und/oder Iod - und/oder geradkettiges oder verzweigtes Alkyl mit 1 bis 4 Kohlenstoffatomen substituiertes Cycloalkyl mit 3 bis 7 Kohlenstoffatomen steht;
- R¹⁰: außerdem für jeweils gegebenenfalls im Arylteil einfach oder mehrfach, gleich oder verschieden substituiertes Arylalkyl oder Aryl mit jeweils 6 bis 10 Kohlenstoffatomen im Arylteil und gegebenenfalls 1 bis 4 Kohlenstoffatomen im geradkettigen oder verzweigten Alkylteil steht, oder für einen gegebenenfalls einfach oder mehrfach, gleich oder verschieden substituierten und/oder benzannellierten, gesättigten oder ungesättigten, fünf- bis siebengliedrigen Heterocyclylrest mit 1 bis 3 gleichen oder verschiedenen Heteroatomen - insbesondere Stickstoff, Sauerstoff und/oder Schwefel - steht, wobei als Aryl- bzw. Heterocyclylsubstituenten jeweils infrage kommen:
Halogen, Cyano, Nitro, Amino, N-Acetylamino, jeweils geradkettiges oder verzweigtes Alkyl, Alkoxy, Alkylthio, Alkylsulfinyl oder Alkylsulfonyl mit jeweils 1 bis 6 Kohlenstoffatomen, jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen, jeweils geradkettiges oder verzweigtes Alkoxycarbonyl oder Alkoximinoalkyl mit jeweils 1 bis 6 Kohlenstoffatomen in den einzelnen Alkylteilen sowie gegebenenfalls einfach oder mehrfach, gleich oder verschieden durch Halogen und/oder geradkettiges oder verzweigtes Alkyl oder Alkoxy mit jeweils 1 bis 6 Kohlenstoffatomen und/oder geradkettiges oder verzweigtes Halogenalkyl oder Halogenalkoxy mit jeweils 1 bis 6 Kohlenstoffatomen und 1 bis 13 gleichen oder verschiedenen Halogenatomen substituiertes Phenyl;
- R¹¹: für Wasserstoff steht;
- R¹¹: außerdem für gegebenenfalls einfach oder mehrfach, gleich oder verschieden substituiertes, geradkettiges oder verzweigtes Alkyl mit 1 bis 14 Kohlenstoffatomen steht, wobei als Substituenten infrage kommen:
Halogen - insbesondere Fluor, Chlor, Brom und/oder Iod -, Cyano, Carboxyl, Carbamoyl, jeweils geradkettiges oder verzweigtes Alkoxy, Alkoxyalkoxy, Alkylthio, Alkylsulfinyl, Alkylsulfonyl, Alkoxycarbonyl, N-Alkylaminocarbonyl, N,N-Dialkylaminocarbonyl oder Alkylsulfonylaminocarbonyl mit jeweils 1 bis 8 Kohlenstoffatomen in den einzelnen Alkylteilen oder Heterocyclyl, wobei als Heterocyclylrest ein fünf- bis siebengliedriger, gegebenenfalls benzannellierter, gesättigter oder ungesättigter Heterocyclus mit 1 bis 3 gleichen oder verschiedenen Heteroatomen - insbesondere Stickstoff, Sauerstoff und/oder Schwefel - steht;
- R¹¹: außerdem für jeweils gegebenenfalls einfach oder mehrfach, gleich oder verschieden durch Halogen - insbesondere Fluor, Chlor, Brom und/oder Iod - substituiertes Alkenyl oder Alkinyl mit jeweils 2 bis 8 Kohlenstoffatomen steht;
- R¹¹: außerdem für gegebenenfalls einfach oder mehrfach, gleich oder verschieden durch Halogen - insbesondere Fluor, Chlor, Brom und/oder Iod - und/oder geradkettiges oder verzweigtes Alkyl mit 1 bis 4 Kohlenstoffatomen substituiertes Cycloalkyl mit 3 bis 7 Kohlenstoffatomen steht;
- R¹¹: außerdem für jeweils gegebenenfalls im Arylteil einfach oder mehrfach, gleich oder verschieden substituiertes Arylalkyl oder Aryl mit jeweils 6 bis 10 Kohlenstoffatomen im Arylteil und gegebenenfalls 1 bis 4 Kohlenstoffatomen im geradkettigen oder verzweigten Alkylteil steht, wobei als Arylsubstituenten jeweils infrage kommen:
Halogen, Cyano, Nitro, Amino, N-Acetylamino, jeweils geradkettiges oder verzweigtes Alkyl, Alkoxy, Alkylthio, Alkylsulfinyl oder Alkylsulfonyl mit jeweils 1 bis 6 Kohlenstoffatomen, jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen, jeweils geradkettiges oder verzweigtes Alkoxycarbonyl oder Alkoximinoalkyl mit jeweils 1 bis 6 Kohlenstoffatomen in den einzelnen Alkylteilen sowie gegebenenfalls einfach oder mehrfach, gleich oder verschieden durch Halogen und/oder geradkettiges oder verzweigtes Alkyl oder Alkoxy mit jeweils 1 bis 6 Kohlenstoffatomen und/oder geradkettiges oder verzweigtes Halogenalkyl oder Halogenalkoxy mit jeweils 1 bis 6 Kohlenstoffatomen und 1 bis 13 gleichen oder verschiedenen Halogenatomen substituiertes Phenyl;
- R¹²: für gegebenenfalls einfach oder mehrfach, gleich oder verschieden substituiertes, geradkettiges oder verzweigtes Alkyl mit 1 bis 8 Kohlenstoffatomen steht, wobei als Substituenten infrage kommen:
Halogen - insbesondere Fluor, Chlor, Brom und/oder Iod -, Cycloalkyl mit 3 bis 8 Kohlenstoffatomen oder Heterocyclyl, wobei als Heterocyclylrest ein fünf- bis siebengliedriger, gegebenenfalls benzannellierter, gesättigter oder ungesättigter Heterocyclus mit 1 bis 3 gleichen oder verschiedenen Heteroatomen - insbesondere Stickstoff, Sauerstoff und/ oder Schwefel - steht;
- R¹²: außerdem für gegebenenfalls einfach oder mehrfach, gleich oder verschieden durch Halogen - insbesondere Fluor, Chlor, Brom und/oder Iod - und/oder geradkettiges oder verzweigtes Alkyl mit 1 bis 4 Kohlenstoffatomen substituiertes Cycloalkyl mit 3 bis 7 Kohlenstoffatomen steht;
- R¹²: außerdem für jeweils gegebenenfalls im Arylteil einfach oder mehrfach, gleich oder verschieden substituiertes Arylalkyl oder Aryl mit jeweils 6 bis 10 Kohlenstoffatomen im Arylteil und gegebenenfalls 1 bis 4 Kohlenstoffatomen im geradkettigen oder verzweigten Alkylteil steht oder für einen gegebenenfalls einfach oder mehrfach, gleich oder verschieden substituierten und/oder benzannellierten, gesättigten oder ungesättigten, fünf- bis siebengliedrigen Heterocyclylrest mit 1 bis 3 gleichen oder verschiedenen Heteroatomen - insbesondere Stickstoff, Sauerstoff und/oder Schwefel - steht, wobei als Aryl- bzw. Heterocyclylsubstituenten jeweils infrage kommen:
Halogen, Cyano, Nitro, Amino, N-Acetylamino, jeweils geradkettiges oder verzweigtes Alkyl, Alkoxy, Alkylthio, Alkylsulfinyl oder Alkylsulfonyl mit jeweils 1 bis 6 Kohlenstoffatomen, jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen, jeweils geradkettiges oder verzweigtes Alkoxycarbonyl oder Alkoximinoalkyl mit jeweils 1 bis 6 Kohlenstoffatomen in den einzelnen Alkylteilen sowie gegebenenfalls einfach oder mehrfach, gleich oder verschieden durch Halogen und/oder geradkettiges oder verzweigtes Alkyl oder Alkoxy mit jeweils 1 bis 6 Kohlenstoffatomen und/oder geradkettiges oder verzweigtes Halogenalkyl oder Halogenalkoxy mit jeweils 1 bis 6 Kohlenstoffatomen und 1 bis 13 gleichen oder verschiedenen Halogenatomen substituiertes Phenyl und
- n: für eine Zahl 0, 1 oder 2 steht,
gefunden.

Die Verbindungen der Formel (I) können gegebenenfalls in Abhängigkeit von der Art der Substituenten als geometrische und/oder optische Isomere oder Isomerengemische unterschiedlicher Zusammensetzung vorliegen. Sowohl die reinen Isomeren als auch die Isomerengemische werden erfindungsgemäß beansprucht.

Weiterhin wurde gefunden, daß man die neuen substituierten 1-Aryltriazolinone der allgemeinen Formel (I), 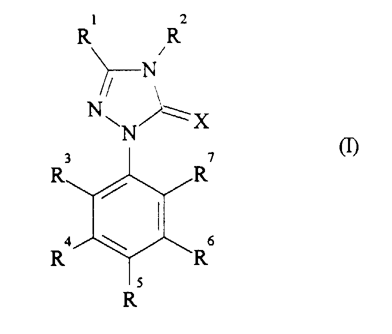 in welcher
R¹, R², R³, R⁴, R⁵, R⁶, R⁷ und X die oben genannten Bedeutungen haben,
erhält, wenn man
a) 1H-Triazolinone der Formel (II), 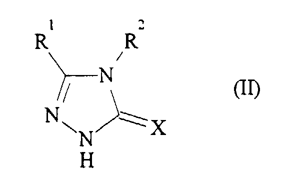 in welcher
   R¹, R² und X die oben angegebene Bedeutung haben,
   mit Halogenbenzol-Derivaten der Formel (III), 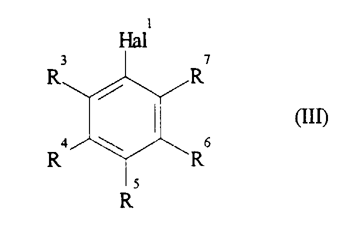 in welcher
   R³, R⁴, R⁵, R⁶ und R⁷ die oben angegebenen Bedeutungen haben und Hal¹ für Halogen steht,
   gegebenenfalls in Gegenwart eines Verdünnungsmittels und gegebenenfalls in Gegenwart eines Reaktionshilfsmittels umsetzt, oder wenn man
b) substituierte 1-Aryltriazolinone der Formel (Ia), 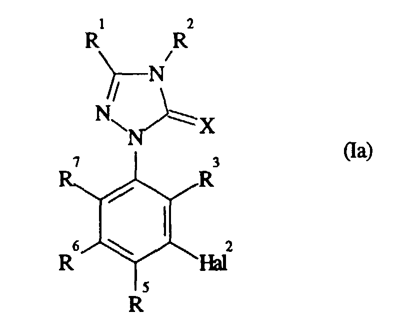 in welcher
   R¹, R², R³, R⁶, R⁵, R⁷ und X die oben angegebenen Bedeutungen haben und Hal² für Halogen steht,
   mit Nukleophilen der Formel (IV),

   H-R¹³ (IV)

   in welcher
   R¹³ für einen Rest der Formel -O-R¹⁰, -S-R¹⁰ oder -NR¹¹R¹⁰ steht, wobei
   R¹⁰ und R¹¹ die oben angegebenen Bedeutungen haben,
   gegebenenfalls in Gegenwart eines Verdünnungsmittels und gegebenenfalls in Gegenwart eines Reaktionshilfsmittels umsetzt, oder wenn man
c) substituierte Triazolinone der Formel (V), 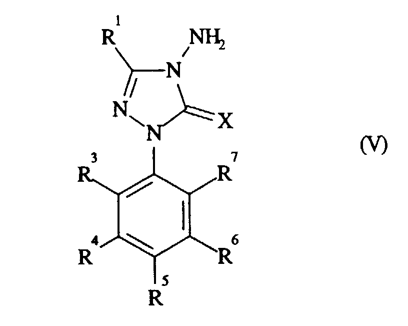 in welcher
   R¹, R³, R⁴, R⁵, R⁶, R⁷ und X die oben angegebenen Bedeutungen haben,
   mit Alkylierungs, Acylierungs- oder Sulfonylierungsmitteln der Formel (VI),

   R⁹-E (VI)

   in welcher
   - R⁹: die oben angegebene Bedeutung hat und
   - E: für eine elektronenanziehende Abgangsgruppe steht,
   gegebenenfalls in Gegenwart eines Verdünnungsmittels und gegebenenfalls in Gegenwart eines Reaktionshilfsmittels umsetzt, oder wenn man
d) 4-Alkylidenimino-triazolinone der Formel (VII), 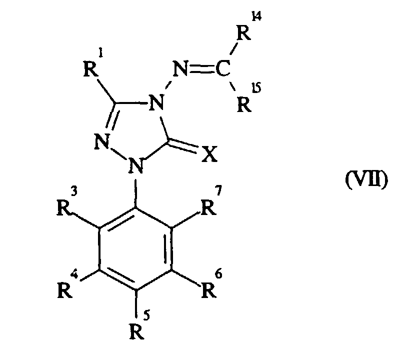 in welcher
   R¹, R³, R⁴, R⁵, R⁶, R⁷ und X die oben angegebenen Bedeutungen haben,
   R¹⁴ für Wasserstoff oder Alkyl steht und
   R¹⁵ für Alkyl oder Alkoxy steht,
mit einen Reduktionsmittel gegebenenfalls in Gegenwart eines Verdünnungsmittels und gegebenenfalls in Gegenwart eines Reaktionshilfsmittels umsetzt

Schließlich wurde gefunden, daß die neuen substituierten 1-Aryltriazolinone der allgemeinen Formel (I) herbizide Eigenschaften besitzen.

Überraschenderweise zeigen die erfindungsgemäßen substituierten 1-Aryltriazolinone der allgemeinen Formel (I) eine erheblich bessere herbizide Wirksamkeit gegenüber Problemunkräutern bei vergleichbarer Verträglichkeit gegenüber Nutzpflanzen im Vergleich zu den aus dem Stand der Technik bekannten substituierten Triazolinonen, wie beispielsweise die Verbindung 3-Methyl-4-propargyl-1-(2,5-difluor-4-cyano-phenyl)-1,2,4-triazolin-5-on, welche chemisch und wirkungsmäßig naheliegende Verbindungen sind.

Die erfindungsgemäßen substituierten 1-Aryltriazolinone sind durch die Formel (I) allgemein definiert.

Besonders bevorzugt sind Verbindungen der Formel (I), bei welchen
- R¹: für Wasserstoff oder für jeweils geradkettiges oder verzweigtes Alkyl, Alkoxy, Alkylthio oder Alkylsulfonyl mit jeweils 1 bis 6 Kohlenstoffatomen steht, außerdem für geradkettiges oder verzweigtes Halogenalkyl mit 1 bis 6 Kohlenstoffatomen und 1 bis 13 gleichen oder verschiedenen Halogenatomen - insbesondere Fluor, Chlor oder Brom - steht oder für Cycloalkyl mit 3 bis 7 Kohlenstoffatomen steht,
- R²: für einen Rest der Formel -NR⁸R⁹steht,
- R³, R⁶: jeweils für Wasserstoff stehen,
- R⁷: für Wasserstoff, Fluor, Chlor, Brom, Amino oder Nitro steht,
- R⁴: für Wasserstoff, Fluor, Chlor, Brom, Cyano, Nitro, oder für einen der Reste -R¹⁰, -O-R¹⁰, -O-R¹⁰, -S(O)-R¹⁰, -SO₂-R¹⁰, -SO₂-OR¹⁰, -SO₂-NR¹¹R¹⁰, -CO-OR¹⁰, -CO-NR¹¹R¹⁰, -O-SO₂-R¹⁰, -N(R¹¹)-SO₂-R¹⁰, -NR¹¹R¹⁰, -NH-P(O)(R¹¹)(OR¹⁰) oder -NH-P(O)(OR¹¹)(OR¹⁰) steht,
- R⁵: für Cyano steht und
- X: für Sauerstoff oder Schwefel steht, wobei
- R⁸: für Wasserstoff, für geradkettiges oder verzweigtes Alkyl mit 1 bis 6 Kohlenstoffatomen oder für geradkettiges oder verzweigtes Halogenalkyl mit 1 bis 6 Kohlenstoffatomen und 1 bis 13 gleichen oder verschiedenen Halogenatomen - insbesondere Fluor, Chlor oder Brom - steht und außerdem für einen Rest der Formel -CO-R¹² oder für einen Rest der Formel -S(O)ₙ-R¹² steht,
- R⁹: für geradkettiges oder verzweigtes Alkyl mit 1 bis 6 Kohlenstoffatomen oder für geradkettiges oder verzweigtes Halogenalkyl mit 1 bis 6 Kohlenstoffatomen und 1 bis 13 gleichen oder verschiedenen Halogenatomen - insbesondere Fluor, Chlor oder Brom - steht und außerdem für einen Rest der Formel -CO-R¹² oder für einen Rest der Formel -S(O)ₙ-R¹² steht,
- R¹⁰: für Wasserstoff steht;
- R¹⁰: außerdem für gegebenenfalls einfach oder zweifach, gleich oder verschieden substituiertes, geradkettiges oder verzweigtes Alkyl mit 1 bis 12 Kohlenstoffatomen steht, wobei als Substituenten infrage kommen:
Cyano, Carboxyl, Carbamoyl, jeweils geradkettiges oder verzweigtes Alkoxy, Alkoxyalkoxy, Alkylthio, Alkylsulfinyl, Alkylsulfonyl, Alkoxycarbonyl, N-Alkylaminocarbonyl, N,N-Dialkylaminocarbonyl oder Alkylsulfonylaminocarbonyl mit jeweils 1 bis 6 Kohlenstoffatomen in den einzelnen Alkylteilen oder Heterocyclyl, wobei als Heterocyclylrest ein fünf- bis siebengliedriger, gegebenenfalls benzannellierter, gesättigter oder ungesättigter Heterocyclus mit 1 bis 3 gleichen oder verschiedenen Heteroatomen - insbesondere Stickstoff, Sauerstoff und/oder Schwefel - steht;
- R¹⁰: außerdem für geradkettiges oder verzweigtes Halogenalkyl mit 1 bis 6 Kohlenstoffatomen und 1 bis 13 gleichen oder verschiedenen Halogenatomen - insbesondere Fluor, Chlor und/oder Brom - steht,
- R¹⁰: außerdem für jeweils gegebenenfalls einfach bis dreifach, gleich oder verschieden durch Halogen - insbesondere Fluor, Chlor und/oder Brom - substituiertes Alkenyl oder Alkinyl mit jeweils 2 bis 6 Kohlenstoffatomen steht;
- R¹⁰: außerdem für gegebenenfalls einfach bis dreifach, gleich oder verschieden durch Halogen - insbesondere Fluor, Chlor und/oder Brom - und/oder geradkettiges oder verzweigtes Alkyl mit 1 bis 3 Kohlenstoffatomen substituiertes Cycloalkyl mit 3 bis 7 Kohlenstoffatomen steht;
- R¹⁰: außerdem für jeweils gegebenenfalls im Phenylteil einfach bis fünffach, gleich oder verschieden substituiertes Phenylalkyl oder Phenyl mit gegebenenfalls 1 bis 3 Kohlenstoffatomen im geradkettigen oder verzweigten Alkylteil steht, oder für einen gegebenenfalls einfach bis dreifach, gleich oder verschieden substituierten und/oder benzannellierten, gesättigten oder ungesättigten, fünf- bis siebengliedrigen Heterocyclylrest mit 1 bis 3 gleichen oder verschiedenen Heteroatomen - insbesondere Stickstoff, Sauerstoff und/oder Schwefel - steht, wobei als Phenyl- bzw. Heterocyclylsubstituenten jeweils infrage kommen:
Fluor, Chlor, Brom, Cyano, Nitro, Amino, N-Acetylamino, jeweils geradkettiges oder verzweigtes Alkyl, Alkoxy, Alkylthio, Alkylsulfinyl oder Alkylsulfonyl mit jeweils 1 bis 4 Kohlenstoffatomen, jeweils geradkettiges oder verzweigtes Halogenalkyl, Halogenalkoxy, Halogenalkylthio, Halogenalkylsulfinyl oder Halogenalkylsulfonyl mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen sowie gegebenenfalls einfach bis fünffach, gleich oder verschieden durch Fluor, Chlor, Brom und/oder geradkettiges oder verzweigtes Alkyl oder Alkoxy mit jeweils 1 bis 4 Kohlenstoffatomen und/oder geradkettiges oder verzweigtes Halogenalkyl oder Halogenalkoxy mit jeweils 1 bis 4 Kohlenstoffatomen und 1 bis 9 gleichen oder verschiedenen Halogenatomen substituiertes Phenyl;
- R¹¹: für Wasserstoff steht;
- R¹¹: außerdem für gegebenenfalls einfach oder zweifach, gleich oder verschieden substituiertes, geradkettiges oder verzweigtes Alkyl mit 1 bis 12 Kohlenstoffatomen steht, wobei als Substituenten infrage kommen:
Cyano, Carboxyl, Carbamoyl, jeweils geradkettiges oder verzweigtes Alkoxy, Alkoxyalkoxy, Alkylthio, Alkylsulfinyl, Alkylsulfonyl, Alkoxycarbonyl, N-Alkylaminocarbonyl, N,N-Dialkylaminocarbonyl oder Alkylsulfonylaminocarbonyl mit jeweils 1 bis 6 Kohlenstoffatomen in den einzelnen Alkylteilen oder Heterocyclyl, wobei als Heterocyclylrest ein fünf- bis siebengliedriger, gegebenenfalls benzannellierter, gesättigter oder ungesättigter Heterocyclus mit 1 bis 3 gleichen oder verschiedenen Heteroatomen - insbesondere Stickstoff, Sauerstoff und/oder Schwefel - steht;
- R¹¹: außerdem für geradkettiges oder verzweigtes Halogenalkyl mit 1 bis 6 Kohlenstoffatomen und 1 bis 13 gleichen oder verschiedenen Halogenatomen - insbesondere Fluor, Chlor und/oder Brom - steht,
- R¹¹: außerdem für jeweils gegebenenfalls einfach bis dreifach, gleich oder verschieden durch Halogen - insbesondere Fluor, Chlor und/oder Brom - substituiertes Alkenyl oder Alkinyl mit jeweils 2 bis 6 Kohlenstoffatomen steht;
- R¹¹: außerdem für gegebenenfalls einfach bis dreifach, gleich oder verschieden durch Halogen - insbesondere Fluor, Chlor und/oder Brom - und/oder geradkettiges oder verzweigtes Alkyl mit 1 bis 3 Kohlenstoffatomen substituiertes Cycloalkyl mit 3 bis 7 Kohlenstoffatomen steht;
- R¹¹: außerdem für jeweils gegebenenfalls im Phenylteil einfach bis fünffach, gleich oder verschieden substituiertes Phenylalkyl oder Phenyl mit gegebenenfalls 1 bis 3 Kohlenstoffatomen im geradkettigen oder verzweigten Alkylteil steht, wobei als Phenylsubstituenten jeweils infrage kommen:
Fluor, Chlor, Brom, Cyano, Nitro, Amino, N-Acetylamino, jeweils geradkettiges oder verzweigtes Alkyl, Alkoxy, Alkylthio, Alkylsulfinyl oder Alkylsulfonyl mit jeweils 1 bis 4 Kohlenstoffatomen, jeweils geradkettiges oder verzweigtes Halogenalkyl, Halogenalkoxy, Halogenalkylthio, Halogenalkylsulfinyl oder Halogenalkylsulfonyl mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen sowie gegebenenfalls einfach bis fünffach, gleich oder verschieden durch Fluor, Chlor, Brom und/oder geradkettiges oder verzweigtes Alkyl oder Alkoxy mit jeweils 1 bis 4 Kohlenstoffatomen und/oder geradkettiges oder verzweigtes Halogenalkyl oder Halogenalkoxy mit jeweils 1 bis 4 Kohlenstoffatomen und 1 bis 9 gleichen oder verschiedenen Halogenatomen substituiertes Phenyl;
- R¹²: für gegebenenfalls einfach oder zweifach, gleich oder verschieden substituiertes, geradkettiges oder verzweigtes Alkyl mit 1 bis 12 Kohlenstoffatomen steht, wobei als Substituenten infrage kommen:
Cycloalkyl mit 3 bis 7 Kohlenstoffatomen oder Heterocyclyl, wobei als Heterocyclylrest ein fünf- bis siebengliedriger, gegebenenfalls benzannellierter, gesättigter oder ungesättigter Heterocyclus mit 1 bis 3 gleichen oder verschiedenen Heteroatomen - insbesondere Stickstoff, Sauerstoff und/oder Schwefel - steht;
- R¹²: außerdem für geradkettiges oder verzweigtes Halogenalkyl mit 1 bis 6 Kohlenstoffatomen und 1 bis 13 gleichen oder verschiedenen Halogenatomen - insbesondere Fluor, Chlor und/oder Brom - steht,
- R¹²: außerdem für gegebenenfalls einfach bis dreifach, gleich oder verschieden durch Halogen - insbesondere Fluor, Chlor und/oder Brom - und/oder geradkettiges oder verzweigtes Alkyl mit 1 bis 3 Kohlenstoffatomen substituiertes Cycloalkyl mit 3 bis 7 Kohlenstoffatomen steht;
- R¹²: außerdem für jeweils gegebenenfalls im Phenylteil einfach bis fünffach, gleich oder verschieden substituiertes Phenylalkyl oder Phenyl mit gegebenenfalls 1 bis 3 Kohlenstoffatomen im geradkettigen oder verzweigten Alkylteil steht, oder für einen gegebenenfalls einfach bis dreifach, gleich oder verschieden substituierten und/oder benzannellierten, gesättigten oder ungesättigten, fünf- bis siebengliedrigen Heterocyclylrest mit 1 bis 3 gleichen oder verschiedenen Heteroatomen - insbesondere Stickstoff, Sauerstoff und/oder Schwefel - steht, wobei als Phenyl- bzw. Heterocyclylsubstituenten jeweils infrage kommen:
Fluor, Chlor, Brom, Cyano, Nitro, Amino, N-Acetylamino, jeweils geradkettiges oder verzweigtes Alkyl, Alkoxy, Alkylthio, Alkylsulfinyl oder Alkylsulfonyl mit jeweils 1 bis 4 Kohlenstoffatomen, jeweils geradkettiges oder verzweigtes Halogenalkyl, Halogenalkoxy, Halogenalkylthio, Halogenalkylsulfinyl oder Halogenalkylsulfonyl mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen sowie gegebenenfalls einfach bis fünffach, gleich oder verschieden durch Fluor, Chlor, Brom und/oder geradkettiges oder verzweigtes Alkyl oder Alkoxy mit jeweils 1 bis 4 Kohlenstoffatomen und/oder geradkettiges oder verzweigtes Halogenalkyl oder Halogenalkoxy mit jeweils 1 bis 4 Kohlenstoffatomen und 1 bis 9 gleichen oder verschiedenen Halogenatomen substituiertes Phenyl und
- n: für eine Zahl 0, 1 oder 2 steht.

Ganz besonders bevorzugt sind Verbindungen der Formel (I), bei welchen
- R¹: für Wasserstoff oder für jeweils geradkettiges oder verzweigtes Alkyl, Alkoxy, Alkylthio oder Alkylsulfonyl mit jeweils 1 bis 4 Kohlenstoffatomen steht, außerdem für geradkettiges oder verzweigtes Halogenalkyl mit 1 bis 4 Kohlenstoffatomen und 1 bis 9 gleichen oder verschiedenen Halogenatomen - insbesondere Fluor, Chlor oder Brom - steht, oder für Cycloalkyl mit 3, 5 oder 6 Kohlenstoffatomen steht,
- R²: für einen Rest der Formel -NR⁸R⁹steht,
- R³, R⁶: jeweils für Wasserstoff stehen,
- R⁷: für Wasserstoff, Fluor, Chlor, Brom, Amino oder Nitro steht,
- R⁴: für Wasserstoff, Fluor, Chlor, Brom, Cyano, Nitro, oder für einen der Reste -R¹⁰, -O-R¹⁰, -S-R¹⁰, -S(O)-R¹⁰, -SO₂-R¹⁰, -SO₂-OR¹⁰, -SO₂-NR¹¹R¹⁰, -CO-OR¹⁰, -CO-NR¹¹R¹⁰, -O-SO₂-R¹⁰, -N(R¹¹)-SO₂-R¹⁰, -NR¹¹R¹⁰, -NH-P(O)(R¹¹)(OR¹⁰) oder -NH-P(O)(OR¹¹)(OR¹⁰) steht,
- R⁵: für Cyano steht und
- X: für Sauerstoff oder Schwefel steht, wobei
- R⁸: für Wasserstoff, für geradkettiges oder verzweigtes Alkyl mit 1 bis 4 Kohlenstoffatomen oder für geradkettiges oder verzweigtes Halogenalkyl mit 1 bis 4 Kohlenstoffatomen und 1 bis 9 gleichen oder verschiedenen Halogenatomen - insbesondere Fluor, Chlor oder Brom - steht und außerdem für einen Rest der Formel -CO-R¹² oder für einen Rest der Formel -S(O)ₙ-R¹² steht,
- R⁹: für geradkettiges oder verzweigtes Alkyl mit 1 bis 4 Kohlenstoffatomen oder für geradkettiges oder verzweigtes Halogenalkyl mit 1 bis 4 Kohlenstoffatomen und 1 bis 9 gleichen oder verschiedenen Halogenatomen - insbesondere Fluor, Chlor oder Brom - steht und außerdem für einen Rest der Formel -CO-R¹² oder für einen Rest der Formel -S(O)ₙ-R¹² steht,
- R¹⁰: für Wasserstoff steht;
- R¹⁰: außerdem für gegebenenfalls einfach substituiertes, geradkettiges oder verzweigtes Alkyl mit 1 bis 8 Kohlenstoffatomen steht, wobei als Substituenten infrage kommen:
Cyano, Carboxyl, Carbamoyl, jeweils geradkettiges oder verzweigtes Alkoxy, Alkoxyalkoxy, Alkylthio, Alkylsulfinyl, Alkylsulfonyl, Alkoxycarbonyl, N-Alkylaminocarbonyl, N,N-Dialkylaminocarbonyl oder Alkylsulfonylaminocarbonyl mit jeweils 1 bis 4 Kohlenstoffatomen in den einzelnen Alkylteilen oder Heterocyclyl, wobei als Heterocyclylrest ein fünf- oder sechsgliedriger, gegebenenfalls benzannellierter, gesättigter oder aromatischer Heterocyclus mit 1 bis 3 gleichen oder verschiedenen Heteroatomen - insbesondere Stickstoff, Sauerstoff und/oder Schwefel - steht;
- R¹⁰: außerdem für geradkettiges oder verzweigtes Halogenalkyl mit 1 bis 4 Kohlenstoffatomen und 1 bis 9 gleichen oder verschiedenen Halogenatomen - insbesondere Fluor, Chlor und/oder Brom - steht,
- R¹⁰: außerdem für jeweils gegebenenfalls einfach bis dreifach, gleich oder verschieden durch Halogen - insbesondere Fluor, Chlor und/oder Brom - substituiertes Alkenyl oder Alkinyl mit jeweils 2 bis 6 Kohlenstoffatomen steht;
- R¹⁰: außerdem für gegebenenfalls einfach bis dreifach, gleich oder verschieden durch Halogen - insbesondere Fluor, Chlor und/oder Brom - und/oder geradkettiges oder verzweigtes Alkyl mit 1 bis 3 Kohlenstoffatomen substituiertes Cycloalkyl mit 3, 5 oder 6 Kohlenstoffatomen steht;
- R¹⁰: außerdem für jeweils gegebenenfalls im Phenylteil einfach bis dreifach, gleich oder verschieden substituiertes Phenylalkyl oder Phenyl mit gegebenenfalls 1 bis 3 Kohlenstoffatomen im geradkettigen oder verzweigten Alkylteil steht oder für einen gegebenenfalls einfach bis dreifach, gleich oder verschieden substituierten und/oder benzannellierten, gesättigten oder aromatischen, fünf- oder sechsgliedrigen Heterocyclylrest mit 1 bis 3 gleichen oder verschiedenen Heteroatomen - insbesondere Stickstoff, Sauerstoff und/oder Schwefel - steht, wobei als Phenyl- bzw. Heterocyclylsubstituenten jeweils infrage kommen:
Fluor, Chlor, Brom, Cyano, Nitro, Amino, N-Acetylamino, jeweils geradkettiges oder verzweigtes Alkyl, Alkoxy, Alkylthio, Alkylsulfinyl oder Alkylsulfonyl mit jeweils 1 bis 3 Kohlenstoffatomen, jeweils geradkettiges oder verzweigtes Halogenalkyl, Halogenalkoxy, Halogenalkylthio, Haogenalkylsulfinyl oder Halogenalkylsulfonyl mit jeweils 1 bis 3 Kohlenstoffatomen und 1 bis 7 gleichen oder verschiedenen Halogenatomen, jeweils geradkettiges oder verzweigtes Alkoxycarbonyl oder Alkoximinoalkyl mit jeweils bis 3 Kohlenstoffatomen in den einzelnen Alkylteilen sowie gegebenenfalls einfach bis dreifach, gleich oder verschieden durch Fluor, Chlor, Brom und/oder geradkettiges oder verzweigtes Alkyl oder Alkoxy mit jeweils 1 bis 3 Kohlenstoffatomen und/oder geradkettiges oder verzweigtes Halogenalkyl oder Halogenalkoxy mit jeweils 1 bis 3 Kohlenstoffatomen und 1 bis 7 gleichen oder verschiedenen Halogenatomen substituiertes Phenyl;
- R¹¹: für Wasserstoff steht;
- R¹¹: außerdem für gegebenenfalls einfach substituiertes, geradkettiges oder verzweigtes Alkyl mit 1 bis 8 Kohlenstoffatomen steht, wobei als Substituenten infrage kommen:
Cyano, Carboxyl, Carbamoyl, jeweils geradkettiges oder verzweigtes Alkoxy, Alkoxyalkoxy, Alkylthio, Alkylsulfinyl, Alkylsulfonyl, Alkoxycarbonyl, N-Alkylaminocarbonyl, N,N-Dialkylaminocarbonyl oder Alkylsulfonylaminocarbonyl mit jeweils 1 bis 4 Kohlenstoffatomen in den einzelnen Alkylteilen oder Heterocyclyl, wobei als Heterocyclylrest ein fünf- oder sechsgliedriger, gegebenenfalls benzannellierter, gesättigter oder aromatischer Heterocyclus mit 1 bis 3 gleichen oder verschiedenen Heteroatomen - insbesondere Stickstoff, Sauerstoff und/oder Schwefel - steht;
- R¹¹: außerdem für geradkettiges oder verzweigtes Halogenalkyl mit 1 bis 4 Kohlenstoffatomen und 1 bis 9 gleichen oder verschiedenen Halogenatomen - insbesondere Fluor, Chlor und/oder Brom - steht,
- R¹¹: außerdem für jeweils gegebenenfalls einfach bis dreifach, gleich oder verschieden durch Halogen - insbesondere Fluor, Chlor und/oder Brom - substituiertes Alkenyl oder Alkinyl mit jeweils 2 bis 6 Kohlenstoffatomen steht;
- R¹¹: außerdem für gegebenenfalls einfach bis dreifach, gleich oder verschieden durch Halogen - insbesondere Fluor, Chlor und/oder Brom - und/oder geradkettiges oder verzweigtes Alkyl mit 1 bis 3 Kohlenstoffatomen substituiertes Cycloalkyl mit 3 bis 7 Kohlenstoffatomen steht;
- R¹¹: außerdem für jeweils gegebenenfalls im Phenylteil einfach bis dreifach, gleich oder verschieden substituiertes Phenylalkyl oder Phenyl mit gegebenenfalls 1 bis 3 Kohlenstoffatomen im geradkettigen oder verzweigten Alkylteil steht, wobei als Phenylsubstituenten jeweils infrage kommen:
Fluor, Chlor, Brom, Cyano, Nitro, Amino, N-Acetylamino, jeweils geradkettiges oder verzweigtes Alkyl, Alkoxy, Alkylthio, Alkylsulfinyl oder Alkylsulfonyl mit jeweils 1 bis 4 Kohlenstoffatomen, jeweils geradkettiges oder verzweigtes Halogenalkyl, Halogenalkoxy, Halogenalkylthio, Halogenalkylsulfinyl oder Halogenalkylsulfonyl mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkyteilen sowie gegebenenfalls einfach bis dreifach, gleich oder verschieden durch Fluor, Chlor, Brom und/oder geradkettiges oder verzweigtes Alkyl oder Alkoxy mit jeweils 1 bis 4 Kohlenstoffatomen und/oder geradkettiges oder verzweigtes Halogenalkyl oder Halogenalkoxy mit jeweils 1 bis 4 Kohlenstoffatomen und 1 bis 9 gleichen oder verschiedenen Halogenatomen substituiertes Phenyl;
- R¹²: für gegebenenfalls einfach oder zweifach, gleich oder verschieden substituiertes, geradkettiges oder verzweigtes Alkyl mit 1 bis 12 Kohlenstoffatomen steht, wobei als Substituenten infrage kommen:
Cycloalkyl mit 3 bis 7 Kohlenstoffatomen oder Heterocyclyl, wobei als Heterocyclylrest ein fünf- bis sechsgliedriger, gegebenenfalls benzannellierter, gesättigter sättigter oder Heterocyclus mit 1 bis 3 gleichen oder verschiedenen Heteroatomen - insbesondere Stickstoff, Sauerstoff und/oder Schwefel - steht;
- R¹²: außerdem für geradkettiges oder verzweigtes Halogenalkyl mit 1 bis 6 Kohlenstoffatomen und 1 bis 13 gleichen oder verschiedenen Halogenatomen - insbesondere Fluor, Chlor und/oder Brom - steht,
- R¹²: außerdem für gegebenenfalls einfach bis dreifach, gleich oder verschieden durch Halogen - insbesondere Fluor, Chlor und/oder Brom - und/oder geradkettiges oder verzweigtes Alkyl mit 1 bis 3 Kohlenstoffatomen substituiertes Cycloalkyl mit 3 bis 7 Kohlenstoffatomen steht;
- R¹²: außerdem für jeweils gegebenenfalls im Phenylteil einfach bis dreifach, gleich oder verschieden substituiertes Phenylalkyl oder Phenyl mit gegebenenfalls 1 bis 3 Kohlenstoffatomen im geradkettigen oder verzweigten Alkylteil steht, oder für einen gegebenenfalls einfach bis dreifach, gleich oder verschieden substituierten und/oder benzannellierten, gesättigten oder ungesättigten, fünf- bis sechsgliedrigen Heterocyclylrest mit 1 bis 3 gleichen oder verschiedenen Heteroatomen - insbesondere Stickstoff, Sauerstoff und/oder Schwefel - steht, wobei als Phenyl- bzw. Heterocyclylsubstituenten jeweils infrage kommen:
Fluor, Chlor, Brom, Cyano, Nitro, Amino, N-Acetylamino, jeweils geradkettiges oder verzweigtes Alkyl, Alkoxy, Alkylthio, Alkylsulfinyl oder Alkylsulfonyl mit jeweils 1 bis 4 Kohlenstoffatomen, jeweils geradkettiges oder verzweigtes Halogenalkyl, Halogenalkoxy,Halogenalkylthio, Halogenalkylsulfinyl oder Halogenalkylsulfonyl mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen sowie gegebenenfalls einfach bis dreifach, gleich oder verschieden durch Fluor, Chlor, Brom und/oder geradkettiges oder verzweigtes Alkyl oder Alkoxy mit jeweils 1 bis 4 Kohlenstoffatomen und/oder geradkettiges oder verzweigtes Halogenalkyl oder Halogenalkoxy mit jeweils 1 bis 4 Kohlenstoffatomen und 1 bis 9 gleichen oder verschiedenen Halogenatomen substituiertes Phenyl und
- n: für eine Zahl 0, 1 oder 2 steht.

Im einzelnen seien außer den bei den Herstellungsbeispielen aufgeführten Verbindungen die folgenden substituierten Triazolinone der allgemeinen Formel (I) genannt:

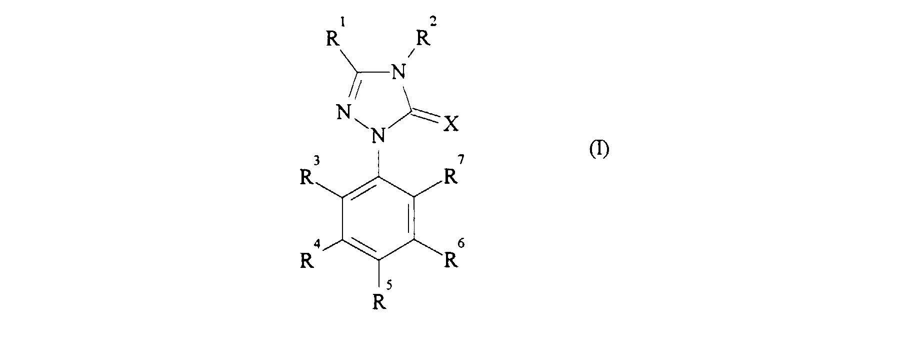

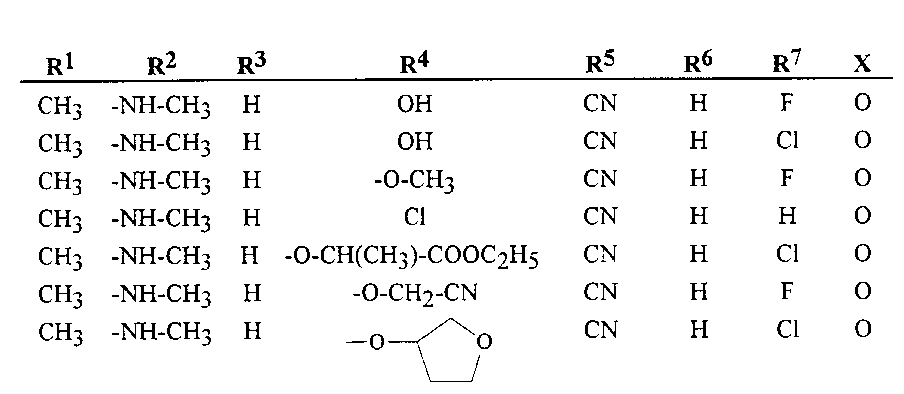

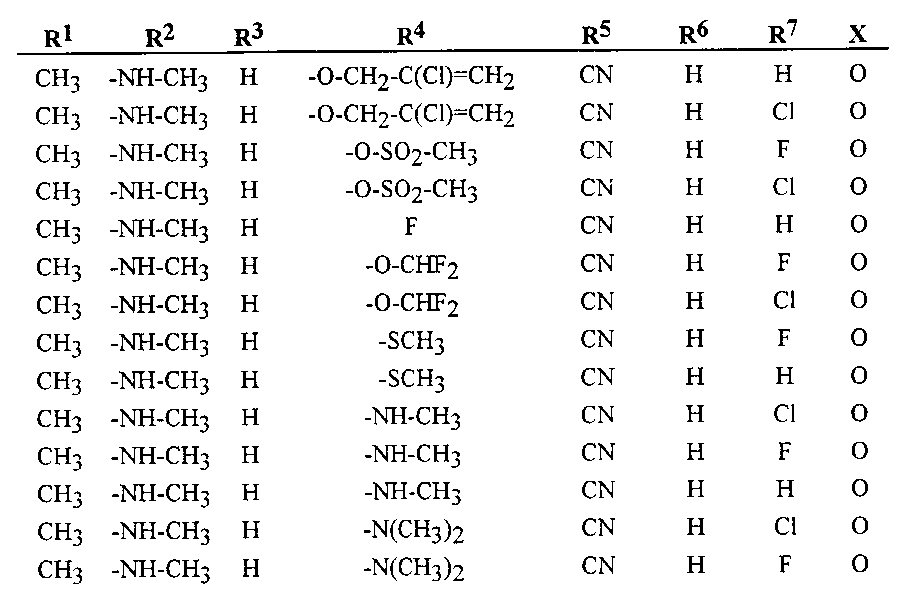

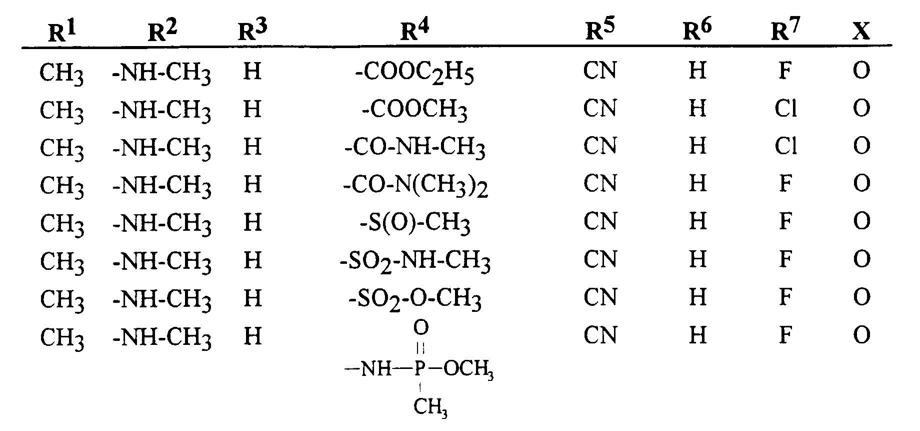

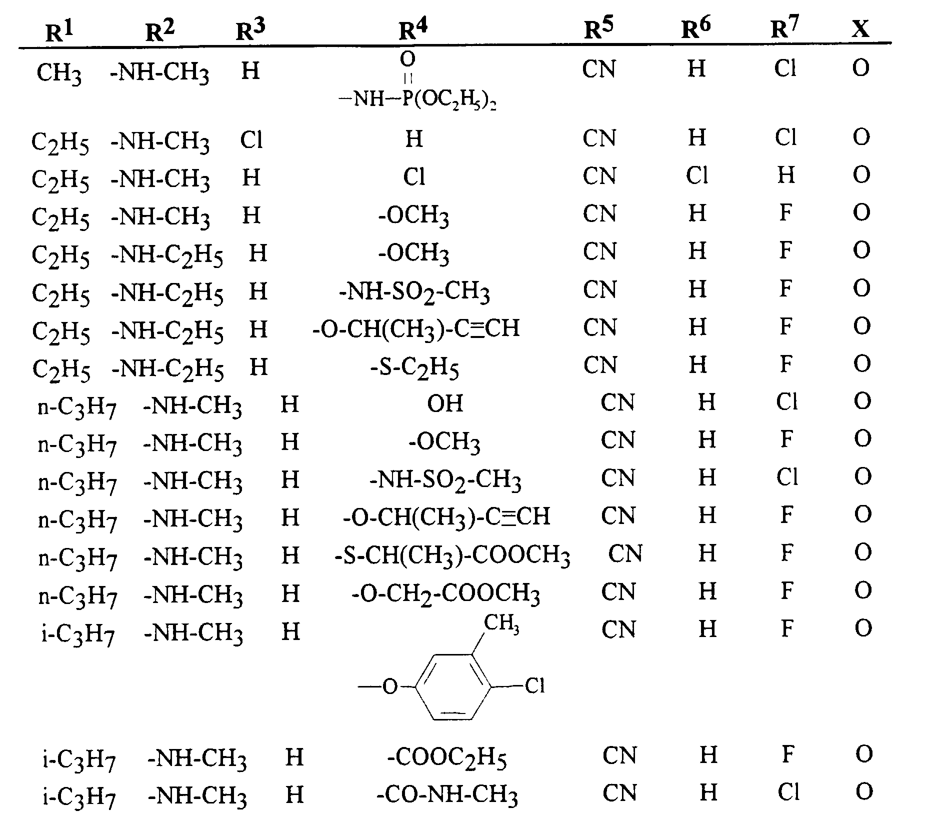

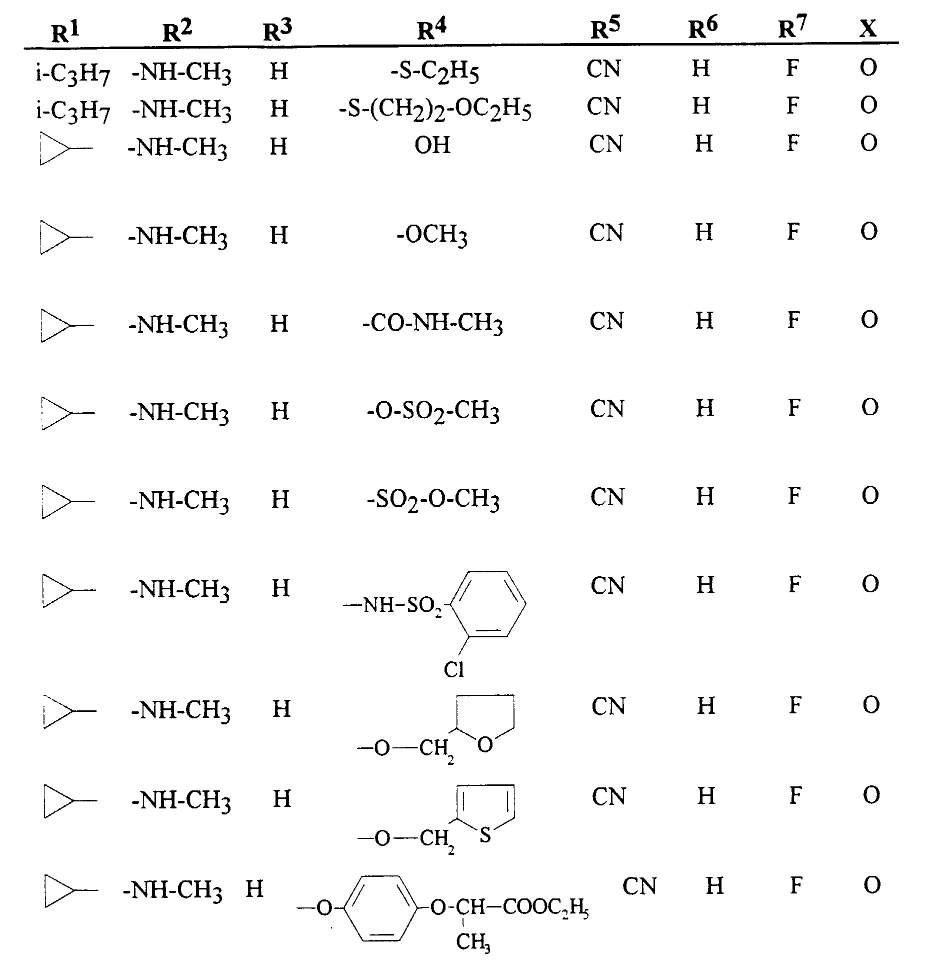

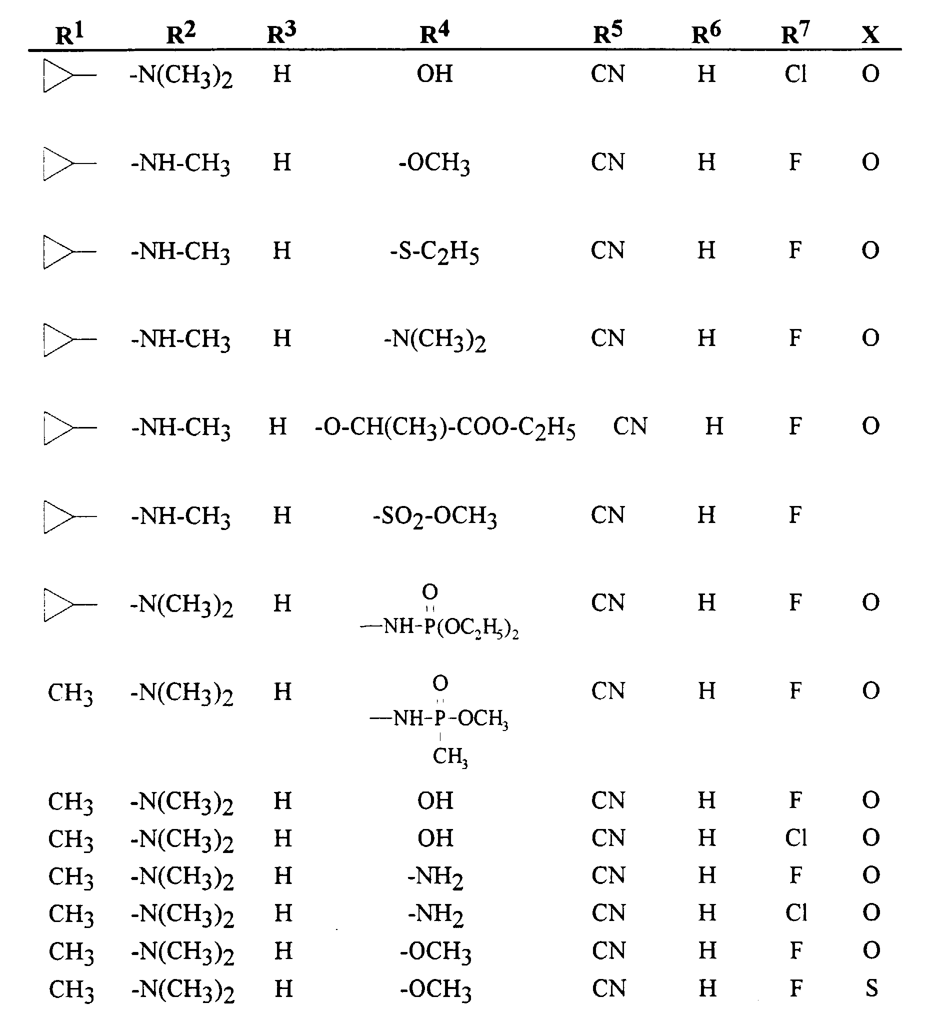

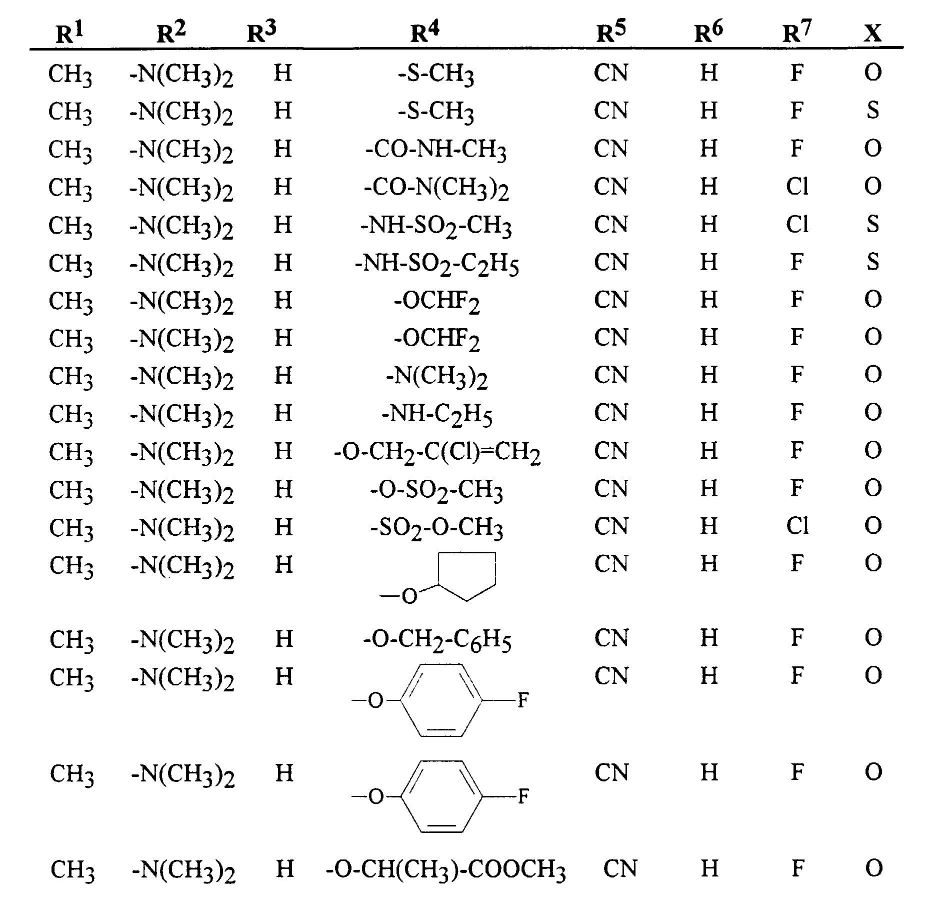

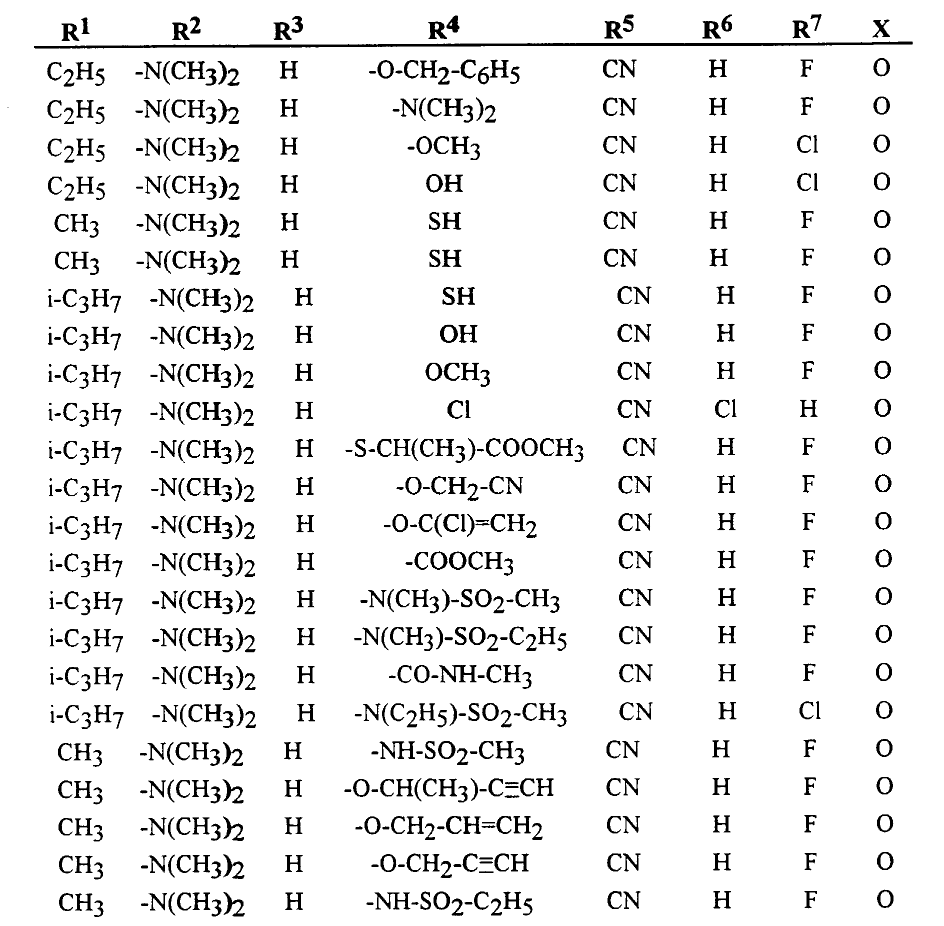

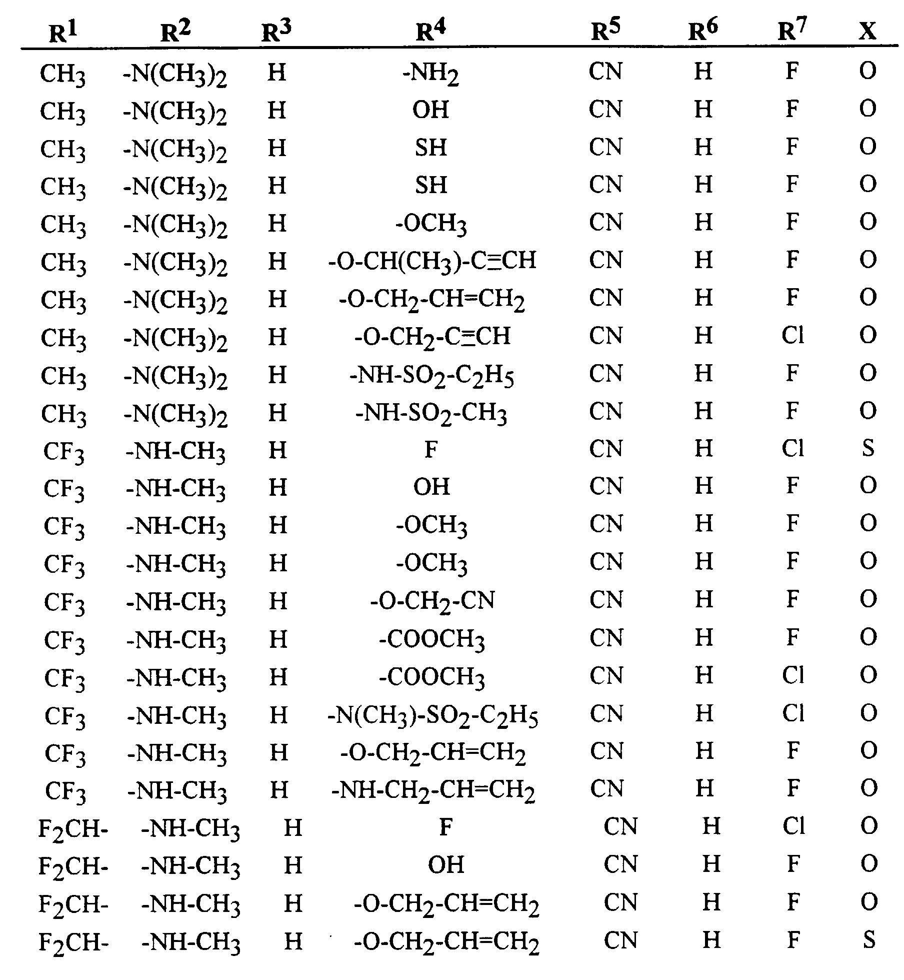

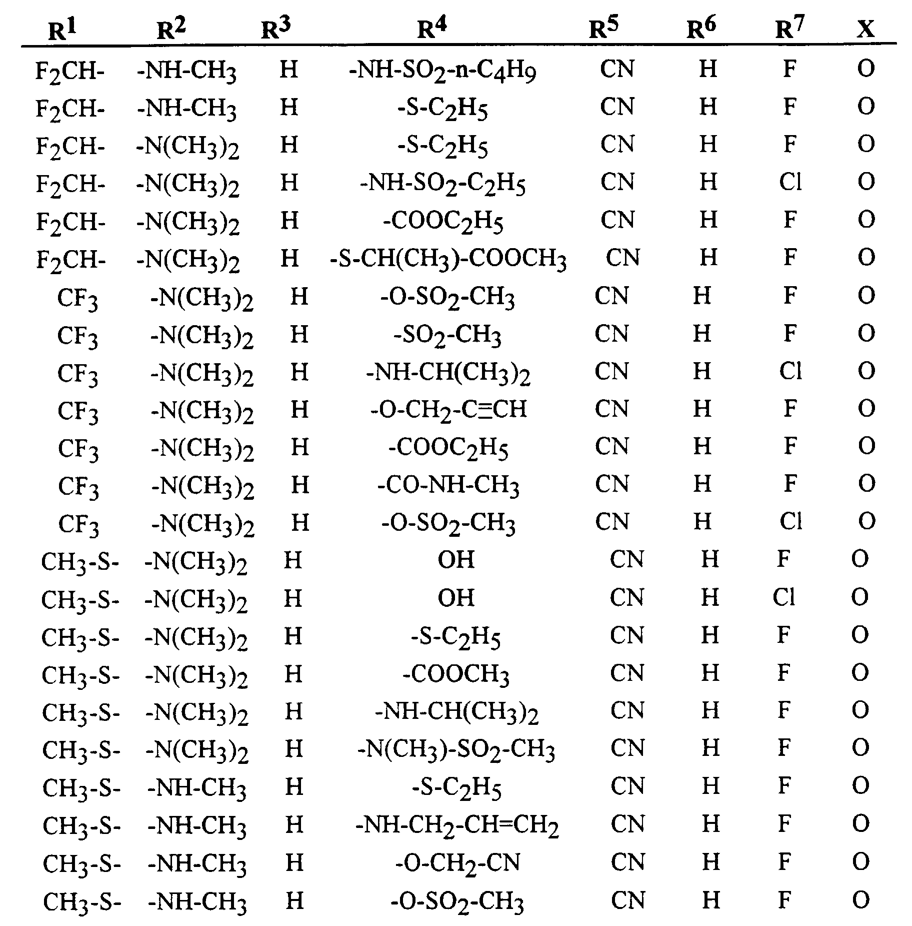

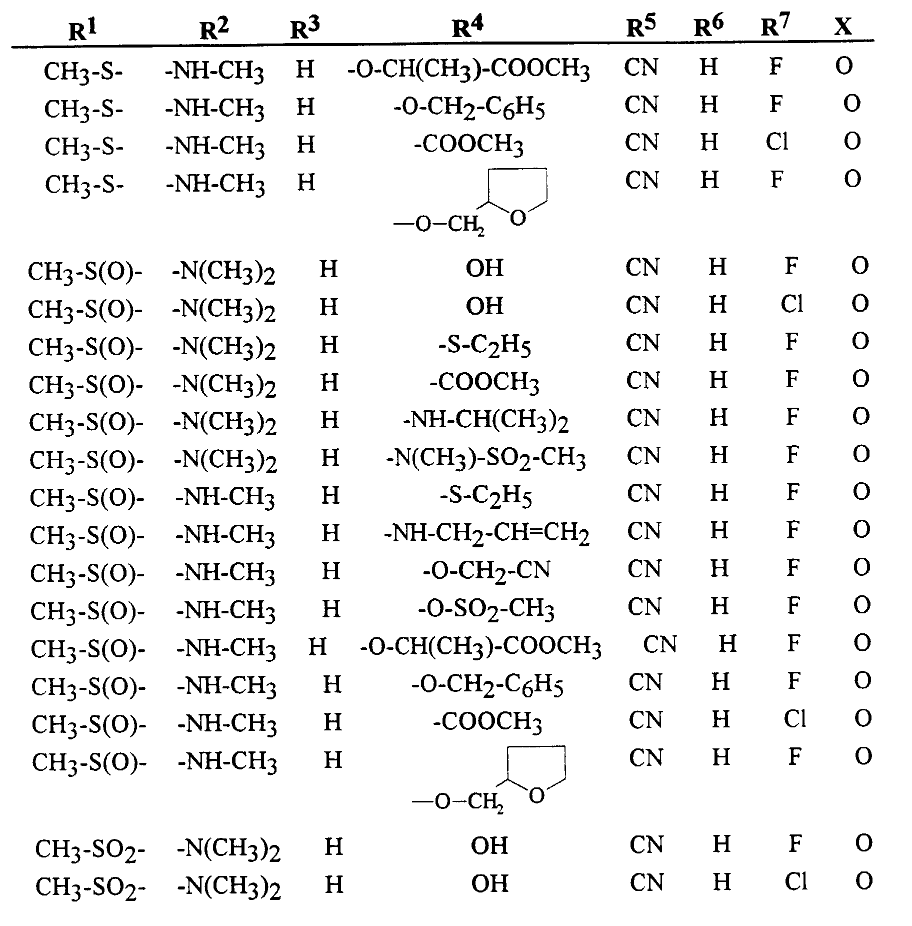

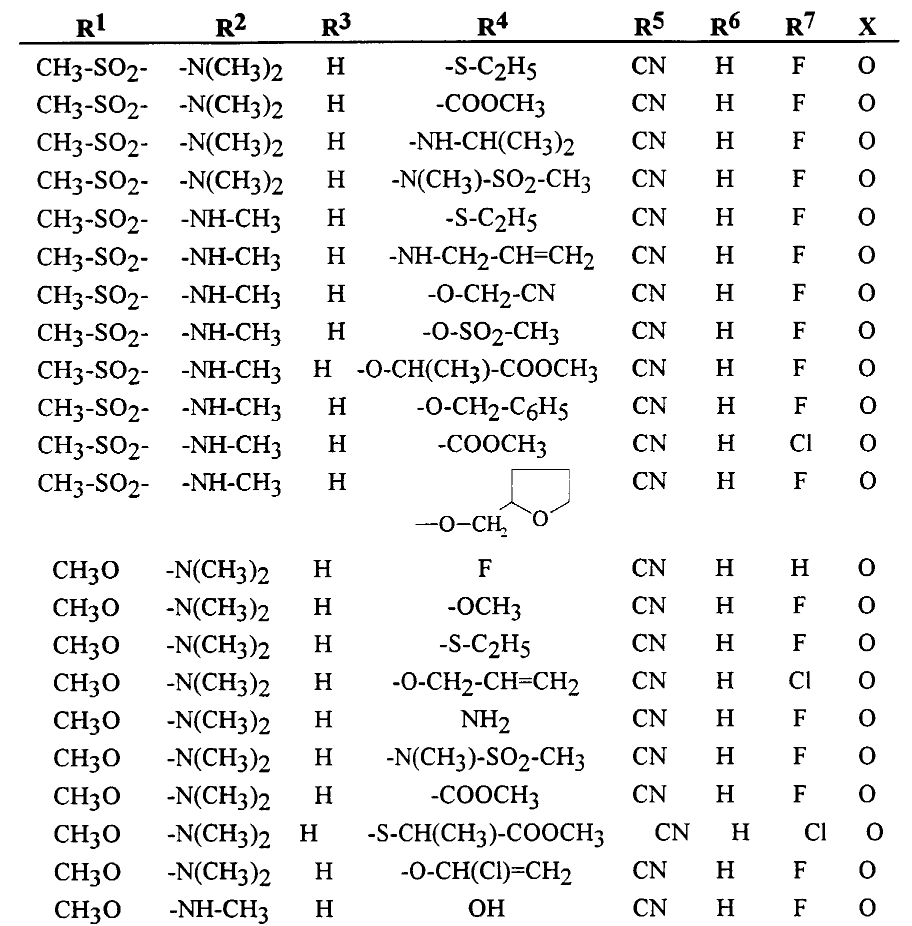

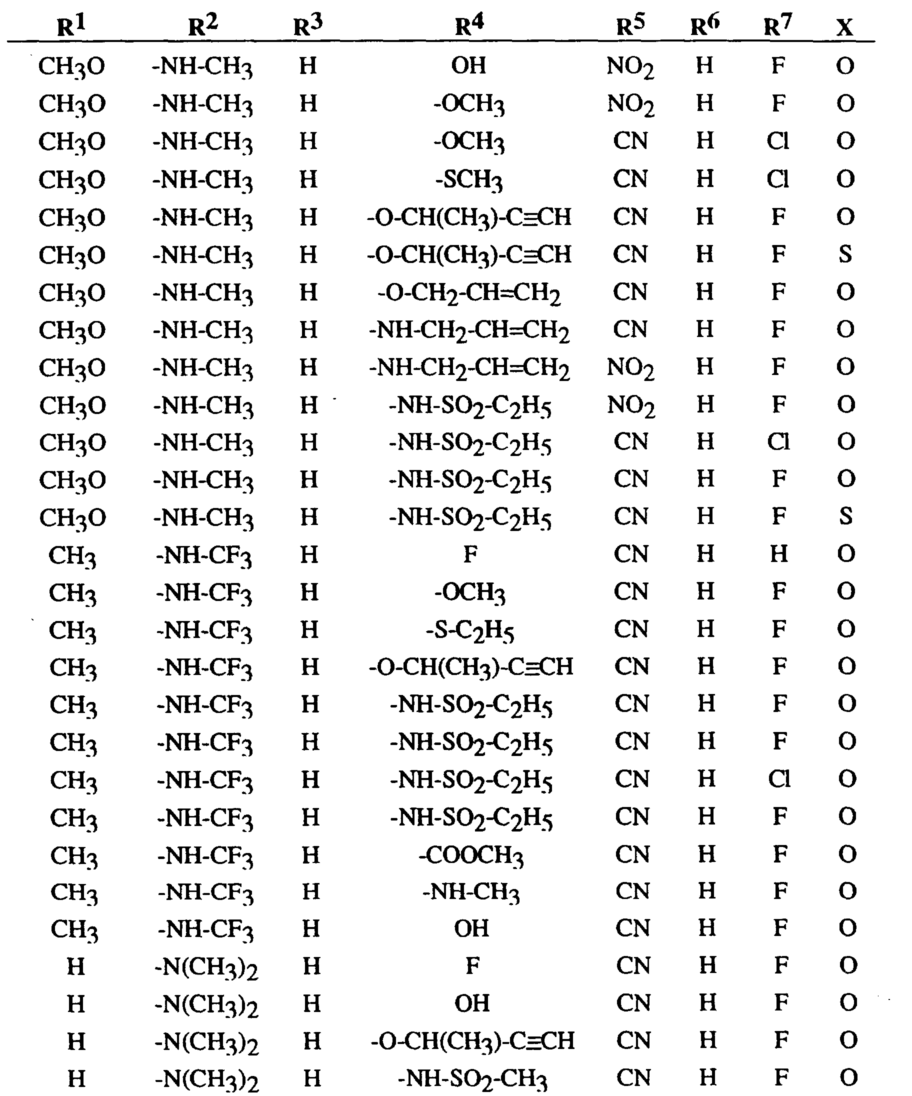

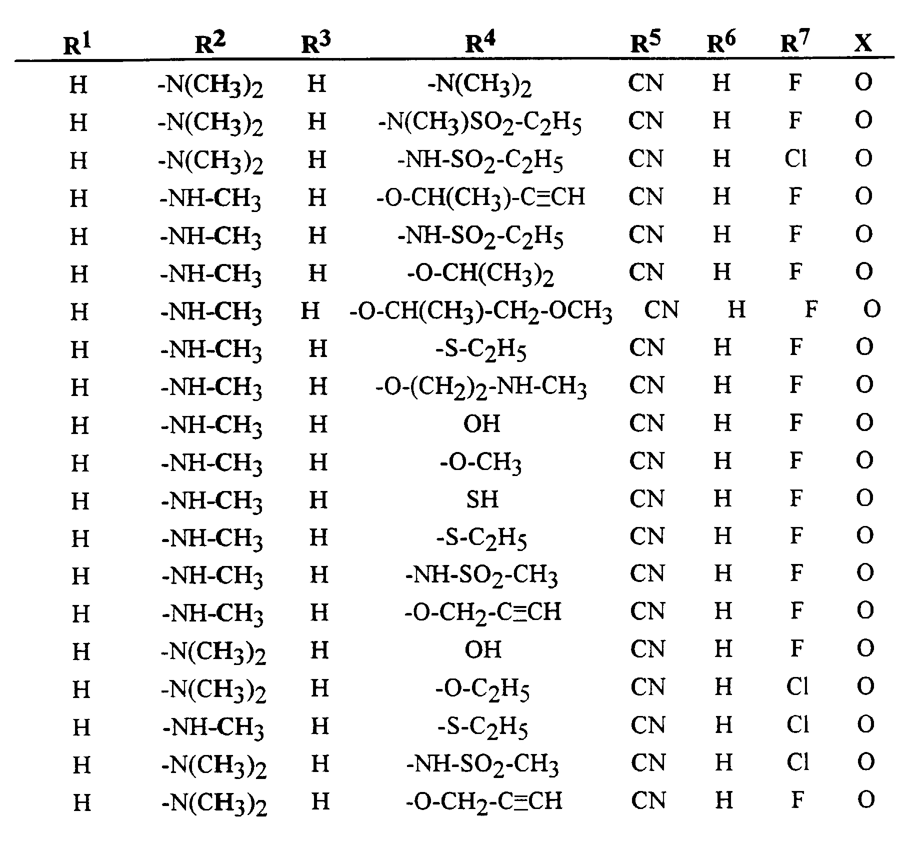

Verwendet man beispielsweise 3-Methyl-4-dimethylamino-1,2,4-triazolin-5-on und 2,4,5-Trifluorbenzonitril als Ausgangsstoffe, so läßt sich der Reaktionsablauf des erfindungsgemäßen Verfahrens (a) durch das folgende Formelschema darstellen:

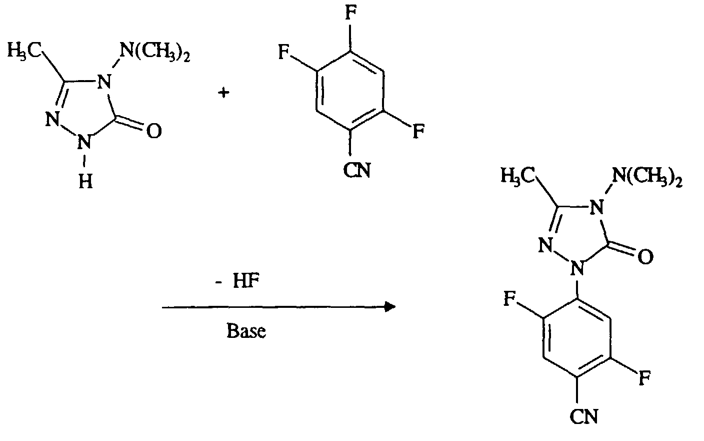

Verwendet man beispielsweise 1-(4-Cyano-2,5-difluorphenyl)-3-methyl-4-dimethylamino-1,2,4-triazolin-5-on und 1-Butin-3-ol als Ausgangsstoffe, so läßt sich der Reaktionsablauf des erfindungsgemäßen Verfahrens (b) durch das folgende Formelschema darstellen:

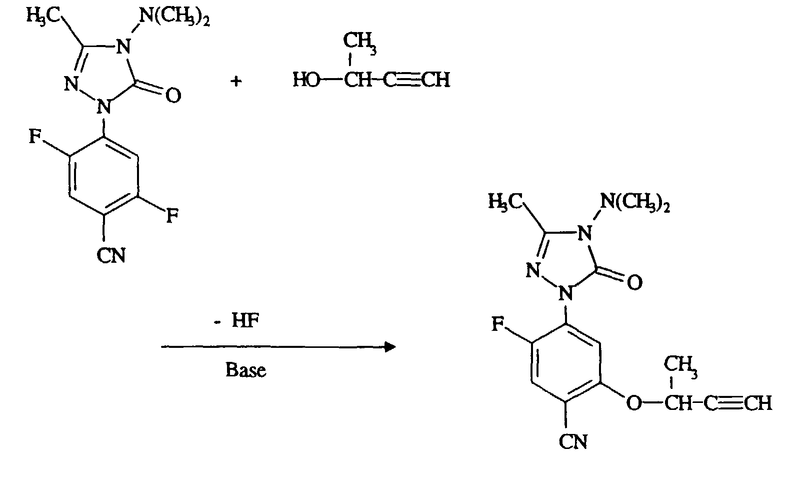

Verwendet man beispielsweise 1-(4-Cyano-2-fluor-5-methoxy-phenyl)-4-amino-3-methyl-1,2,4-triazolin-5-on als Ausgangsverbindung und Acetylchlorid als Acylierungsmittel, so läßt sich der Reaktionsablauf des erfindungsgemäßen Verfahrens (c) durch das folgende Formelschema darstellen:

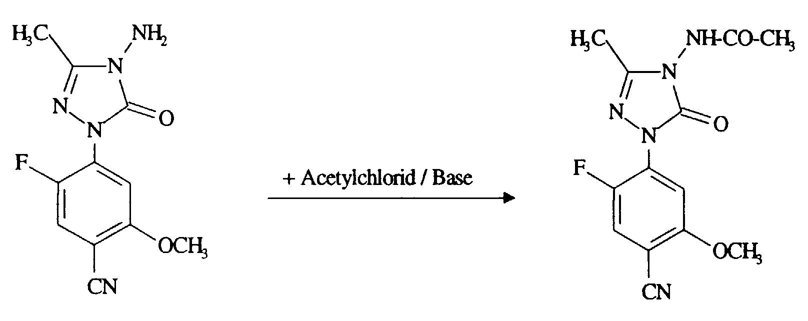

Verwendet man beispielsweise 1-(4-Cyano-2-fluor-5-methoxy-phenyl)-3-methyl-4-isopropylidenimino-1,2,4-triazolin-5-on als Ausgangsverbindung und Natriumborhydrid als Reduktionsmittel, so läßt sich der Reaktionsablauf des erfindungsgemäßen Verfahrens (d) durch das folgende Formelschema darstellen:

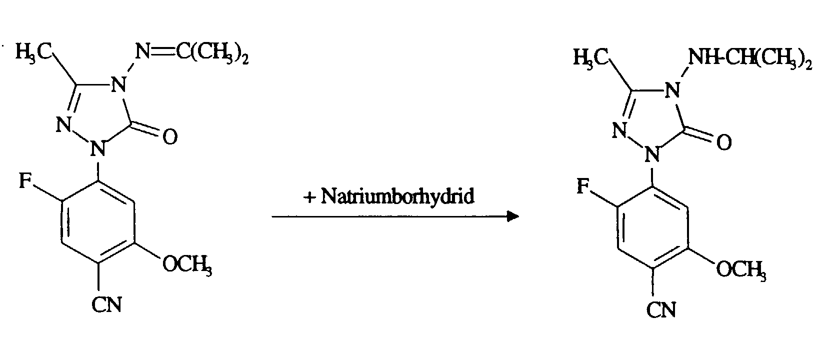

Die zur Durchführung des erfindungsgemäßen Verfahrens (a) als Ausgangsstoffe benötigten 1H-Triazolinone sind durch die Formel (II) allgemein definiert. In dieser Formel (II) stehen R¹, R² und X vorzugsweise und besonders bevorzugt für diejenigen Reste, die bereits im Zusammenhang mit der Beschreibung der erfindungsgemäßen Verbindungen der Formel (I) als bevorzugt und besonders bevorzugt für diese Substituenten genannt wurden.
Die 1H-Triazolinone der Formel (II) sind bekannt oder erhältlich in Analogie zu bekannten Verfahren (vergl. z.B. Chimica Acta Turcica 9, 381 [1981]; EP 399 294; EP 422 469; J. Heterocycl. Chem. 10, 387-390 [1973]; Indian J. Chem. 7, 959-963 [1969]; DE 37 19 575; DE 38 03 523;, Liebigs Ann. Chem. 637, 135 [1960]; J. Heterocycl. Chem. 16, 403 [1979]; J. Heterocycl. Chem. 17, 1691 [1980]; J. Indian Chem. Soc. 57, 270-272 [1980]; Indian J. Chem. Sect. B 22B, 270-271 [1983]; Chem. Ber. 98, 3025 [1965]; JP 52-125168; Europ. J. Med. Chem. 18, 215 [1983]).

Die zur Durchführung des erfindungsgemäßen Verfahrens (a) weiterhin als Ausgangsstoffe benötigten Halogenbenzol-Derivate sind durch die Formel (III) allgemein definiert In dieser Formel (III) stehen R³, R⁴, R⁵, R⁶ und R⁷ vorzugsweise und besonders bevorzugt für diejenigen Reste, die bereits im Zusammenhang mit der Beschreibung der erfindungsgemäßen Verbindungen der Formel (I) als bevorzugt und besonders bevorzugt für diese Substituenten genannt wurden. Hal¹ steht vorzugsweise für Fluor, Chlor oder Brom, insbesondere für Fluor oder Chlor.
Die Halogenbenzol-Derivate der Formel (III) sind allgemein bekannt oder erhältlich in Analogie zu bekannten Verfahren (vergl. z.B. EP 191 181; EP 441 004; EP 431 373). Noch nicht bekannt ist die Verbindung 5-Chlor-2,4-difluorbenzonitril. Man erhält sie, wenn man die bekannte Verbindung 2,4,5-Trichlorbenzonitril (vergl. z.B. EP 441 004) mit Kaliumfluorid gegebenenfalls in Gegenwart eines Verdünnungsmittels wie beispielsweise Tetramethylensulfon bei Temperaturen zwischen 100°C und 200°C umsetzt (vergleiche hierzu auch die Herstellungsbeispiele).

Die zur Durchführung des erfindungsgemäßen Verfahren (b) als Edukte benötigten substituierten Triazolinone sind durch die Formel (Ia) allgemein definiert In dieser Formel (Ia) stehen R¹, R², R³, R⁶, R⁵, R⁷ und X vorzugsweise und besonders bevorzugt für diejenigen Reste, die bereits im Zusammenhang mit der Beschreibung der erfindungsgemäßen Stoffe der Formel (I) als bevorzugt und besonders bevorzugt für diese Substituenten genannt wurden. Hal² steht vorzugsweise für Fluor, Chlor oder Brom, insbesondere für Fluor oder Chlor.
Die substituierten Triazolinone der Formel (Ia) erfindungsgemäße Verbindungen und erhältlich mit Hilfe der erfindungsgemäßen Verfahren (a), (c) und/oder (d).

Die zur Durchführung des erfindungsgemäßen Verfahren (b) weiterhin als Edukte benötigten Nukleophile sind durch die Formel (IV) allgemein definiert. In dieser Formel (IV) steht R¹³ vorzugsweise für einen Rest der Formel -O-R¹⁰, -S-R¹⁰ oder -NR¹¹R¹⁰, wobei R¹⁰ und R¹¹ vorzugsweise für diejenigen Reste stehen, die bereits im Zusammenhang mit der Beschreibung der erfindungsgemäßen Stoffe der Formel (I) als bevorzugt und besonders bevorzugt für diese Substituenten genannt wurden. Die Nukleophile der Formel (IV) sind allgemein bekannte Verbindungen der organischen Chemie.

Die zur Durchführung des erfindungsgemäßen Verfahren (c) als Edukte benötigten substituierten Triazolinone sind durch die Formel (V) allgemein definiert. In dieser Formel (V) stehen R¹, R³, R⁴, R⁵, R⁶, R⁷ und X vorzugsweise und besonders bevorzugt für diejenigen Reste, die bereits im Zusammenhang mit der Beschreibung der erfindungsgemäßen Stoffe der Formel (I) als bevorzugt und besonders bevorzugt für diese Substituenten genannt wurden.
Die substituierten Triazolinone der Formel (V) sind noch nicht bekannt. Sie sind jedoch größtenteils Gegenstand von eigenen noch nicht veröffentlichten Patentanmeldungen und erhältlich mit Hilfe der dort beschriebenen Verfahren, beispielsweise imdem man 4-Amino-1H-triazolinone der Formel (VIII), 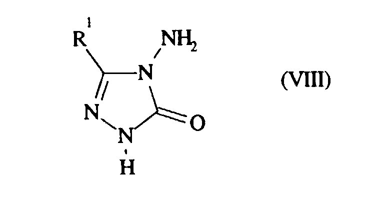 in welcher
- R¹: die oben angegebene Bedeutung hat,
mit Halogenbenzol-Derivaten der Formel (III), 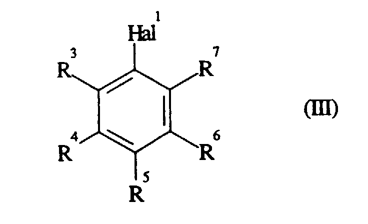 in welcher
R³, R⁴, R⁵, R⁶ und R⁷ die oben angegebenen Bedeutungen haben und Hal¹ für Halogen steht,
gegebenenfalls in Gegenwart eines Verdünnungsmittels und gegebebenfalls in Gegenwart eines Reaktionshilfsmittels in Analogie zur Durchführung des erfindungsgemäßen Verfahrens (a) umsetzt.

4-Amino-1H-triazolinone der Formel (VIII) sind bekannt oder erhältlich in Analogie zu bekannten Verfahren (vergl. z.B. EP 294 666; J. Heterocycl. Chem. 10, 387-390 [1973]; Indian J. Chem. 7, 959-963 [1969]; DE 37 19 575; DE 38 03 523;, Liebigs Ann. Chem. 637, 135 [1960]; J. Heterocycl. Chem. 16, 403 [1979]; J. Heterocycl. Chem. 17, 1691 [1980]; J. Indian Chem. Soc. 57, 270-272 [1980]; Indian J. Chem. Sect. B 22B, 270-271 [1983]; Chem. Ber. 98, 3025 [1965]; JP 52-125168; Europ. J. Med. Chem. 18, 215 [1983]).

Die zur Durchführung des erfindungsgemäßen Verfahren (c) weiterhin als Edukte benötigten Alkylierungs-, Acylierungs- und Sulfonylierungsmittel sind durch die Formel (VI) allgemein definiert. In dieser Formel (VI) steht R⁹ vorzugsweise und besonders bevorzugt für diejenigen Reste, die bereits im Zusammenhang mit der Beschreibung der erfindungsgemäßen Stoffe der Formel (I) als bevorzugt und besonders bevorzugt für diese Substituenten genannt wurden. E steht für einen üblichen elektronenanziehenden Abgangsrest wie beispielsweise Halogen, insbesondere für Chlor, Brom oder Iod oder im Fall der Alkylierungsmittel für jeweils gegebenenfalls substituiertes Alkylsulfonyloxy, Alkoxysulfonyloxy oder Arylsulfonyloxy, wie insbesondere Methansulfonyloxy, Trifluormethansulfonyloxy, Methoxysulfonyloxy, Ethoxysulfonyloxy oder p-Toluolsulfonyloxy.
Die Alkylierungs-, Acylierungs- und Sulfonylierungsmittel der Formel (VI) sind allgemein bekannte Verbindungen der organischen Chemie.

Die zur Durchführung des erfindungsgemäßen Verfahren (d) als Edukte benötigten substituierten 4-Alkylidenimino-triazolinone sind durch die Formel (VII) allgemein definiert In dieser Formel (VII) stehen R¹, R³, R⁴, R⁵, R⁶, R⁷ und X vorzugsweise und besonders bevorzugt für diejenigen Reste, die bereits im Zusammenhang mit der Beschreibung der erfindungsgemäßen Stoffe der Formel (I) als bevorzugt und besonders bevorzugt für diese Substituenten genannt wurden. R¹⁴ steht vorzugsweise für Wasserstoff oder für geradkettiges oder verzweigtes Alkyl mit 1 bis 4 Kohlenstoffatomen, insbesondere für Wasserstoff, Methyl oder Ethyl. R¹⁵ steht vorzugsweise für jewiels geradkettiges oder verzweigtes Alkyl oder Alkoxy mit jeweils 1 bis 4 Kohlenstoffatomen, insbesondere für Methyl, Ethyl, Methoxy oder Ethoxy.

Die 4-Alkylidenimino-triazolinone der Formel (VII) sind noch nicht bekannt. Sie sind jedoch größtenteils Gegenstand von eigenen noch nicht veröffentlichten Patentanmeldungen und erhältlich mit Hilfe der dort beschriebenen Verfahren, beispielsweise imdem man 4-Alkylidenimino-1H-triazolinone der Formel (IX), 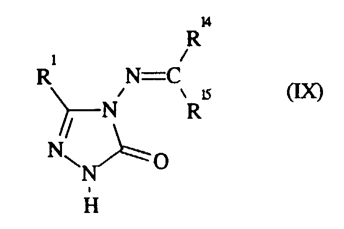 in welcher
R¹, R¹⁴ und R¹⁵ die oben angegebene Bedeutung haben,
mit Halogenbenzol-Derivaten der Formel (III), 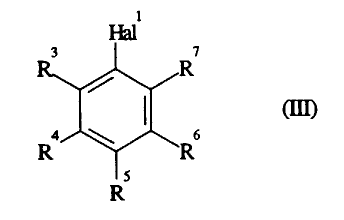 in welcher
R³, R⁴, R⁵, R⁶ und R⁷ die oben angegebenen Bedeutungen haben und Hal¹ für Halogen steht,
gegebenenfalls in Gegenwart eines Verdünnungsmittels und gegebebenfalls in Gegenwart eines Reaktionshilfsmittels in Analogie zur Durchführung des erfindungsgemäßen Verfahrens (a) umsetzt.

4-Alkylidenimino-1H-triazolinone der Formel (IX) sind bekannt oder erhältlich in Analogie zu bekannten Verfahren (vergl. z.B. EP 294 666; EP 399 294).

Als Verdünnungsmittel zur Durchführung des erfindungsgemäßen Verfahrens (a) kommen inerte organische Lösungsmittel infrage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Dichlorbenzol, Petrolether, Hexan, Cyclohexan, Dichlormethan, Chloroform oder Tetrachlorkohlenstoff; Ether, wie Diethylether, Diisopropylether, Dioxan, Tetrahydrofuran oder Ethylenglykoldimethyl- oder -diethylether; Ketone, wie Aceton, Butanon oder Methyl-isobutyl-keton; Nitrile, wie Acetonitril, Propionitril oder Benzonitril; Amide, wie N,N-Dimethylformamid, N,N-Dimethylacetamid, N-Methylformanilid, N-Methylpyrrolidon oder Hexamethylphosphorsäuretriamid oder Ester, wie Essigsäuremethylester oder Essigsäureethylester.

Das erfindungsgemäße Verfahren (a) wird vorzugsweise in Gegenwart eines geeigneten Reaktionshilfsmittels durchgeführt. Als solche kommen alle üblichen anorganischen oder organischen Basen infrage. Hierzu gehören beispielsweise Erdalkali- oder Alkalimetallhydroxide, wie Natriumhydroxid, Calciumhydroxid, Kaliumhydroxid oder auch Ammoniumhydroxid, Alkalimetallcarbonate, wie Natriumcarbonat, Kaliumcarbonat, Kaliumhydrogencarbonat, Natriumhydrogencarbonat oder Ammoniumcarbonat, Alkali- oder Erdalkalimetallacetate, wie Natriumacetat, Kaliumacetat, Calciumacetat oder Ammoniumacetat, sowie tertiäre Amine, wie Trimethylamin, Triethylamin, Tributylamin, N,N-Dimethylanilin, Pyridin, Piperidin, N-Methylpiperidin, N,N-Dimethylaminopyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens (a) in einem größeren Bereich variiert werden. Im allgemeinen arbeitet man bei Temperaturen zwischen 0°C und +180°C, vorzugsweise bei Temperaturen zwischen +20°C und +120°C.

Das erfindungsgemäße Verfahren (a) wird üblicherweise unter Normaldruck durchgeführt. Es ist jedoch auch möglich unter erhöhtem oder vermindertem Druck zu arbeiten.

Zur Durchführung des erfindungsgemäßen Verfahrens (a) setzt man pro Mol 1H-Triazolinon der Formel (II) im allgemeinen 1,0 bis 3,0 Mol, vorzugsweise 1,0 bis 1,5 Mol Halogenbenzol-Derivat der Formel (III) und gegebenenfalls 1,0 bis 3,0 Mol, vorzugsweise 1,0 bis 1,5 Mol Base als Reaktionshilfsmittel ein. Die Reaktionsdurchführung, Aufarbeitung und Isolierung der Reaktionsprodukte erfolgt nach allgemein üblichen, bekannten Verfahren (vergleiche auch die Herstellungsbeispiele).

Als Verdünnungsmittel zur Durchführung des erfindungsgemäßen Verfahrens (b) kommen inerte organische Lösungsmittel infrage. Vorzugsweise verwendet man die bei der Beschreibung der Durchführung des erfindungsgemäßen Verfahrens (a) aufgezählten Lösungsmittel.

Das erfindungsgemäße Verfahren (b) wird vorzugsweise in Gegenwart eines geeigneten Reaktionshilfsmittels durchgeführt. Als solche kommen alle üblichen anorganischen oder organischen Basen infrage. Hierzu gehören beispielsweise Erdalkali- oder Alkalimetallhydride, -hydroxide, -amide, -alkoholate, -acetate, -carbonate oder -hydrogencarbonate, wie beispielsweise Natriumhydrid, Natriumamid, Natriummethylat, Natriumethylat, Kalium-tert-butylat, Natriumhydroxid, Kaliumhydroxid, Ammoniumhydroxid, Natriumacetat, Kaliumacetat, Calciumacetat, Ammoniumacetat, Natriumcarbonat, Kaliumcarbonat, Kaliumhydrogencarbonat, Natriumhydrogencarbonat oder Ammoniumcarbonat sowie tertiäre Amine, wie Trimethylamin, Triethylamin, Tributylamin, N,N-Dimethylanilin, Pyridin, N-Methylpiperidin, N,N-Dimethylaminopyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens (b) in einem größeren Bereich variiert werden. Im allgemeinen arbeitet man bei Temperaturen zwischen -20°C und +150°C, vorzugsweise bei Temperaturen zwischen 0°C und +120°C.

Das erfindungsgemäße Verfahren (b) wird üblicherweise unter Normaldruck durchgeführt. Es ist jedoch auch möglich unter erhöhtem oder vermindertem Druck zu arbeiten.

Zur Durchführung des erfindungsgemäßen Verfahrens (b) setzt man pro Mol substituiertem Triazolinon der Formel (Ia) im allgemeinen 1,0 bis 3,0 Mol, vorzugsweise 1,0 bis 1,5 Mol Nukleophil der Formel (IV) und gegebenenfalls 0,1 bis 3,0 Mol, vorzugsweise 1,0 bis 1,5 Mol Base als Reaktionshilfsmittel ein.
Die Reaktionsdurchführung, Aufarbeitung und Isolierung der Reaktionsprodukte erfolgt nach allgemein üblichen, bekannten Verfahren.

Als Verdünnungsmittel zur Durchführung des erfindungsgemäßen Verfahrens (c) kommen inerte organische Lösungsmittel infrage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Dichlorbenzol, Petrolether, Hexan, Cyclohexan, Dichlormethan, Chloroform, Tetrachlorkohlenstoff; Ether, wie Diethylether, Diisopropylether, Dioxan, Tetrahydrofuran oder Ethylenglykoldimethyl- oder -diethylether; Nitrile, wie Acetonitril, Propionitril oder Benzonitril; Amide, wie N,N-Dimethylformamid, N,N-Dimethylacetamid, N-Methylformanilid, N-Methylpyrrolidon oder Hexamethylphosphorsäuretriamid; Ester wie Essigsäuremethylester oder Essigsäureethylester oder Sulfoxide, wie Dimethylsulfoxid.

Das erfindungsgemäße Verfahren (c) kann gegebenenfalls auch in einem Zweiphasensystem, wie beispielsweise Wasser/Toluol oder Wasser/Dichlormethan, gegebenenfalls in Gegenwart eines geeigneten Phasentransferkatalysators, durchgeführt werden. Als Beispiele für solche Katalysatoren seien genannt: Tetrabutylammoniumiodid, Tetrabutylammoniumbromid, Tetrabutylammoniumchlorid, Tributyl-methylphosphoniumbromid, Trimethyl-C₁₃/C₁₅-alkylammoniumchlorid, Trimethyl-C₁₃/C₁₅-alkylammoniumbromid, Dibenzyl-dimethy-ammoniummethylsulfat, Dimethyl-C₁₂/C₁₄-alkyl-benzylammoniumchlorid, Dimethyl-C₁₂/C₁₄-alkyl-benzylammoniumbromid, Tetrabutylammoniumhydroxid, Triethylbenzylammoniumchlorid, Methyltrioctylammoniumchlorid, Trimethylbenzylammoniumchlorid, 15-Krone-5, 18-Krone-6 oder Tris-[2-(2-methoxyethoxy)-ethyl]-amin.

Das erfindungsgemäße Verfahren (c) wird vorzugsweise in Gegenwart eines geeigneten Reaktionshilfsmittels durchgeführt. Als solche kommen alle üblichen anorganischen oder organischen Basen infrage. Hierzu gehören beispielsweise Erdalkali- oder Alkalimetallhydride, -hydroxide, -amide, -alkoholate, -acetate, -carbonate oder -hydrogencarbonate, wie beispielsweise Natriumhydrid, Natriumamid, Natriummethylat, Natriumethylat, Kalium-tert.-butylat, Natriumhydroxid, Kaliumhydroxid, Ammoniumhydroxid, Natriumacetat, Kaliumacetat, Calciumacetat, Ammoniumacetat, Natriumcarbonat, Kaliumcarbonat, Kaliumhydrogencarbonat, Natriumhydrogencarbonat oder Ammoniumcarbonat sowie tertiäre Amine, wie Trimethylamin, Triethylamin, Tributylamin, N,N-Dimethylanilin, Pyridin, N-Methylpiperidin, N,N-Dimethylaminopyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens (c) in einem größeren Bereich variiert werden. Im allgemeinen arbeitet man bei Temperaturen zwischen -20°C und +150°C, vorzugsweise bei Temperaturen zwischen 0°C und +120°C.

Das erfindungsgemäße Verfahren (c) wird üblicherweise unter Normaldruck durchgeführt. Es ist jedoch auch möglich unter erhöhtem oder vermindertem Druck zu arbeiten.

Zur Durchführung des erfindungsgemäßen Verfahrens (c) setzt man pro Mol substituiertem Triazolinon der Formel (V) im allgemeinen 1,0 bis 3,0 Mol, vorzugsweise 1,0 bis 2,0 Mol Akylierungs-, Acylierungs oder Sulfonylierungsmittel der Formel (VI) und gegebenenfalls 1,0 bis 3,0 Mol, vorzugsweise 1,0 bis 2,0 Mol Base als Reaktionshilfsmittel ein.
Die Reaktionsdurchführung, Aufarbeitung und Isolierung der Reaktionsprodukte erfolgt in beiden Fällen nach allgemein üblichen, bekannten Verfahren.

Als Reduktionsmittel zur Durchführung des erfindungsgemäßen Verfahrens (d) kommen übliche Reduktionsmittel infrage. Mit besonderem Vorzug verwendet man komplexe Hydride wie beispielsweise Lithiumaluminiumhydrid oder Natriumborhydrid.

Als Verdünnungsmittel zur Durchführung des erfindungsgemäßen Verfahrens (d) kommen in Abhängigkeit vom verwendeten Reduktionsmittel übliche organische oder anorganische Lösungsmittel infrage. Vorzugsweise verwendet man Alkohole, wie Methanol, Ethanol, Propanol oder Butanol, Etheralkohole, wie Methoxyethanol oder Ethoxyethanol, Ether, wie Diethylether, Diisopropylether, Dioxan oder Tetrahydrofuran, deren Gemische mit Wasser oder reines Wasser als Verdünnungsmittel.

Die Reaktionstemperaturen können bei der Durchführung des erfindungsgemäßen Verfahrens (d) in einem größeren Bereich variiert werden. Im allgemeinen arbeitet man bei Temperaturen zwischen -20°C und +100°C, vorzugsweise bei Temperaturen zwischen 0°C und +80°C.

Das erfindungsgemäße Verfahrens (d) wird üblicherweise unter Normaldruck durchgeführt. Es ist jedoch auch möglich unter erhöhtem oder vermindertem Druck zu arbeiten.

Zur Durchführung des erfindungsgemäßen Verfahrens (d) setzt man pro Mol 4-Alkylidenimino-triazolinon der Formel (VII) im allgemeinen 0,5 bis 5,0 Mol, vorzugsweise 1,0 bis 3,0 Mol Reduktionsmittel ein. Die Reaktionsdurchführung, Aufarbeitung und Isolierung der Reaktionsprodukte erfolgt nach allgemein üblichen, bekannten Verfahren.

Die Reinigung der Endprodukte der Formel (I) erfolgt mit Hilfe üblicher Verfahren, beispielsweise durch Säulenchromatographie oder durch Umkristallisieren.
Die Charakterisierung erfolgt mit Hilfe des Schmelzpunktes oder bei nicht kristallisierenden Verbindungen mit Hilfe der Protonen-Kernresonanzspektroskopie (¹H-NMR).

Die erfindungsgemäßen Wirkstoffe können als Defoliants, Desiccants, Krautabtötungsmittel und insbesondere als Unkrautvernichtungsmittel verwendet werden.Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewandten Menge ab.
Die erfindungsgemäßen Wirkstoffe können z.B. bei den folgenden Pflanzen verwendet werden:
Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea, Trifolium, Ranunculus, Taraxacum.
Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.
Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.
Monokotyle Kulturen der Gattungen: Oryza, Zea, Triticum, Hordeum, Avena, Secale, Sorghum, Panicum, Saccharum, Ananas, Asparagus, Allium.
Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Zitrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Dabei lassen sich die erfindungsgemäßen Wirkstoffe mit besonders gutem Erfolg zur Bekämpfung von dikotylen Unkräutern in mono- und dikotylen Kulturen wie beispielsweise Soja, Sonnenblumen oder Gerste einsetzen. Daneben besitzen die erfindungsgemäßen Wirstoffe in entsprechenden Aufwandmengen auch fungizide Wirksamkeit und lassen sich zur Bekämpfung von Reiskrankheiten, wie beispielsweise gegen den Erreger der Reisfleckenkrankheit (Pyricularia oryzae) einsetzen.

Die Wirkstoffe können in Abhängigkeit von ihren jeweiligen physikalischen und/oder chemischen Eigenschaften in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen infrage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen infrage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen infrage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen infragen: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen infrage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zinn verwendet werden.

Die Formulierungen enthalten im allgemeinen zwischen 0,1 und 95 Gewichtsprozent Wirkstoff, vorzugsweise zwischen 0,5 und 90%.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.
Für die Mischungen kommen bekannte Herbizide infrage, beispielsweise Anilide, wie z.B. Diflufenican und Propanil; Arylcarbonsäuren, wie z.B. Dichlorpicolinsäure, Dicamba oder Picloram; Aryloxyalkansäuren, wie z.B. 2,4-D, 2,4-DB, 2,4-DP, Fluroxypyr, MCPA, MCPP und Triclopyr; Aryloxy-phenoxy-alkansäureester, wie z.B. Diclofop-methyl, Fenoxaprop-ethyl, Fluazifop-butyl, Haloxyfop-methyl und Quizalofop-ethyl; Azinone, wie z.B. Chloridazon und Norflurazon; Carbamate, wie z.B. Chlorpropham, Desmedipham, Phenmedipham und Propham; Chloracetanilide, wie z.B. Alachlor, Acetochlor, Butachlor, Metazachlor, Metolachlor, Pretilachlor und Propachlor; Dinitroaniline, wie z.B. Oryzalin, Pendimethalin und Trifluralin; Diphenylether, wie z.B. Acifluorfen, Bifenox, Fluoroglycofen, Fomesafen, Halosafen, Lactofen und Oxyfluorfen; Harnstoffe, wie z.B. Chlortoluron, Diuron, Fluometuron, Isoproturon, Linuron und Methabenzthiazuron; Hydroxylamine, wie z.B. Alloxydim, Clethodim, Cycloxydim, Sethoxydim und Tralkoxydim; Imidazolinone, wie z.B. Imazethapyr, Imazamethabenz, Imazapyr und Imazaquin; Nitrile, wie z.B. Bromoxynil, Dichlobenil und Ioxynil; Oxyacetamide, wie z.B. Mefenacet; Sulfonylharnstoffe, wie z.B. Amidosulfuron, Bensulfuron-methyl, Chlorimuron-ethyl, Chlorsulfuron, Cinosulfuron, Metsulfuron-methyl, Nicosulfuron, Primisulfuron, Pyrazosulfuron-ethyl, Thifensulfuron-methyl, Triasulfuron und Tribenuron-methyl; Thiolcarbamate, wie z.B. Butylate, Cycloate, Diallate, EPTC, Esprocarb, Molinate, Prosulfocarb, Thiobencarb und Triallate; Triazine, wie z.B. Atrazin, Cyanazin, Simazin,Simetryne, Terbutryne und Terbutylazin; Triazinone, wie z.B. Hexazinon, Metamitron und Metribuzin; Sonstige, wie z.B. Aminotriazol, Benfuresate, Bentazone, Cinmethylin, Clomazone, Clopyralid, Difenzoquat, Dithiopyr, Ethofumesate, Fluorochloridone, Glufosinate, Glyphosate, Isoxaben, Pyridate, Quinchlorac, Quinmerac, Sulphosate und Tridiphane.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können dabei als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen; Sprühen, Streuen.

Die erfindungsgemäßen Wirkstoffe können sowohl vor als auch nach dem Auflaufen der Pflanzen appliziert werden. Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 10 kg Wirkstoff pro Hektar Bodenfläche, vorzugsweise zwischen 0,05 und 5 kg pro Hektar.

Die Herstellung und die Verwendung der erfindungsgemäßen Wirkstoffe geht aus den nachfolgenden Beispielen hervor.

### Herstellungsbeispiele:

### Beispiel 1:

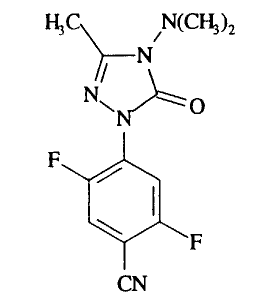

### (Verfahren a)

71 g (0,5 Mol) 4-Dimethylamino-3-methyl-1H-1,2,4-triazolin-5-on (vergl. z.B. EP 422 469) und 78,5 g (0,5 Mol) 2,4,5-Trifluorbenzonitril (vergl. z.B. EP 191 181) werden in 400 ml Dimethylsulfoxid bei Raumtemperatur mit 83 g (0,06 Mol) Kaliumcarbonat versetzt und anschließend zwei Stunden bei 40°C bis 50°C gerührt. Zur Aufarbeitung wird die abgekühlte Reaktionsmischung filtriert, das Filtrat im Vakuum eingeengt, der Rückstand mit Wasser verrührt, der ausgefallene Feststoff abgesaugt, mit Wasser gewaschen und getrocknet.

Man erhält 111 g (80 % der Theorie) 1-(4-Cyano-2,5-difluorphenyl)-3-methyl-4-dimethylamino-1,2,4-triazolin-5-on vom Schmelzpunkt 116°C.

### Beispiel 2:

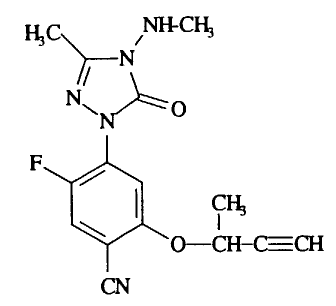

### (Verfahren b)

Zu 1,05 g (0,015 Mol) 3-Butin-1-ol in 100 ml Acetonitril gibt man bei Raumtemperatur 0,6 g (0,015 Mol) Natriumhydrid (60% in Paraffinöl), rührt 10 Minuten bei Raumtemperatur, gibt anschließend 2,12 g (0,008 Mol) 1-(4-Cyano-2,5-difluorphenyl)-3-methyl-4-(N-methylamino)-1,2,4-triazolin-5-on zu und rührt weitere 16 Stunden bei Raumtemperatur. Zur Aufarbeitung wird die Reaktionsmischung filtriert, das Filtrat im Vakuum eingeengt, der Rückstand mit Wasser verrührt, der ausgefallene Feststoff abgesaugt, mit Wasser gewaschen und getrocknet.

Man erhalt 1,96 g (78 % der Theorie) 1-(4-Cyano-2-fluor-5-but-1-in-3-yl-oxyphenyl)-3-methyl-4-(N-methylamino)-1,2,4-triazolin-5-on vom Schmelzpunkt 184-185°C.

### Beispiel 3:

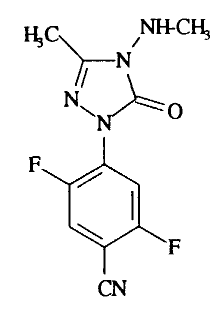

### (Verfahren a)

12,8 g (0,1 Mol) 4-(N-Methylamino)-3-methy-1H-1,2,4-triazolin-5-on (vergl. z.B. EP 399 294) und 15,7 g (0,1 Mol) 2,4,5-Trifluorbenzonitril (vergl. z.B. EP 191 181) werden in 200 ml Dimethylsulfoxid bei Raumtemperatur mit 16,5 g (0,12 Mol) Kaliumcarbonat versetzt und anschließend drei Stunden bei 40°C bis 50°C gerührt. Zur Aufarbeitung wird die abgekühlte Reaktionsmischung filtriert, das Filtrat im Vakuum eingeengt, der Rückstand mit Wasser verrührt, der ausgefallene Feststoff abgesaugt, mit Wasser gewaschen und getrocknet.

Man erhält 12,8 g (48 % der Theorie) 1-(4-Cyano-2,5-difluor-phenyl)-3-methyl-4-(N-methylamino)-1,2,4-triazolin-5-on vom Schmelzpunkt 128-131°C.

### Beispiel 4:

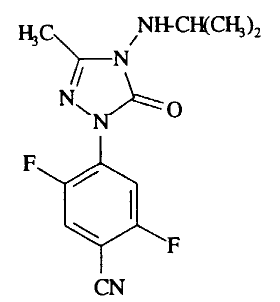

### (Verfahren a)

1,9 g (0,012 Mol) 3-Methyl-4-(N-isopropylamino)-1H-1,2,4-triazolin-5-on (Herstellung analog EP 399 294) und 1,9 g (0,012 Mol) 2,4,5-Trifluorbenzonitril (vergl. z.B. EP 191 181) werden in 100 ml Dimethylsulfoxid bei Raumtemperatur mit 1,9 g (0,014 Mol) Kaliumcarbonat versetzt und anschließend zwei Stunden bei Raumtemperatur und 1,5 Stunden bei 40-50°C gerührt. Zur Aufarbeitung wird die abgekühlte Reaktionsmischung in Wasser gegeben, der ausgefallene Feststoff abgesaugt, mit Wasser gewaschen und getrocknet.

Man erhält 1,3 g (54,3 % der Theorie) 1-(4-Cyano-2,5-difluorphenyl)-3-methyl-4-(N-isopropylamino)-1,2,4-triazolin-5-on vom Schmelzpunkt 35-36°C.

### Herstellung der Ausgangsverbindungen:

### Beispiel III-1:

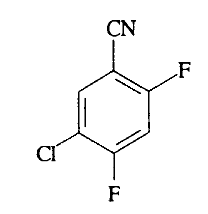

Zu 250 g (4,31 Mol) Kaliumfluorid in 400 ml destilliertem Tetramethylensulfon gibt man unter Rühren bei Raumtemperatur 220 g (1,06 Mol) 2,4,5-Trichlorbenzonitril (vergl. z.B. EP 441 004) und rührt anschließend 10 Stunden bei 195°C bis 200°C. Zur Aufarbeitung wird abgekühlt, 500 ml Wasser zugegeben und die Mischung einer Wasserdampfdestillation unterzogen. Der organische Anteil wird in Dichlormethan aufgenommen, über Natriumsulfat getrocknet, im Vakuum eingeengt und destilliert.

Man erhält 108 g (58 % der Theorie) 2,4-Difluor-5-chlorbenzonitril vom Siedepunkt 105-107°C bei 30 mbar und vom Schmelzpunkt 48-50°C.

In entsprechender Weise und gemäß den allgemeinen Angaben zur Herstellung erhält man die folgenden substituierten Triazolinone der allgemeinen Formel (I):

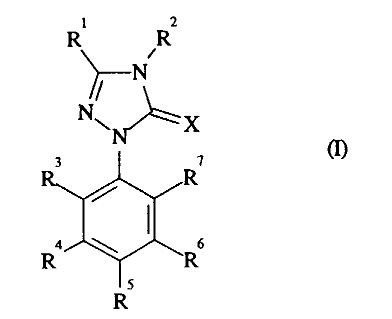

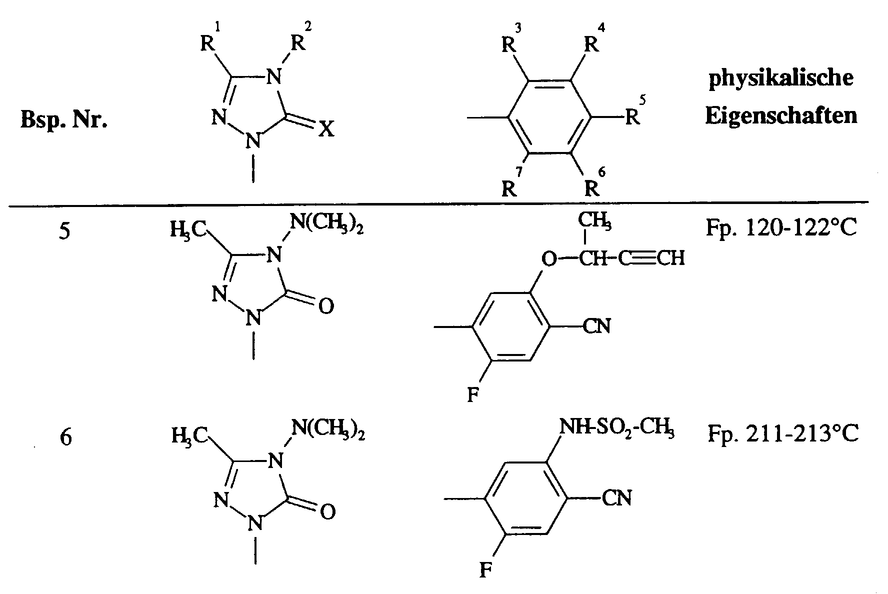

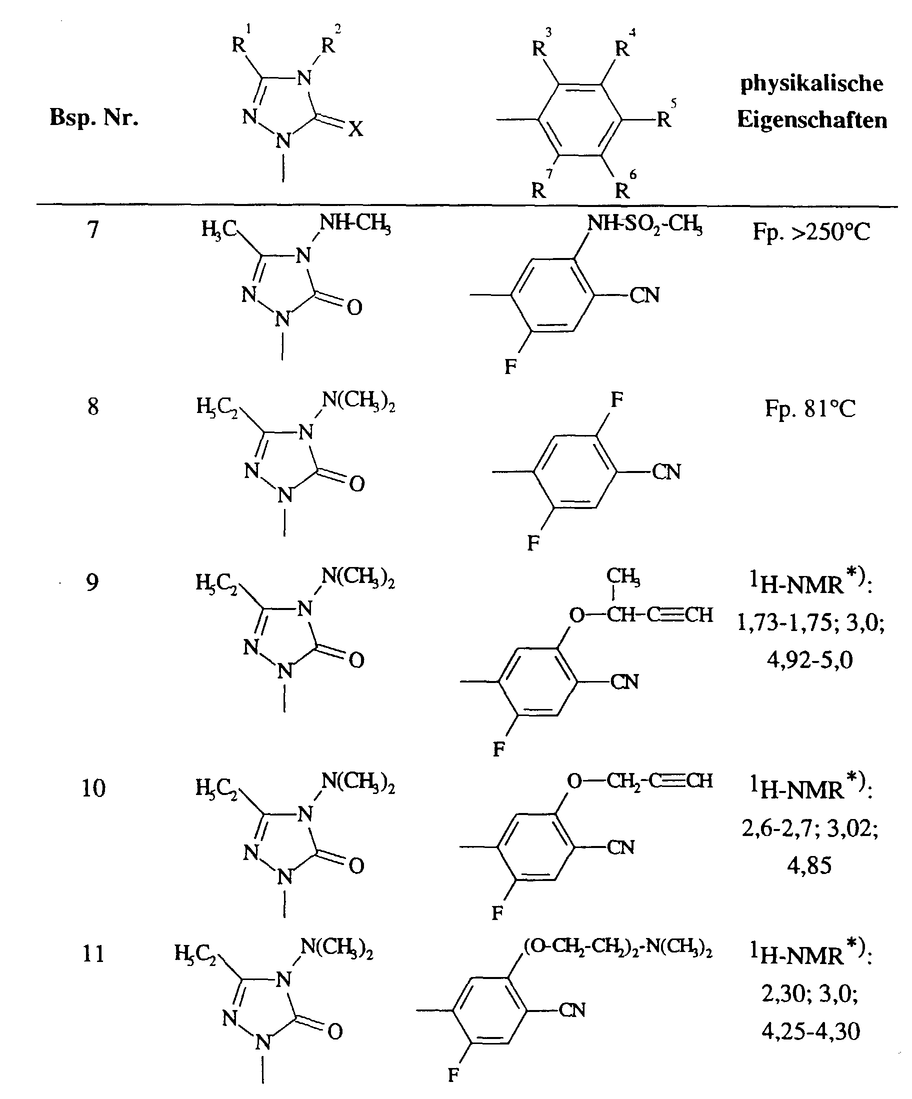

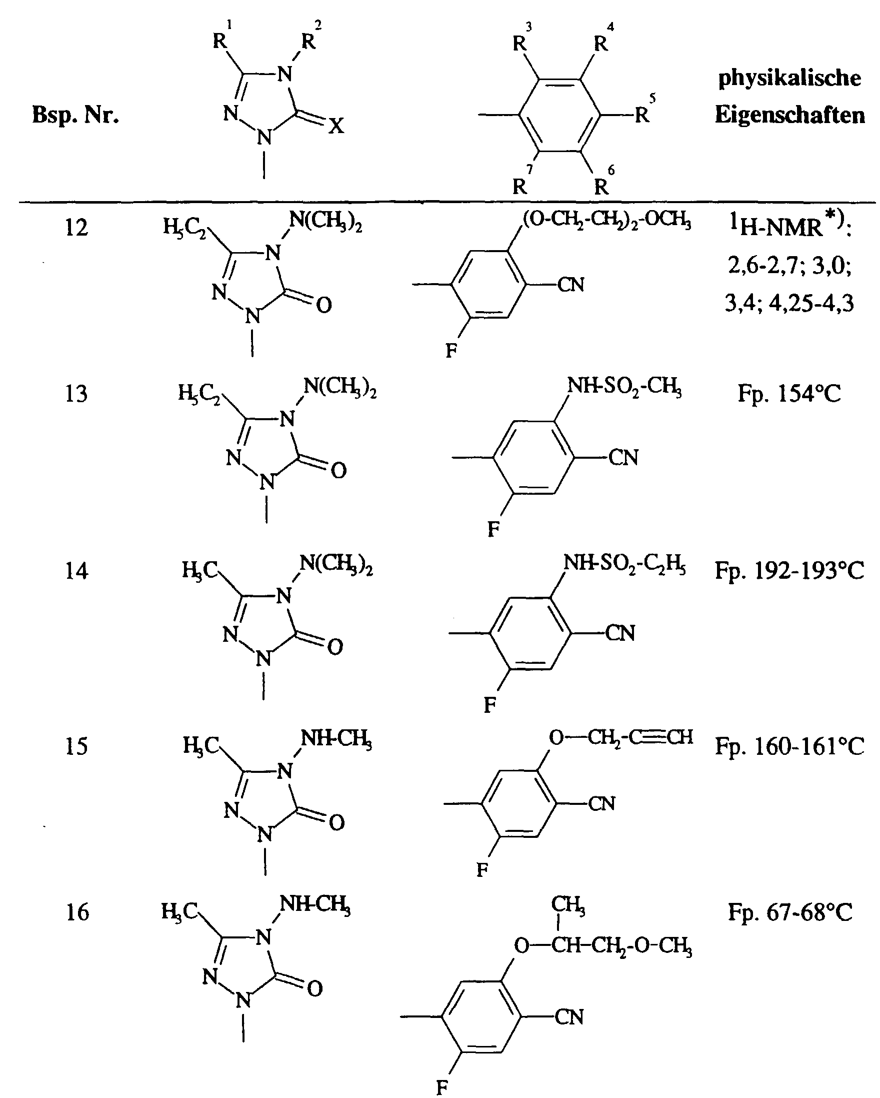

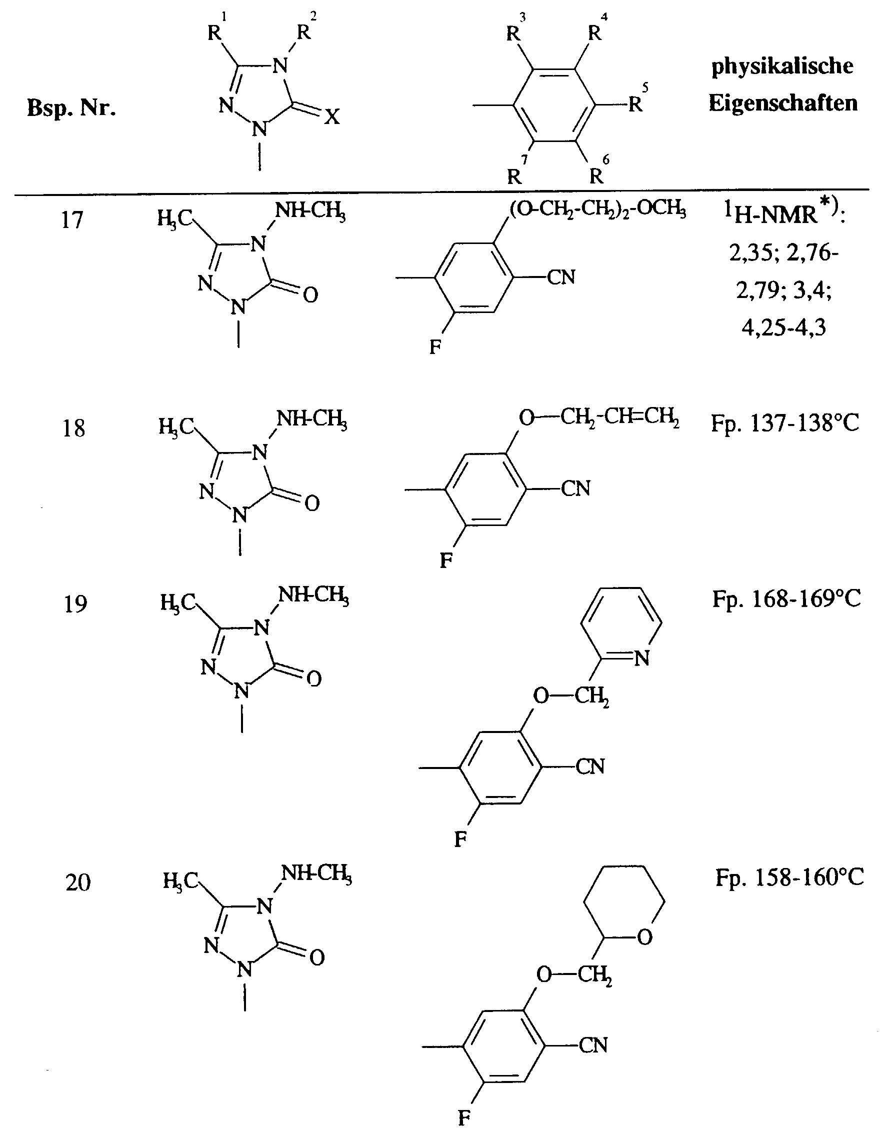

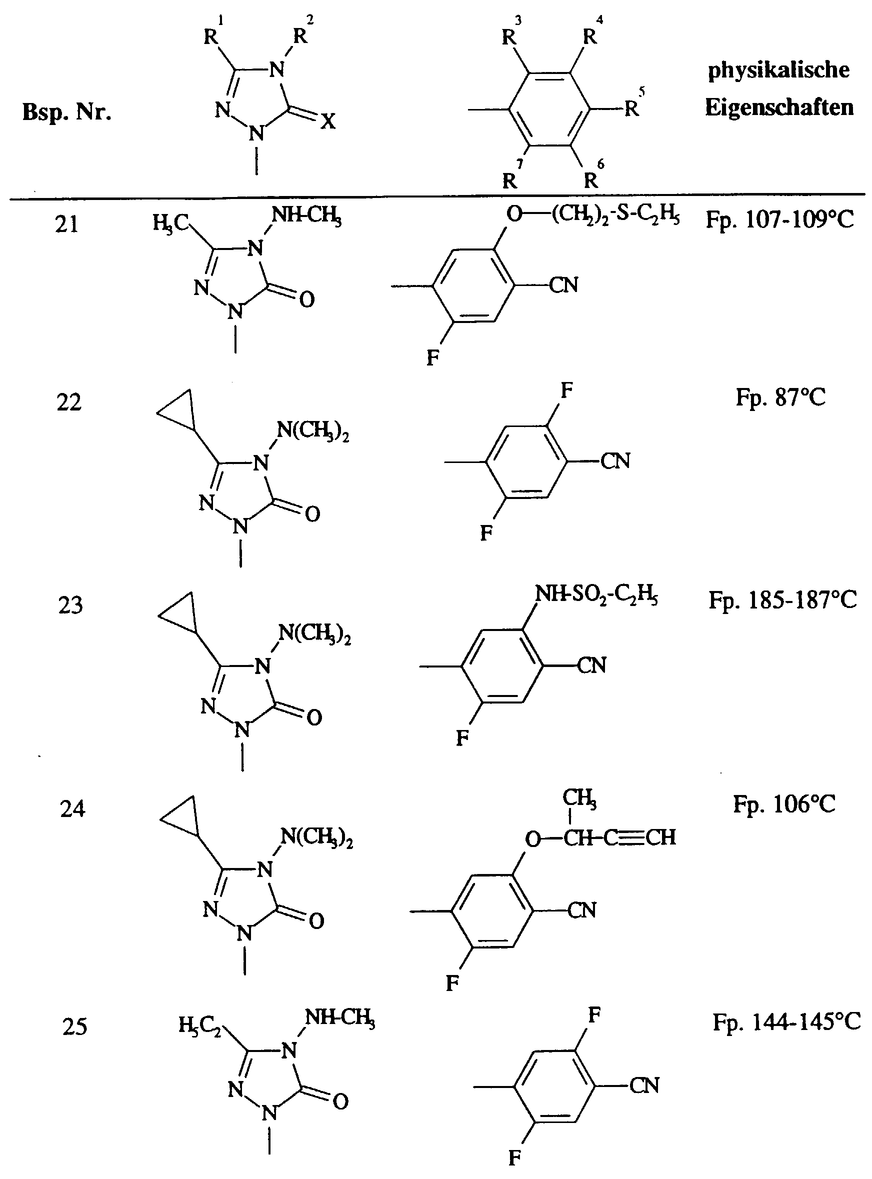

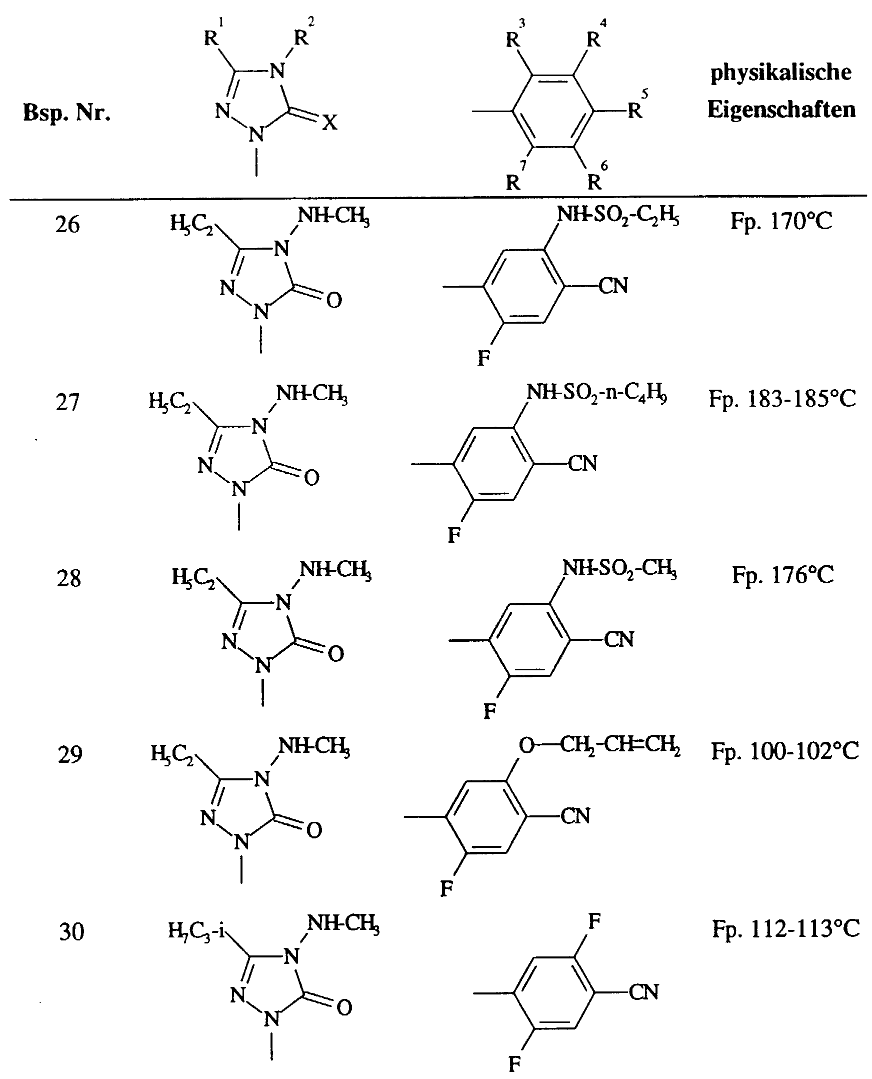

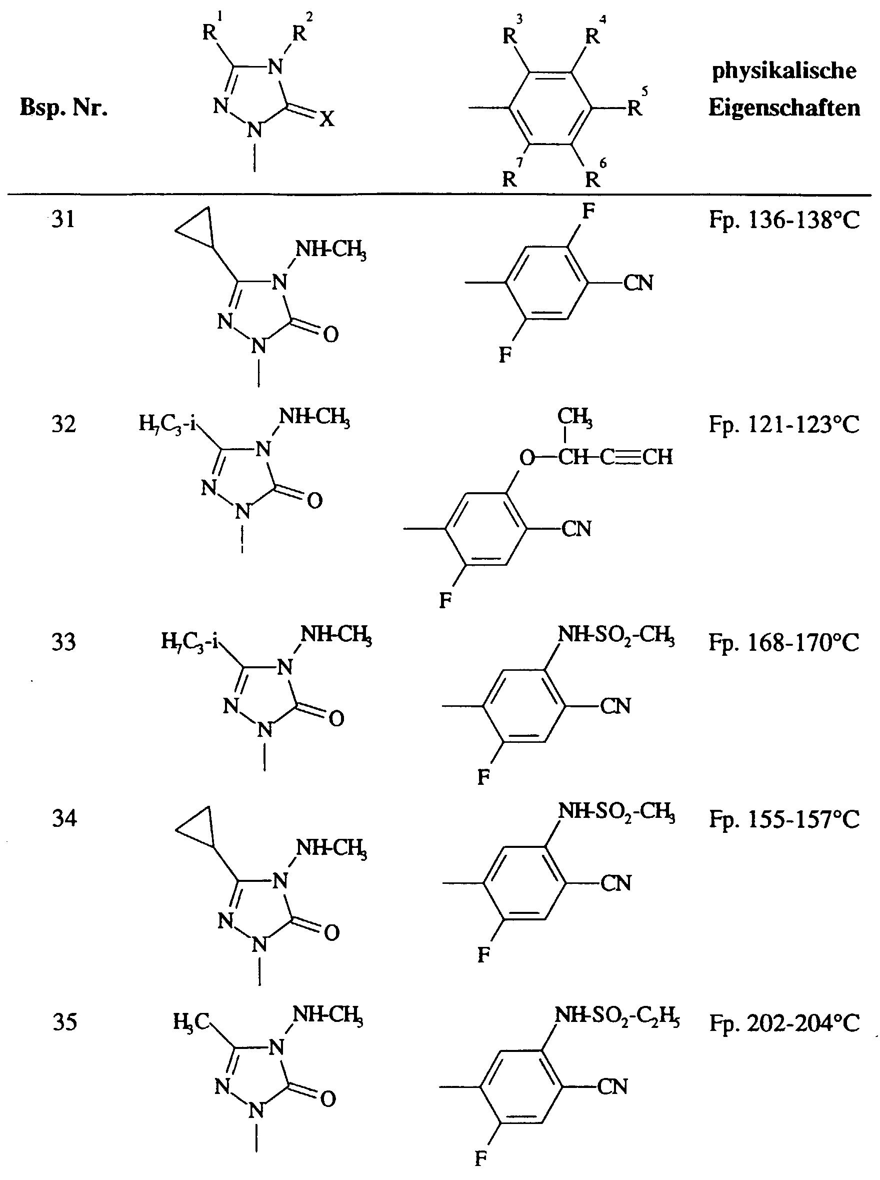

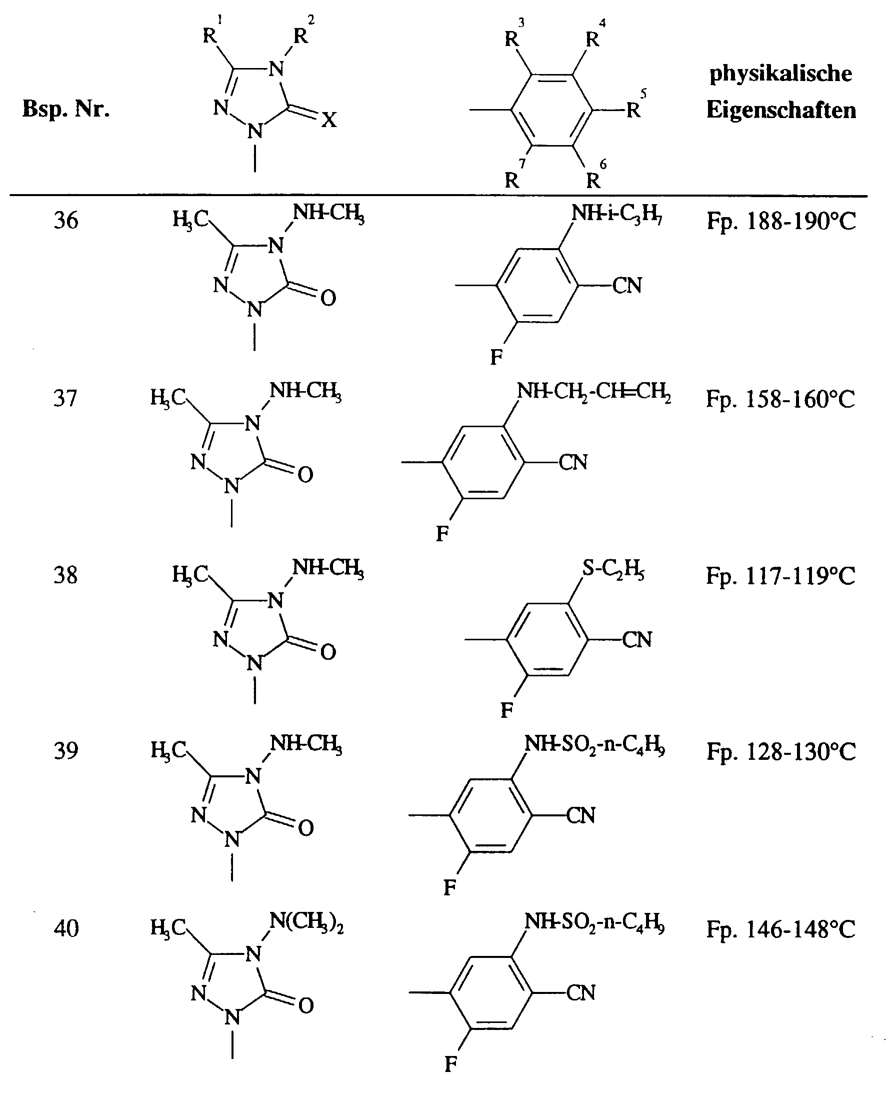

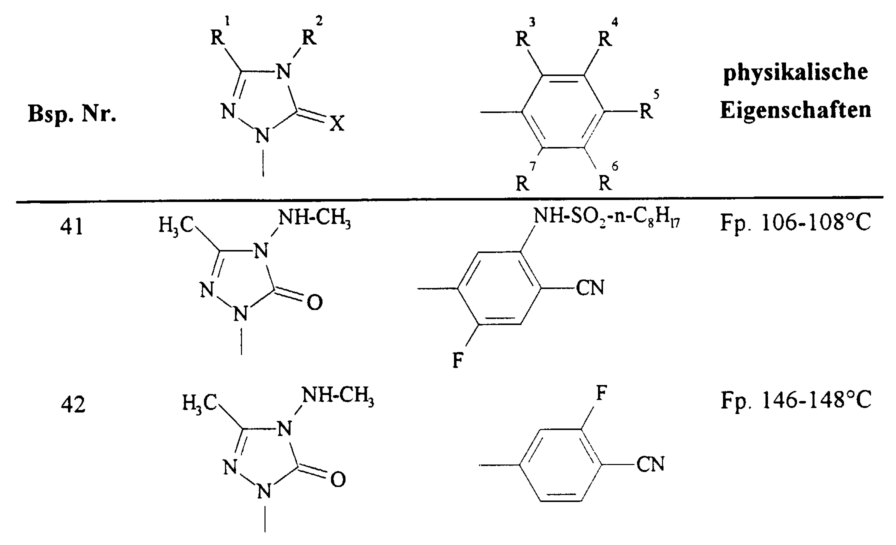

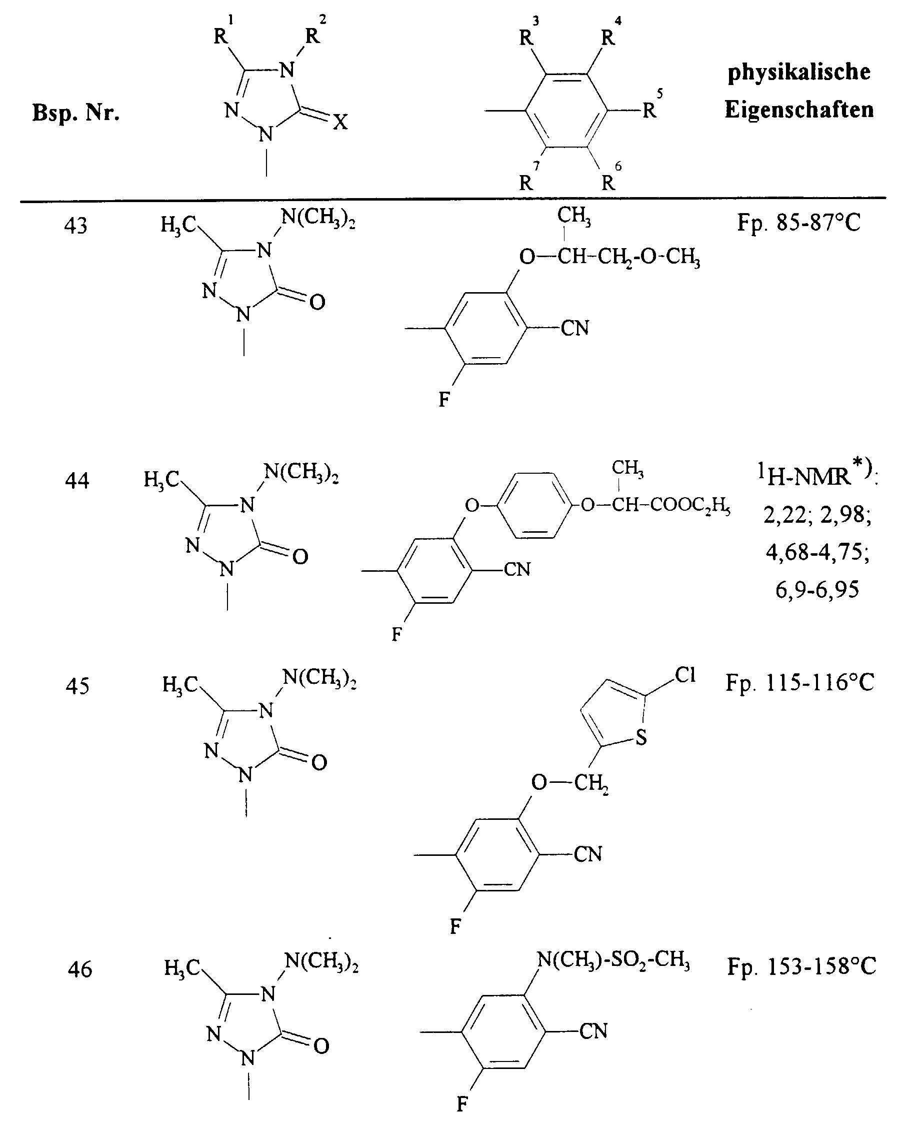

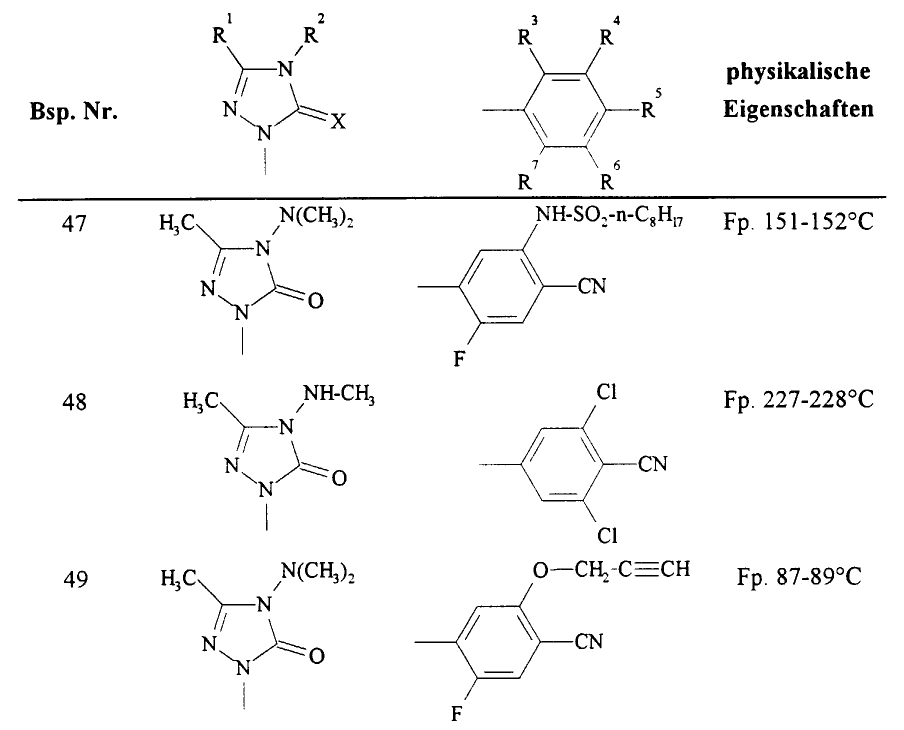

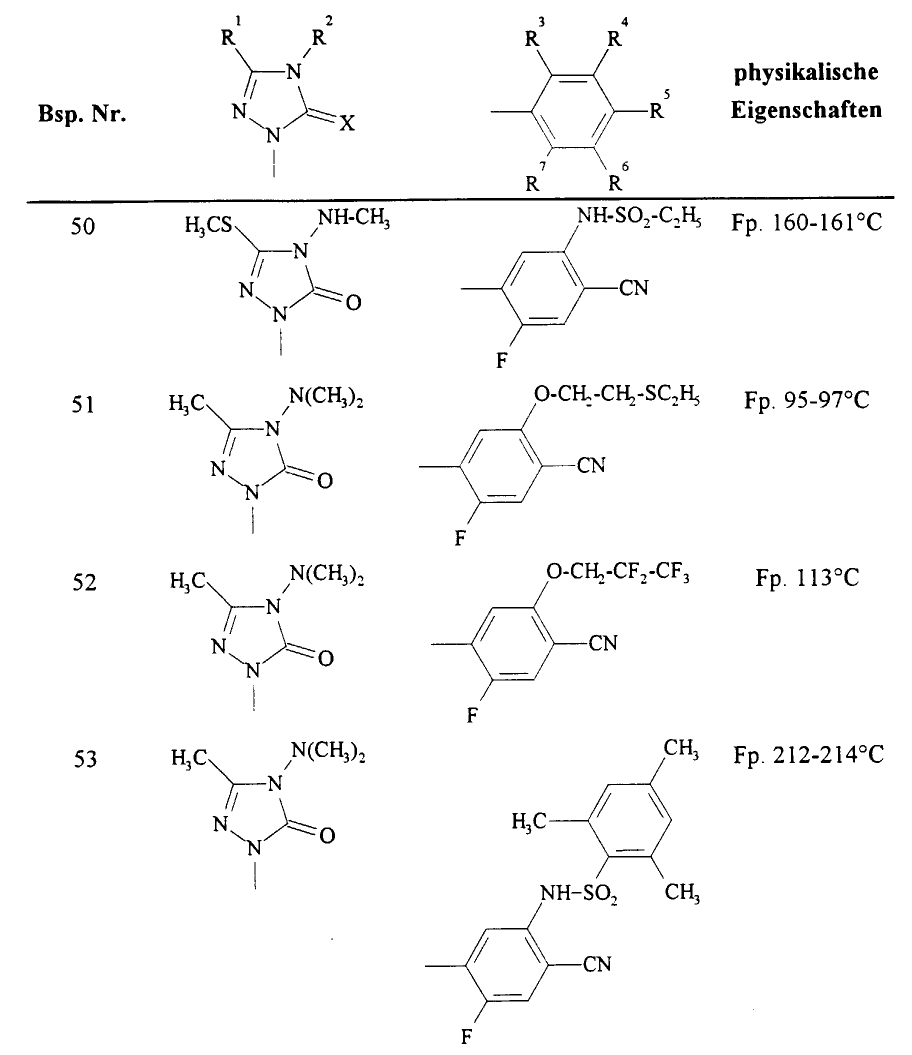

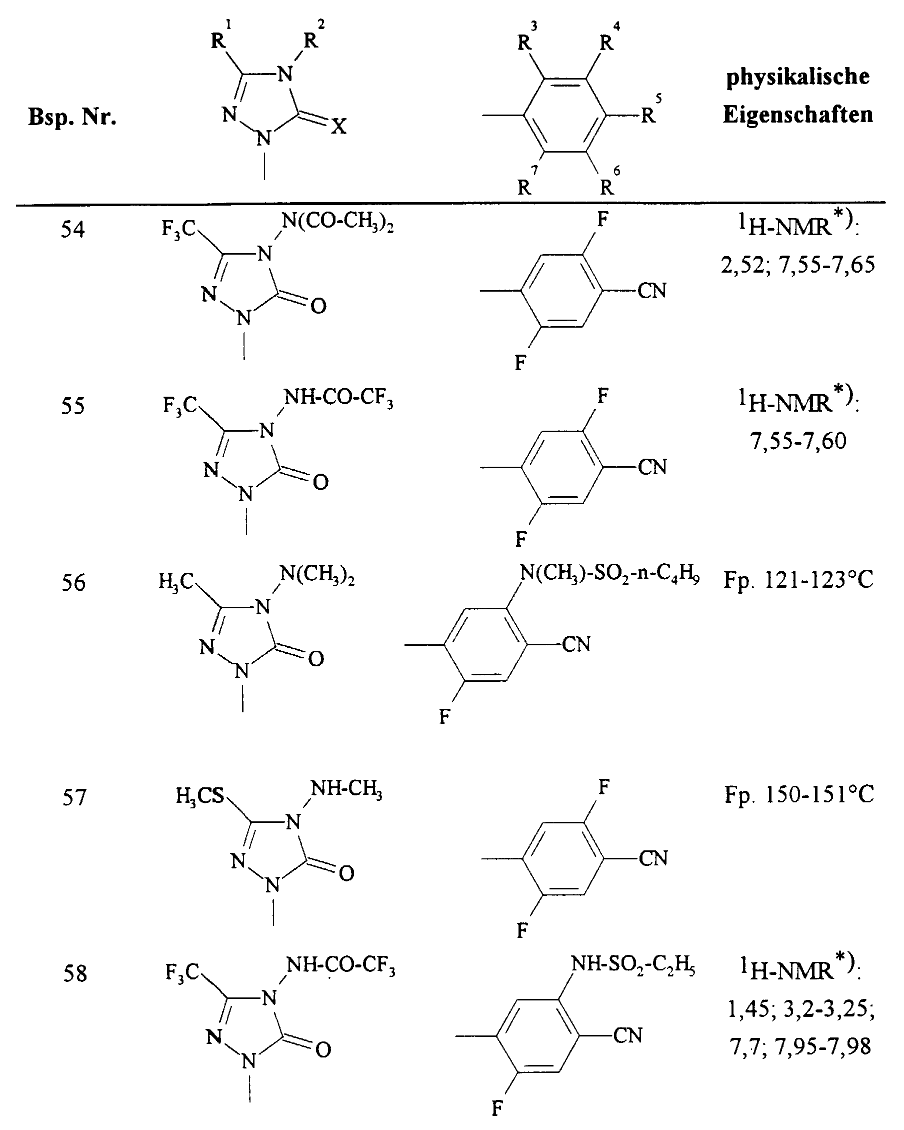

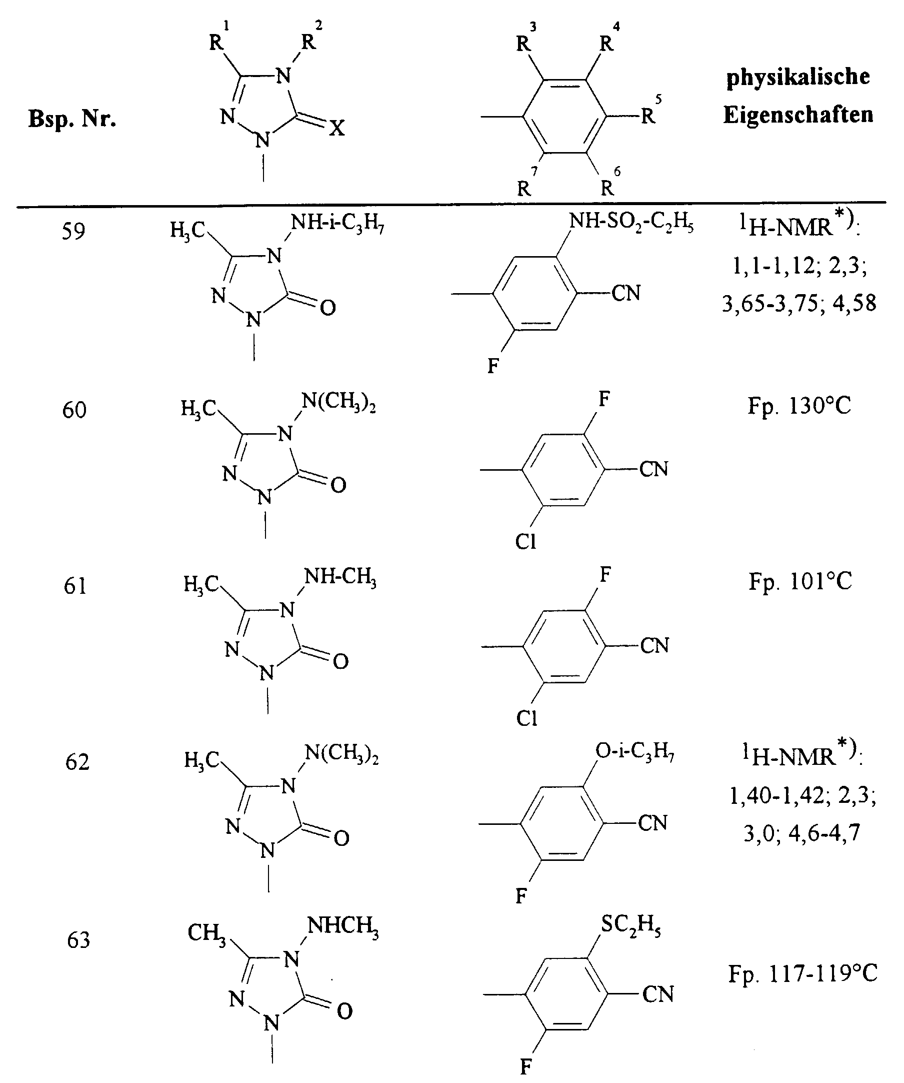

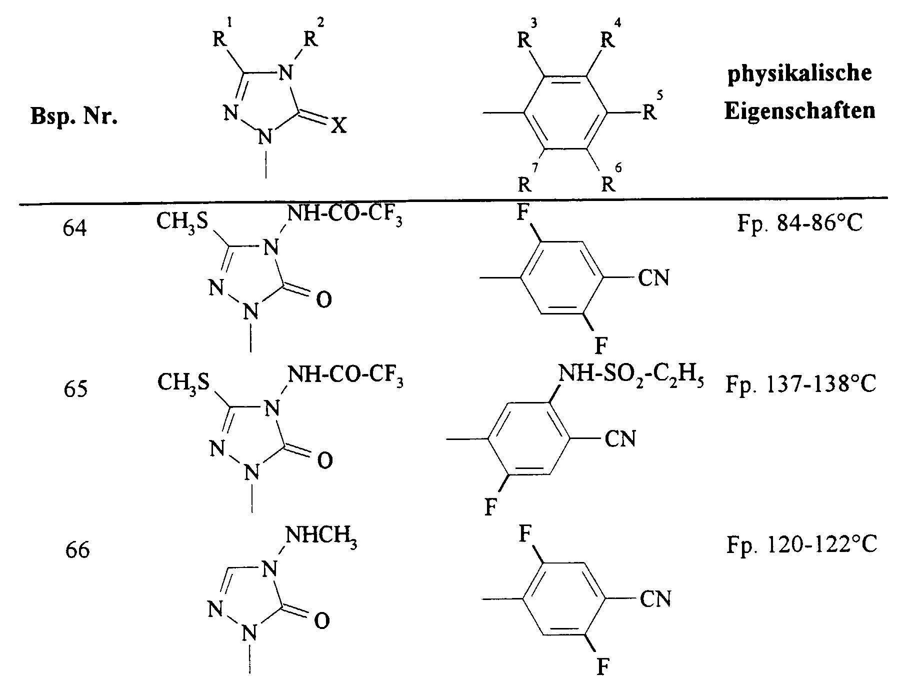

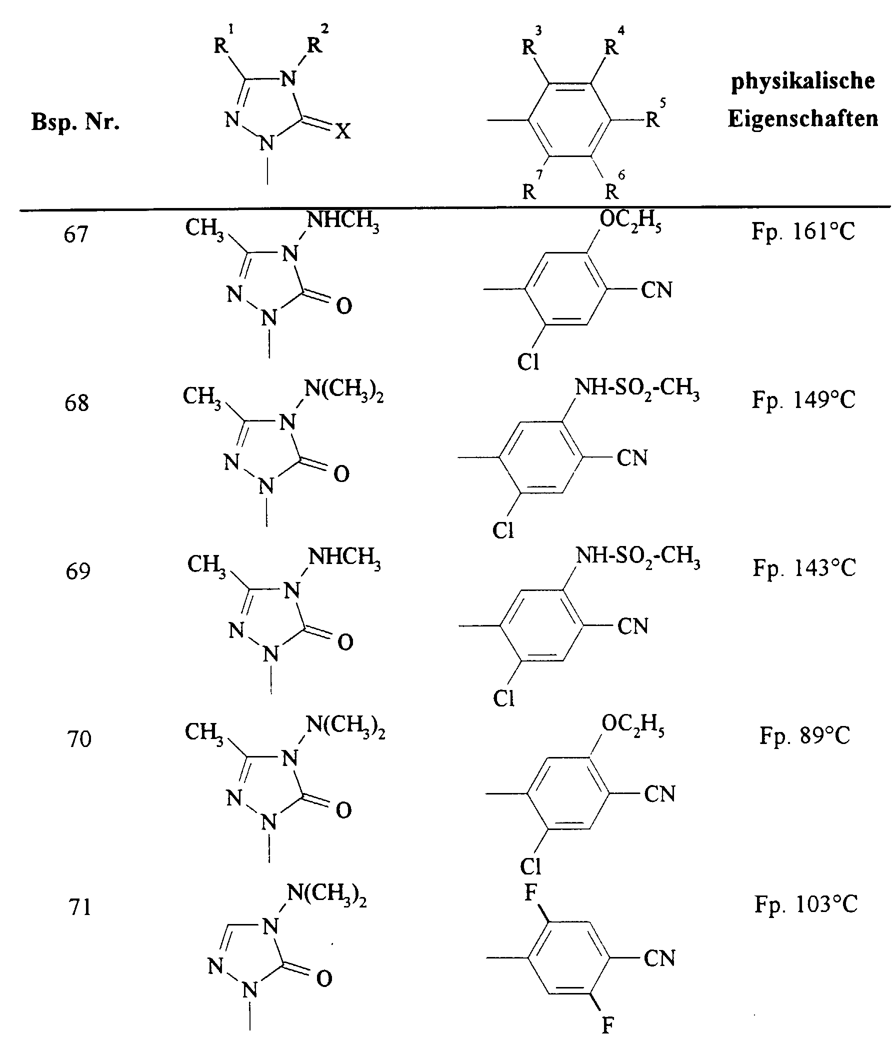

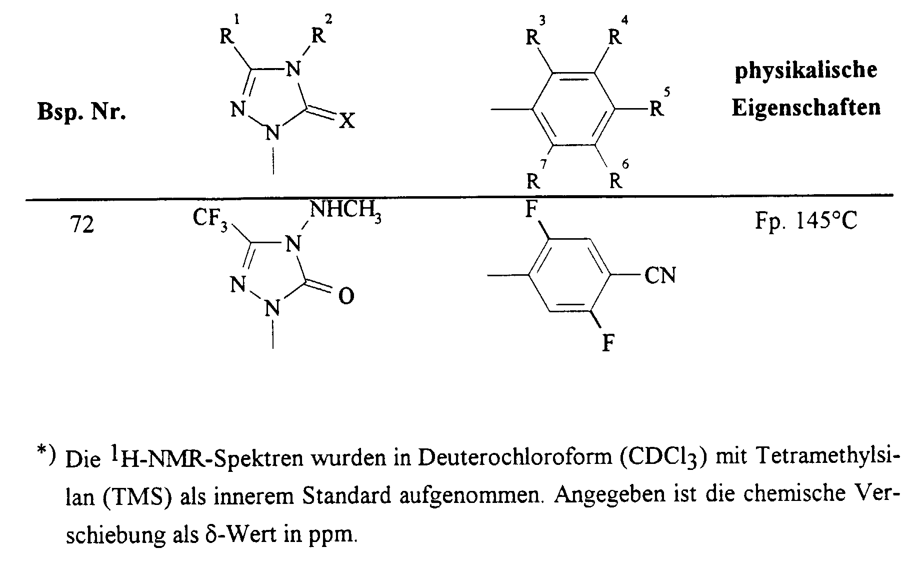

### Anwendungsbeispiele:

In dem folgenden Anwendungsbeispiel wurde die nachstehend aufgeführte Verbindung als Vergleichssubstanz eingesetzt:

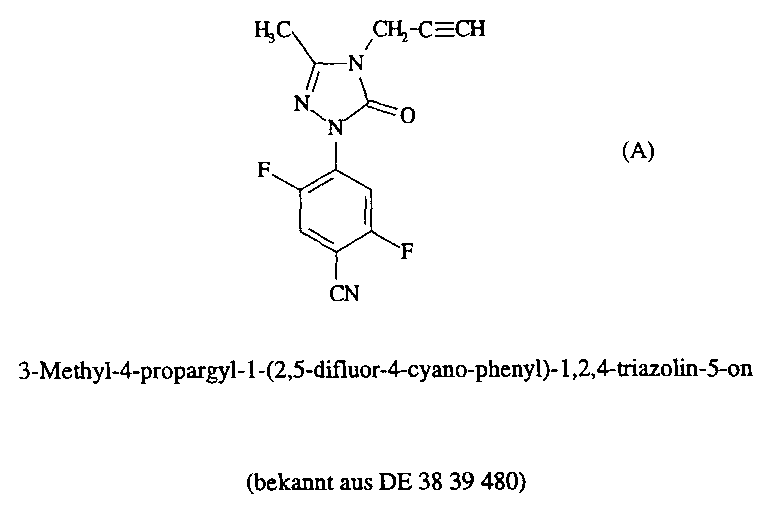

### Beispiel A:

### Pre-emergence-Test

- Lösungsmittel:: 5 Gewichtsteile Aceton
- Emulgator:: 1 Gewichtsteil Alkylarylpolyglykolether

Zur Herstellung einer zweckmäßigen Wirkstoffzubereitung vermischt man ein Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffes pro Flächeneinheit Nach drei Wochen wird der Schädigungsgrad der Pflanzen bonitiert in % Schädigung im Vergleich zur Entwicklung der unbehandelten Kontrolle.

Es bedeuten:
0 % = keine Wirkung (wie unbehandelte Kontrolle)
100 % = totale Vernichtung

Eine deutliche Überlegenheit in der Wirksamkeit gegenüber dem Stand der Technik (A) zeigen in diesem Test z.B. die Verbindungen gemäß den Herstellungsbeispielen 5 und 6 in Kulturen wie Soja, Sonnenblumen und Gerste bei Aufwandmengen von 250 g/ha (Soja 0 bis 30 %, Sonnenblumen 0 %, Gerste 0 bis 100 %) gegenüber Unkräutern wie Abuthilon (95 bis 100 %), Chenopodium (100 %), Galium (80 bis 95 %), Matricaria (95 bis 100 %) und Solanum (95 bis 100 %), auch wenn der Stand der Technik mit einer Aufwandmenge von 500 g/ha angewandt wird.

## Patentansprüche

1. Substituierte 1-Aryltriazolinone der allgemeinen Formel (I), 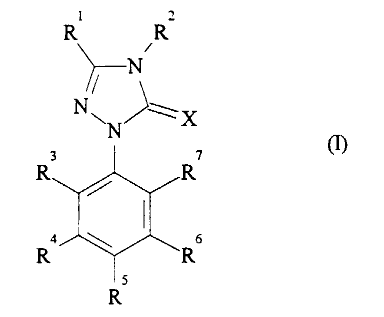 in welcher
R¹ für Wasserstoff oder für jeweils geradkettiges oder verzweigtes Alkyl, Alkoxy, Alkylthio oder Alkylsulfonyl mit jeweils 1 bis 8 Kohlenstoffatomen steht, außerdem für geradkettiges oder verzweigtes Halogenalkyl mit 1 bis 8 Kohlenstoffatomen und 1 bis 17 gleichen oder verschiedenen Halogenatomen steht oder für Cycloalkyl mit 3 bis 8 Kohlenstoffatomen steht,
R² für einen Rest der Formel -NR⁸R⁹steht,
R³, R⁶ jeweils für Wasserstoff stehen,
R⁷ für Wasserstoff, Halogen, Amino oder Nitro steht,
R⁴ für Wasserstoff, Fluor, Chlor, Brom, Iod, Cyano, Nitro, oder für einen der Reste -R¹⁰, -O-R¹⁰, -S-R¹⁰, -S(O)-R¹⁰, -SO₂-R¹⁰, -SO₂-OR¹⁰, -SO₂-NR¹¹R¹⁰, -CO-OR¹⁰, -CO-NR¹¹R¹⁰, -O-SO₂-R¹⁰, -N(R¹¹)-SO₂-R¹⁰, -NR¹¹R¹⁰, -NH-P(O)(R¹¹)(OR¹⁰) oder -NH-P(O)(OR¹¹)(OR¹⁰) steht,
R⁵ für Cyano steht und
X für Sauerstoff oder Schwefel steht, wobei
R⁸ für Wasserstoff, für geradkettiges oder verzweigtes Alkyl mit 1 bis 8 Kohlenstoffatomen oder für geradkettiges oder verzweigtes Halogenalkyl mit 1 bis 8 Kohlenstoffatomen und 1 bis 17 gleichen oder verschiedenen Halogenatomen steht und außerdem für einen Rest der Formel -CO-R¹² oder für einen Rest der Formel -S(O)ₙ-R¹² steht,
R⁹ für geradkettiges oder verzweigtes Alkyl mit 1 bis 8 Kohlenstoffatomen oder für geradkettiges oder verzweigtes Halogenalkyl mit 1 bis 8 Kohlenstoffatomen und 1 bis 17 gleichen oder verschiedenen Halogenatomen steht und außerdem für einen Rest der Formel -CO-R¹² oder für einen Rest der Formel -S(O)ₙ-R¹² steht,
R¹⁰ für Wasserstoff steht;
R¹⁰ außerdem für gegebenenfalls einfach oder mehrfach, gleich oder verschieden substituiertes, geradkettiges oder verzweigtes Alkyl mit 1 bis 14 Kohlenstoffatomen steht, wobei als Substituenten infrage kommen:
Halogen - insbesondere Fluor, Chlor, Brom und/oder Iod -, Cyano, Carboxyl, Carbamoyl, jeweils geradkettiges oder verzweigtes Alkoxy, Alkoxyalkoxy, Alkylthio, Alkylsulfinyl, Alkylsulfonyl, Alkoxycarbonyl, N-Alkylaminocarbonyl, N,N-Dialkylaminocarbonyl oder Alkylsulfonylaminocarbonyl mit jeweils 1 bis 8 Kohlenstoffatomen in den einzelnen Alkylteilen oder Heterocyclyl, wobei als Heterocyclylrest ein fünf- bis siebengliedriger, gegebenenfalls benzannellierter, gesättigter oder ungesättigter Heterocyclus mit 1 bis 3 gleichen oder verschiedenen Heteroatomen - insbesondere Stickstoff, Sauerstoffund/oder Schwefel - steht;
R¹⁰ außerdem für jeweils gegebenenfalls einfach oder mehrfach, gleich oder verschieden durch Halogen - insbesondere Fluor, Chlor, Brom und/oder Iod - substituiertes Alkenyl oder Alkinyl mit jeweils 2 bis 8 Kohlenstoffatomen steht;
R¹⁰ außerdem für gegebenenfalls einfach oder mehrfach, gleich oder verschieden durch Halogen - insbesondere Fluor, Chlor, Brom und/oder Iod - und/oder geradkettiges oder verzweigtes Alkyl mit 1 bis 4 Kohlenstoffatomen substituiertes Cycloalkyl mit 3 bis 7 Kohlenstoffatomen steht;
R¹⁰ außerdem für jeweils gegebenenfalls im Arylteil einfach oder mehrfach, gleich oder verschieden substituiertes Arylalkyl oder Aryl mit jeweils 6 bis 10 Kohlenstoffatomen im Arylteil und gegebenenfalls 1 bis 4 Kohlenstoffatomen im geradkettigen oder verzweigten Alkylteil steht, oder für einen gegebenenfalls einfach oder mehrfach, gleich oder verschieden substituierten und/oder benzannellierten, gesättigten oder ungesättigten, fünf- bis siebengliedrigen rest mit 1 bis 3 gleichen oder verschiedenen Heteroatomen - insbesondere Stickstoff, Sauerstoff und/oder Schwefel - steht, wobei als Aryl- bzw. Heterocyclylsubstituenten jeweils infrage kommen:
Halogen, Cyano, Nitro, Amino, N-Acetylamino, jeweils geradkettiges oder verzweigtes Alkyl, Alkoxy, Alkylthio, Alkylsulfinyl oder Alkylsulfonyl mit jeweils 1 bis 6 Kohlenstoffatomen, jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen, jeweils geradkettiges oder verzweigtes Alkoxycarbonyl oder Alkoximinoalkyl mit jeweils 1 bis 6 Kohlenstoffatomen in den einzelnen Alkylteilen sowie gegebenenfalls einfach oder mehrfach, gleich oder verschieden durch Halogen und/oder geradkettiges oder verzweigtes Alkyl oder Alkoxy mit jeweils 1 bis 6 Kohlenstoffatomen und/oder geradkettiges oder verzweigtes Halogenalkyl oder Halogenalkoxy mit jeweils 1 bis 6 Kohlenstoffatomen und 1 bis 13 gleichen oder verschiedenen Halogenatomen substituiertes Phenyl;
R¹¹ für Wasserstoff steht;
R¹¹ außerdem für gegebenenfalls einfach oder mehrfach, gleich oder verschieden substituiertes, geradkettiges oder verzweigtes Alkyl mit 1 bis 14 Kohlenstoffatomen steht, wobei als Substituenten infrage kommen:
Halogen - insbesondere Fluor, Chlor, Brom und/oder Iod -, Cyano, Carboxyl, Carbamoyl, jeweils geradkettiges oder verzweigtes Alkoxy, Alkoxyalkoxy, Alkylthio, Alkylsulfinyl, Alkylsulfonyl, Alkoxycarbonyl, N-Alkylaminocarbonyl, N,N-Dialkylaminocarbonyl oder Alkylsulfonylaminocarbonyl mit jeweils 1 bis 8 Kohlenstoffatomen in den einzelnen Alkylteilen oder Heterocyclyl, wobei als Heterocyclylrest ein fünf- bis siebengliedriger, gegebenenfalls benzannellierter, gesättigter oder ungesättigter Heterocyclus mit 1 bis 3 gleichen oder verschiedenen Heteroatomen - insbesondere Stickstoff, Sauerstoffund/oder Schwefel - steht;
R¹¹ außerdem für jeweils gegebenenfalls einfach oder mehrfach, gleich oder verschieden durch Halogen - insbesondere Fluor, Chlor, Brom und/oder Iod - substituiertes Alkenyl oder Alkinyl mit jeweils 2 bis 8 Kohlenstoffatomen steht;
R¹¹ außerdem für gegebenenfalls einfach oder mehrfach, gleich oder verschieden durch Halogen - insbesondere Fluor, Chlor, Brom und/oder Iod - und/oder geradkettiges oder verzweigtes Alkyl mit 1 bis 4 Kohlenstoffatomen substituiertes Cycloalkyl mit 3 bis 7 Kohlenstoffatomen steht;
R¹¹ außerdem für jeweils gegebenenfalls im Arylteil einfach oder mehrfach, gleich oder verschieden substituiertes Arylalkyl oder Aryl mit jeweils 6 bis 10 Kohlenstoffatomen im Arylteil und gegebenenfalls 1 bis 4 Kohlenstoffatomen im geradkettigen oder verzweigten Alkylteil steht, wobei als Arylsubstituenten jeweils infrage kommen:
Halogen, Cyano, Nitro, Amino, N-Acetylamino, jeweils geradkettiges oder verzweigtes Alkyl, Alkoxy, Alkylthio, Alkylsulfinyl oder Alkylsulfonyl mit jeweils 1 bis 6 Kohlenstoffatomen, jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen, jeweils geradkettiges oder verzweigtes Alkoxycarbonyl oder Alkoximinoalkyl mit jeweils 1 bis 6 Kohlenstoffatomen in den einzelnen Alkylteilen sowie gegebenenfalls einfach oder mehrfach, gleich oder verschieden durch Halogen und/oder geradkettiges oder verzweigtes Alkyl oder Alkoxy mit jeweils 1 bis 6 Kohlenstoffatomen und/oder geradkettiges oder verzweigtes Halogenalkyl oder Halogenalkoxy mit jeweils 1 bis 6 Kohlenstoffatomen und 1 bis 13 gleichen oder verschiedenen Halogenatomen substituiertes Phenyl;
R¹² für gegebenenfalls einfach oder mehrfach, gleich oder verschieden substituiertes, geradkettiges oder verzweigtes Alkyl mit 1 bis 8 Kohlenstoffatomen steht, wobei als Substituenten infrage kommen:
Halogen - insbesondere Fluor, Chlor, Brom und/oder Iod -, Cycloalkyl mit 3 bis 8 Kohlenstoffatomen oder Heterocyclyl, wobei als Heterocyclylrest ein fünf- bis siebengliedriger, gegebenenfalls benzannellierter, gesättigter oder ungesättigter Heterocyclus mit 1 bis 3 gleichen oder verschiedenen Heteroatomen - insbesondere Stickstoff, Sauerstoff und/oder Schwefel - steht;
R¹² außerdem für gegebenenfalls einfach oder mehrfach, gleich oder verschieden durch Halogen - insbesondere Fluor, Chlor, Brom und/oder Iod - und/oder geradkettiges oder verzweigtes Alkyl mit 1 bis 4 Kohlenstoffatomen substituiertes Cycloalkyl mit 3 bis 7 Kohlenstoffatomen steht;
R¹² außerdem für jeweils gegebenenfalls im Arylteil einfach oder mehrfach, gleich oder verschieden substituiertes Arylalkyl oder Aryl mit jeweils 6 bis 10 Kohlenstoffatomen im Arylteil und gegebenenfalls 1 bis 4 Kohlenstoffatomen im geradkettigen oder verzweigten Alkylteil steht oder für einen gegebenenfalls einfach oder mehrfach, gleich oder verschieden substituierten und/oder benzannellierten, gesättigten oder ungesättigten, fünf- bis siebengliedrigen Heterocyclylrest mit 1 bis 3 gleichen oder verschiedenen Heteroatomen - insbesondere Stickstoff, Sauerstoff und/oder Schwefel - steht, wobei als Aryl- bzw. Heterocyclylsubstituenten jeweils infrage kommen:
Halogen, Cyano, Nitro, Amino, N-Acetylamino, jeweils geradkettiges oder verzweigtes Alkyl, Alkoxy, Alkylthio, Alkylsulfinyl oder Alkylsulfonyl mit jeweils 1 bis 6 Kohlenstoffatomen, jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen, jeweils geradkettiges oder verzweigtes Alkoxycarbonyl oder Alkoximinoalkyl mit jeweils 1 bis 6 Kohlenstoffatomen in den einzelnen Alkylteilen sowie gegebenenfalls einfach oder mehrfach, gleich oder verschieden durch Halogen und/oder geradkettiges oder verzweigtes Alkyl oder Alkoxy mit jeweils 1 bis 6 Kohlenstoffatomen und/oder geradkettiges oder verzweigtes Halogenalkyl oder Halogenalkoxy mit jeweils 1 bis 6 Kohlenstoffatomen und 1 bis 13 gleichen oder verschiedenen Halogenatomen substituiertes Phenyl und
n für eine Zahl 0, 1 oder 2 steht.

2. Substituierte 1-Aryltriazolinone der allgemeinen Formel (I) gemäß Anspruch 1, dadurch gekennzeichnet, daß
R¹ für Wasserstoff oder für jeweils geradkettiges oder verzweigtes Alkyl, Alkoxy, Alkylthio oder Alkylsulfonyl mit jeweils 1 bis 6 Kohlenstoffatomen steht, außerdem für geradkettiges oder verzweigtes Halogenalkyl mit 1 bis 6 Kohlenstoffatomen und 1 bis 13 gleichen oder verschiedenen Halogenatomen - insbesondere Fluor, Chlor oder Brom - steht oder für Cycloalkyl mit 3 bis 7 Kohlenstoffatomen steht,
R² für einen Rest der Formel -NR⁸R⁹steht,
R³, R⁶ jeweils für Wasserstoff stehen,
R⁷ für Wasserstoff, Halogen, Amino oder Nitro steht,
R⁴ für Wasserstoff, Fluor, Chlor, Brom, Cyano, Nitro, oder für einen der Reste -R¹⁰, -O-R¹⁰, -S-R¹⁰, -S(O)-R¹⁰, -SO₂-R¹⁰, -SO₂-OR¹⁰, -SO₂-NR¹¹R¹⁰, -CO-OR¹⁰, -CO-NR¹¹R¹⁰, -O-SO₂-R¹⁰, -N(R¹¹)-SO₂-R¹⁰, -NR¹¹R¹⁰, -NH-P(O)(R¹¹)(OR¹⁰) oder -NH-P(O)(OR¹¹)(OR¹⁰) steht,
R⁵ für Cyano steht und
X für Sauerstoff oder Schwefel steht, wobei
R⁸ für Wasserstoff, für geradkettiges oder verzweigtes Alkyl mit 1 bis 6 Kohlenstoffatomen oder für geradkettiges oder verzweigtes Halogenalkyl mit 1 bis 6 Kohlenstoffatomen und 1 bis 13 gleichen oder verschiedenen Halogenatomen - insbesondere Fluor, Chlor oder Brom - steht und außerdem für einen Rest der Formel -CO-R¹² oder für einen Rest der Formel -S(O)ₙ-R¹² steht,
R⁹ für geradkettiges oder verzweigtes Alkyl mit 1 bis 6 Kohlenstoffatomen oder für geradkettiges oder verzweigtes Halogenalkyl mit 1 bis 6 Kohlenstoffatomen und 1 bis 13 gleichen oder verschiedenen Halogenatomen - insbesondere Fluor, Chlor oder Brom - steht und außerdem für einen Rest der Formel -CO-R¹² oder für einen Rest der Formel -S(O)ₙ-R¹² steht,
R¹⁰ für Wasserstoff steht;
R¹⁰ außerdem für gegebenenfalls einfach oder zweifach, gleich oder verschieden substituiertes, geradkettiges oder verzweigtes Alkyl mit 1 bis 12 Kohlenstoffatomen steht, wobei als Substituenten infrage kommen:
Cyano, Carboxyl, Carbamoyl, jeweils geradkettiges oder verzweigtes Alkoxy, Alkoxyalkoxy, Alkylthio, Alkylsulfinyl, Alkylsulfonyl, Alkoxycarbonyl, N-Alkylaminocarbonyl, N,N-Dialkylaminocarbonyl oder Alkylsulfonylaminocarbonyl mit jeweils 1 bis 6 Kohlenstoffatomen in den einzelnen Alkylteilen oder Heterocyclyl, wobei als Heterocyclylrest ein fünf- bis siebengliedriger, gegebenenfalls benzannellierter, gesättigter oder ungesättigter Heterocyclus mit 1 bis 3 gleichen oder verschiedenen Heteroatomen - insbesondere Stickstoff, Sauerstoff und/oder Schwefel - steht;
R¹⁰ außerdem für geradkettiges oder verzweigtes Halogenalkyl mit 1 bis 6 Kohlenstoffatomen und 1 bis 13 gleichen oder verschiedenen Halogenatomen - insbesondere Fluor, Chlor und/oder Brom - steht,
R¹⁰ außerdem für jeweils gegebenenfalls einfach bis dreifach, gleich oder verschieden durch Halogen - insbesondere Fluor, Chlor und/oder Brom - substituiertes Alkenyl oder Alkinyl mit jeweils 2 bis 6 Kohlenstoffatomen steht;
R¹⁰ außerdem für gegebenenfalls einfach bis dreifach, gleich oder verschieden durch Halogen - insbesondere Fluor, Chlor und/oder Brom - und/oder geradkettiges oder verzweigtes Alkyl mit 1 bis 3 Kohlenstoffatomen substituiertes Cycloalkyl mit 3 bis 7 Kohlenstoffatomen steht;
R¹⁰ außerdem für jeweils gegebenenfalls im Phenylteil einfach bis fünffach, gleich oder verschieden substituiertes Phenylalkyl oder Phenyl mit gegebenenfalls 1 bis 3 Kohlenstoffatomen im geradkettigen oder verzweigten Alkylteil steht, oder für einen gegebenenfalls einfach bis dreifach, gleich oder verschieden substituierten und/oder benzannellierten, gesättigten oder ungesättigten, fünf- bis siebengliedrigen Heterocyclylrest mit 1 bis 3 gleichen oder verschiedenen Heteroatomen - insbesondere Stickstoff, Sauerstoff und/oder Schwefel - steht, wobei als Phenyl- bzw. Heterocyclylsubstituenten jeweils infrage kommen:
Fluor, Chlor, Brom, Cyano, Nitro, Amino, N-Acetylamino, jeweils geradkettiges oder verzweigtes Alkyl, Alkoxy, Alkylthio, Alkylsulfinyl oder Alkylsulfonyl mit jeweils 1 bis 4 Kohlenstoffatomen, jeweils geradkettiges oder verzweigtes Halogenalkyl, Halogenalkoxy, Halogenalkylthio, Halogenalkylsulfinyl oder Halogenalkylsulfonyl mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen sowie gegebenenfalls einfach bis fünffach, gleich oder verschieden durch Fluor, Chlor, Brom und/oder geradkettiges oder verzweigtes Alkyl oder Alkoxy mit jeweils 1 bis 4 Kohlenstoffatomen und/oder geradkettiges oder verzweigtes Halogenalkyl oder Halogenalkoxy mit jeweils 1 bis 4 Kohlenstoffatomen und 1 bis 9 gleichen oder verschiedenen Halogenatomen substituiertes Phenyl;
R¹¹ für Wasserstoff steht;
R¹¹ außerdem für gegebenenfalls einfach oder zweifach, gleich oder verschieden substituiertes, geradkettiges oder verzweigtes Alkyl mit 1 bis 12 Kohlenstoffatomen steht, wobei als Substituenten infrage kommen:
Cyano, Carboxyl, Carbamoyl, jeweils geradkettiges oder verzweigtes Alkoxy, Alkoxyalkoxy, Alkylthio, Alkylsulfinyl, Alkylsulfonyl, Alkoxycarbonyl, N-Alkylaminocarbonyl, N,N-Dialkylaminocarbonyl oder Alkylsulfonylaminocarbonyl mit jeweils 1 bis 6 Kohlenstoffatomen in den einzelnen Alkylteilen oder Heterocyclyl, wobei als Heterocyclylrest ein fünf- bis siebengliedriger, gegebenenfalls benzannellierter, gesättigter oder ungesättigter Heterocyclus mit 1 bis 3 gleichen oder verschiedenen Heteroatomen - insbesondere Stickstoff, Sauerstoff und/oder Schwefel - steht;
R¹¹ außerdem für geradkettiges oder verzweigtes Halogenalkyl mit 1 bis 6 Kohlenstoffatomen und 1 bis 13 gleichen oder verschiedenen Halogenatomen - insbesondere Fluor, Chlor und/oder Brom - steht,
R¹¹ außerdem für jeweils gegebenenfalls einfach bis dreifach, gleich oder verschieden durch Halogen - insbesondere Fluor, Chlor und/oder Brom - substituiertes Alkenyl oder Alkinyl mit jeweils 2 bis 6 Kohlenstoffatomen steht;
R¹¹ außerdem für gegebenenfalls einfach bis dreifach, gleich oder verschieden durch Halogen - insbesondere Fluor, Chlor und/oder Brom - und/oder geradkettiges oder verzweigtes Alkyl mit 1 bis 3 Kohlenstoffatomen substituiertes Cycloalkyl mit 3 bis 7 Kohlenstoffatomen steht;
R¹¹ außerdem für jeweils gegebenenfalls im Phenylteil einfach bis fünffach, gleich oder verschieden substituiertes Phenylalkyl oder Phenyl mit gegebenenfalls 1 bis 3 Kohlenstoffatomen im geradkettigen oder verzweigten Alkylteil steht, wobei als Phenylsubstituenten jeweils infrage kommen:
Fluor, Chlor, Brom, Cyano, Nitro, Amino, N-Acetylamino, jeweils geradkettiges oder verzweigtes Alkyl, Alkoxy, Alkylthio, Alkylsulfinyl oder Alkylsulfonyl mit jeweils 1 bis 4 Kohlenstoffatomen, jeweils geradkettiges oder verzweigtes Halogenalkyl, Halogenalkoxy, Halogenalkylthio, Halogenalkylsulfinyl oder Halogenalkylsulfonyl mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen sowie gegebenenfalls einfach bis fünffach, gleich oder verschieden durch Fluor, Chlor, Brom und/oder geradkettiges oder verzweigtes Alkyl oder Alkoxy mit jeweils 1 bis 4 Kohlenstoffatomen und/oder geradkettiges oder verzweigtes Halogenalkyl oder Halogenalkoxy mit jeweils 1 bis 4 Kohlenstoffatomen und 1 bis 9 gleichen oder verschiedenen Halogenatomen substituiertes Phenyl;
R¹² für gegebenenfalls einfach oder zweifach, gleich oder verschieden substituiertes, geradkettiges oder verzweigtes Alkyl mit 1 bis 12 Kohlenstoffatomen steht, wobei als Substituenten infrage kommen:
Cycloalkyl mit 3 bis 7 Kohlenstoffatomen oder Heterocyclyl, wobei als Heterocyclylrest ein fünf- bis siebengliedriger, gegebenenfalls benzannellierter, gesättigter oder ungesättigter Heterocyclus mit 1 bis 3 gleichen oder verschiedenen Heteroatomen - insbesondere Stickstoff, Sauerstoff und/oder Schwefel - steht;
R¹² außerdem für geradkettiges oder verzweigtes Halogenalkyl mit 1 bis 6 Kohlenstoffatomen und 1 bis 13 gleichen oder verschiedenen Halogenatomen - insbesondere Fluor, Chlor und/oder Brom - steht,
R¹² außerdem für gegebenenfalls einfach bis dreifach, gleich oder verschieden durch Halogen - insbesondere Fluor, Chlor und/oder Brom - und/oder geradkettiges oder verzweigtes Alkyl mit 1 bis 3 Kohlenstoffatomen substituiertes Cycloalkyl mit 3 bis 7 Kohlenstoffatomen steht;
R¹² außerdem für jeweils gegebenenfalls im Phenylteil einfach bis fünffach, gleich oder verschieden substituiertes Phenylalkyl oder Phenyl mit gegebenenfalls 1 bis 3 Kohlenstoffatomen im geradkettigen oder verzweigten Alkylteil steht, oder für einen gegebenenfalls einfach bis dreifach, gleich oder verschieden substituierten und/oder benzannellierten, gesättigten oder ungesättigten, fünf- bis siebengliedrigen Heterocyclylrest mit 1 bis 3 gleichen oder verschiedenen Heteroatomen - insbesondere Stickstoff, Sauerstoff und/oder Schwefel - steht, wobei als Phenyl- bzw. Heterocyclylsubstituenten jeweils infrage kommen:
Fluor, Chlor, Brom, Cyano, Nitro, Amino, N-Acetylamino, jeweils geradkettiges oder verzweigtes Alkyl, Alkoxy, Alkylthio, Alkylsulfinyl oder Alkylsulfonyl mit jeweils 1 bis 4 Kohlenstoffatomen, jeweils geradkettiges oder verzweigtes Halogenalkyl, Halogenalkoxy, Halogenalkylthio, Halogenalkylsulfinyl oder Halogenalkylsulfonyl mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen sowie gegebenenfalls einfach bis fünffach, gleich oder verschieden durch Fluor, Chlor, Brom und/oder geradkettiges oder verzweigtes Alkyl oder Alkoxy mit jeweils 1 bis 4 Kohlenstoffatomen und/oder geradkettiges oder verzweigtes Halogenalkyl oder Halogenalkoxy mit jeweils 1 bis 4 Kohlenstoffatomen und 1 bis 9 gleichen oder verschiedenen Halogenatomen substituiertes Phenyl und
n für eine Zahl 0, 1 oder 2 steht.

3. Verfahren zur Herstellung substituierter 1-Aryltriazolinone der allgemeinen Formel (I), 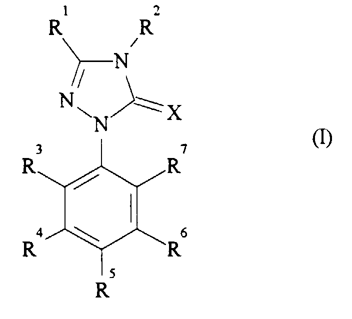 in welcher
R¹, R², R³, R⁴, R⁵, R⁶, R⁷ und r X die in Anspruch 1 genannten Bedeutungen haben,
dadurch gekennzeichnet, daß man
a) 1H-Triazolinone der Formel (II), 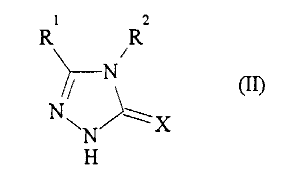 in welcher
R¹, R² und X die oben angegebene Bedeutung haben,
mit Halogenbenzol-Derivaten der Formel (III), 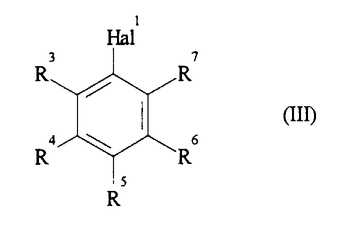 in welcher
R³, R⁴, R⁵, R⁶ und R⁷ die oben angegebenen Bedeutungen haben und Hal1 für Halogen steht,
gegebenenfalls in Gegenwart eines Verdünnungsmittels und gegebenenfalls in Gegenwart eines Reaktionshilfsmittels umsetzt, oder daß man
b) substituierte 1-Aryltriazolinone der Formel (Ia), 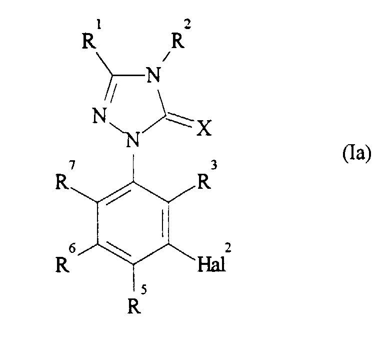 in welcher
R¹, R², R³, R⁶, R⁵, R⁷ und X die oben angegebenen Bedeutungen haben und Hal² für Halogen steht,
mit Nukleophilen der Formel (IV),
H-R¹³ (IV)
in welcher
R¹³ für einen Rest der Formel -O-R¹⁰, -S-R¹⁰ oder -NR¹¹R¹⁰ steht,
wobei R¹⁰ und R¹¹ die oben angegebenen Bedeutungen haben,
gegebenenfalls in Gegenwart eines Verdünnungsmittels und gegebenenfalls in Gegenwart eines Reaktionshilfsmittels umsetzt, oder daß man
c) substituierte Triazolinone der Formel (V), 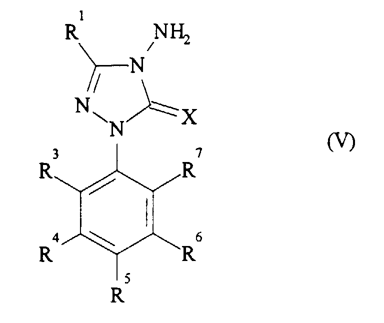 in welcher
R¹, R³, R⁴, R⁵, R⁶, R⁷ und X die oben angegebenen Bedeutungen haben,
mit Alkylierungs, Acylierungs- oder Sulfonylierungsmitteln der Formel (VI),
R⁹-E (VI)
in welcher
R⁹ die oben angegebene Bedeutung hat und
E für eine elektronenanziehende Abgangsgruppe steht,
gegebenenfalls in Gegenwart eines Verdünnungsmittels und gegebenenfalls in Gegenwart eines Reaktionshilfsmittels umsetzt, oder daß man
d) 4-Alkylidenimino-triazolinone der Formel (VII), 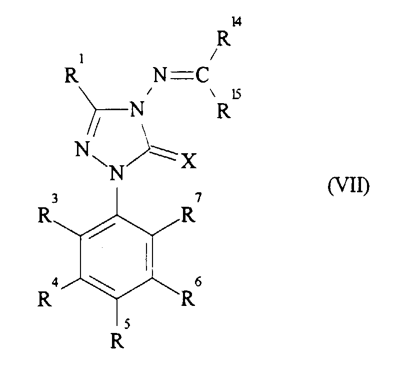 in welcher
R¹, R³, R⁴, R⁵, R⁶, R⁷ und X die oben angegebenen Bedeutungen haben,
R¹⁴ für Wasserstoff oder Alkyl steht und
R¹⁵ für Alkyl oder Alkoxy steht,
mit einen Reduktionsmittel gegebenenfalls in Gegenwart eines Verdünnungsmittels und gegebenenfalls in Gegenwart eines Reaktionshilfsmittels umsetzt.

4. Herbizide Mittel, gekennzeichnet durch einen Gehalt an mindestens einem substituierten 1-Aryltriazolinon der Formel (I) gemäß den Ansprüchen 1 bis 3.

5. Verfahren zur Bekämpfung von unerwünschten Pflanzen, dadurch gekennzeichnet, daß man substituierte 1-Aryltriazolinone der allgemeinen Formel (I) gemäß den Ansprüchen 1 bis 3 auf Pflanzen und/oder ihren Lebensraum einwirken läßt.

6. Verwendung von substituierten 1-Aryltriazolinonen der allgemeinen Formel (I) gemäß der Ansprüche 1 bis 3 zur Bekämpfung von unerwünschten Pflanzen.

7. Verfahren zur Herstellung von herbiziden Mitteln, dadurch gekennzeichnet, daß man substituierte 1-Aryltriazolinone der allgemeinen Formel (I) gemäß der Ansprüche 1 bis 3 mit Streckmitteln und/oder oberflächenaktiven Substanzen vermischt.

8. Substituierte 1-Aryltriazolinone der Formel (Ia) 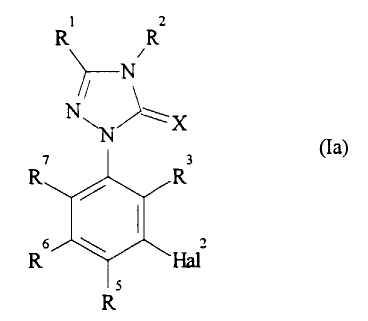 in welcher
R¹ für Wasserstoff oder für jeweils geradkettiges oder verzweigtes Alkyl, Alkoxy, Alkylthio oder Alkylsulfonyl mit jeweils 1 bis 8 Kohlenstoffatomen steht, außerdem für geradkettiges oder verzweigtes Halogenalkyl mit 1 bis 8 Kohlenstoffatomen und 1 bis 17 gleichen oder verschiedenen Halogenatomen steht oder für Cycloalkyl mit 3 bis 8 Kohlenstoffatomen steht,
R² für einen Rest der Formel -NR⁸R⁹steht,
R³, R⁶ jeweils für Wasserstoff stehen,
R⁷ für Wasserstoff, Halogen, Amino oder Nitro stehen,
Hal² für Halogen steht,
R⁵ für Cyano steht und
X für Sauerstoff oder Schwefel steht, wobei
R⁸ für Wasserstoff, für geradkettiges oder verzweigtes Alkyl mit 1 bis 8 Kohlenstoffatomen oder für geradkettiges oder verzweigtes Halogenalkyl mit 1 bis 8 Kohlenstoffatomen und 1 bis 17 gleichen oder verschiedenen Halogenatomen steht und außerdem für einen Rest der Formel -CO-R¹² oder für einen Rest der Formel -S(O)ₙ-R¹² steht,
R⁹ für geradkettiges oder verzweigtes Alkyl mit 1 bis 8 Kohlenstoffatomen oder für geradkettiges oder verzweigtes Halogenalkyl mit 1 bis 8 Kohlenstoffatomen und 1 bis 17 gleichen oder verschiedenen Halogenatomen steht und außerdem für einen Rest der Formel -CO-R¹² oder für einen Rest der Formel -S(O)ₙ-R¹² steht,
R¹² für gegebenenfalls einfach oder mehrfach, gleich oder verschieden substituiertes, geradkettiges oder verzweigtes Alkyl mit 1 bis 8 Kohlenstoffatomen steht, wobei als Substituenten infrage kommen:
Halogen - insbesondere Fluor, Chlor, Brom und/oder Iod -, Cycloalkyl mit 3 bis 8 Kohlenstoffatomen oder Heterocyclyl, wobei als Heterocyclylrest ein fünf- bis siebengliedriger, gegebenenfalls benzannellierter, gesättigter oder ungesättigter Heterocyclus mit 1 bis 3 gleichen oder verschiedenen Heteroatomen - insbesondere Stickstoff, Sauerstoff und/ oder Schwefel - steht;
R¹² außerdem für gegebenenfalls einfach oder mehrfach, gleich oder verschieden durch Halogen - insbesondere Fluor, Chlor, Brom und/oder Iod - und/oder geradkettiges oder verzweigtes Alkyl mit 1 bis 4 Kohlenstoffatomen substituiertes Cycloalkyl mit 3 bis 7 Kohlenstoffatomen steht;
R¹² außerdem für jeweils gegebenenfalls im Arylteil einfach oder mehrfach, gleich oder verschieden substituiertes Arylalkyl oder Aryl mit jeweils 6 bis 10 Kohlenstoffatomen im Arylteil und gegebenenfalls 1 bis 4 Kohlenstoffatomen im geradkettigen oder verzweigten Alkylteil steht oder für einen gegebenenfalls einfach oder mehrfach, gleich oder verschieden substituierten und/oder benzannellierten, gesättigten oder ungesättigten, fünf- bis siebengliedrigen Heterocyclylrest mit 1 bis 3 gleichen oder verschiedenen Heteroatomen - insbesondere Stickstoff, Sauerstoff und/oder Schwefel - steht, wobei als Aryl- bzw. Heterocyclylsubstituenten jeweils infrage kommen:
Halogen, Cyano, Nitro, Amino, N-Acetylamino, jeweils geradkettiges oder verzweigtes Alkyl, Alkoxy, Alkylthio, Alkylsulfinyl oder Alkylsulfonyl mit jeweils 1 bis 6 Kohlenstoffatomen, jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen, jeweils geradkettiges oder verzweigtes Alkoxycarbonyl oder Alkoximinoalkyl mit jeweils 1 bis 6 Kohlenstoffatomen in den einzelnen Alkylteilen sowie gegebenenfalls einfach oder mehrfach, gleich oder verschieden durch Halogen und/oder geradkettiges oder verzweigtes Alkyl oder Alkoxy mit jeweils 1 bis 6 Kohlenstoffatomen und/oder geradkettiges oder verzweigtes Halogenalkyl oder Halogenalkoxy mit jeweils 1 bis 6 Kohlenstoffatomen und 1 bis 13 gleichen oder verschiedenen Halogenatomen substituiertes Phenyl und
n für eine Zahl 0, 1 oder 2 steht.

9. Substituierte Triazolinone der Formel (V) 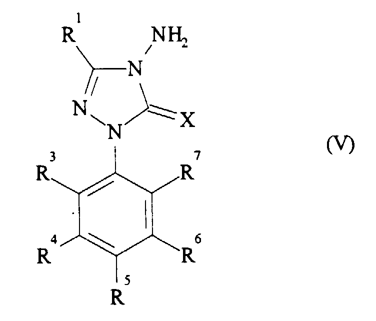 in welcher
R¹ für Wasserstoff oder für jeweils geradkettiges oder verzweigtes Alkyl, Alkoxy, Alkylthio oder Alkylsulfonyl mit jeweils 1 bis 8 Kohlenstoffatomen steht, außerdem für geradkettiges oder verzweigtes Halogenalkyl mit 1 bis 8 Kohlenstoffatomen und 1 bis 17 gleichen oder verschiedenen Halogenatomen steht oder für Cycloalkyl mit 3 bis 8 Kohlenstoffatomen steht,
R³, R⁶ jeweils für Wasserstoff stehen,
R⁷ für Wasserstoff, Halogen, Amino oder Nitro stehen,
R⁴ für Wasserstoff, Fluor, Chlor, Brom, Iod, Cyano, Nitro, oder für einen der Reste -R¹⁰, -O-R¹⁰, -S-R¹⁰, -S(O)-R¹⁰, -SO₂-R¹⁰, -SO₂-OR¹⁰, -SO₂-NR¹¹R¹⁰, -CO-OR¹⁰, -CO-NR¹¹R¹⁰, -O-SO₂-R¹⁰, -N(R¹¹)-SO₂-R¹⁰, -NR¹¹R¹⁰, -NH-P(O)(R¹¹)(OR¹⁰) oder -NH-P(O)(OR¹¹)(OR¹⁰) steht,
R⁵ für Cyano steht und
X für Sauerstoff oder Schwefel steht, wobei
R¹⁰ für Wasserstoff steht;
R¹⁰ außerdem für gegebenenfalls einfach oder mehrfach, gleich oder verschieden substituiertes, geradkettiges oder verzweigtes Alkyl mit 1 bis 14 Kohlenstoffatomen steht, wobei als Substituenten infrage kommen:
Halogen - insbesondere Fluor, Chlor, Brom und/oder Iod -, Cyano, Carboxyl, Carbamoyl, jeweils geradkettiges oder verzweigtes Alkoxy, Alkoxyalkoxy, Alkylthio, Alkylsulfinyl, Alkylsulfonyl, Alkoxycarbonyl, N-Alkylaminocarbonyl, N,N-Dialkylaminocarbonyl oder Alkylsulfonylaminocarbonyl mit jeweils 1 bis 8 Kohlenstoffatomen in den einzelnen Alkylteilen oder Heterocyclyl, wobei als Heterocyclylrest ein fünf- bis siebengliedriger, gegebenenfalls benzannellierter, gesättigter oder ungesättigter Heterocyclus mit 1 bis 3 gleichen oder verschiedenen Heteroatomen - insbesondere Stickstoff, Sauerstoffund/oder Schwefel - steht;
R¹⁰ außerdem für jeweils gegebenenfalls einfach oder mehrfach, gleich oder verschieden durch Halogen - insbesondere Fluor, Chlor, Brom und/oder Iod - substituiertes Alkenyl oder Alkinyl mit jeweils 2 bis 8 Kohlenstoffatomen steht;
R¹⁰ außerdem für gegebenenfalls einfach oder mehrfach, gleich oder verschieden durch Halogen - insbesondere Fluor, Chlor, Brom und/oder Iod - und/oder geradkettiges oder verzweigtes Alkyl mit 1 bis 4 Kohlenstoffatomen substituiertes Cycloalkyl mit 3 bis 7 Kohlenstoffatomen steht;
R¹⁰ außerdem für jeweils gegebenenfalls im Arylteil einfach oder mehrfach, gleich oder verschieden substituiertes Arylalkyl oder Aryl mit jeweils 6 bis 10 Kohlenstoffatomen im Arylteil und gegebenenfalls 1 bis 4 Kohlenstoffatomen im geradkettigen oder verzweigten Alkylteil steht, oder für einen gegebenenfalls einfach oder mehrfach, gleich oder verschieden substituierten und/oder benzannellierten, gesättigten oder ungesättigten, fünf- bis siebengliedrigen Heterocyclylrest mit 1 bis 3 gleichen oder verschiedenen Heteroatomen - insbesondere Stickstoff, Sauerstoff und/oder Schwefel - steht, wobei als Aryl- bzw. Heterocyclylsubstituenten jeweils infrage kommen:
Halogen, Cyano, Nitro, Amino, N-Acetylamino, jeweils geradkettiges oder verzweigtes Alkyl, Alkoxy, Alkylthio, Alkylsulfinyl oder Alkylsulfonyl mit jeweils 1 bis 6 Kohlenstoffatomen, jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen, jeweils geradkettiges oder verzweigtes Alkoxycarbonyl oder Alkoximinoalkyl mit jeweils 1 bis 6 Kohlenstoffatomen in den einzelnen Alkylteilen sowie gegebenenfalls einfach oder mehrfach, gleich oder verschieden durch Halogen und/oder geradkettiges oder verzweigtes Alkyl oder Alkoxy mit jeweils 1 bis 6 Kohlenstoffatomen und/oder geradkettiges oder verzweigtes Halogenalkyl oder Halogenalkoxy mit jeweils 1 bis 6 Kohlenstoffatomen und 1 bis 13 gleichen oder verschiedenen Halogenatomen substituiertes Phenyl;
R¹¹ für Wasserstoff steht;
R¹¹ außerdem für gegebenenfalls einfach oder mehrfach, gleich oder verschieden substituiertes, geradkettiges oder verzweigtes Alkyl mit 1 bis 14 Kohlenstoffatomen steht, wobei als Substituenten infrage kommen:
Halogen - insbesondere Fluor, Chlor, Brom und/oder Iod -, Cyano, Carboxyl, Carbamoyl, jeweils geradkettiges oder verzweigtes Alkoxy, Alkoxyalkoxy, Alkylthio, Alkylsulfinyl, Alkylsulfonyl, Alkoxycarbonyl, N-Alkylaminocarbonyl, N,N-Dialkylaminocarbonyl oder Alkylsulfonylaminocarbonyl mit jeweils 1 bis 8 Kohlenstoffatomen in den einzelnen Alkylteilen oder Heterocyclyl, wobei als Heterocyclylrest ein fünf- bis siebengliedriger, gegebenenfalls benzannellierter, gesättigter oder ungesättigter Heterocyclus mit 1 bis 3 gleichen oder verschiedenen Heteroatomen - insbesondere Stickstoff, Sauerstoffund/oder Schwefel - steht;
R¹¹ außerdem für jeweils gegebenenfalls einfach oder mehrfach, gleich oder verschieden durch Halogen - insbesondere Fluor, Chlor, Brom und/oder Iod - substituiertes Alkenyl oder Alkinyl mit jeweils 2 bis 8 Kohlenstoffatomen steht;
R¹¹ außerdem für gegebenenfalls einfach oder mehrfach, gleich oder verschieden durch Halogen - insbesondere Fluor, Chlor, Brom und/oder Iod - und/oder geradkettiges oder verzweigtes Alkyl mit 1 bis 4 Kohlenstoffatomen substituiertes Cycloalkyl mit 3 bis 7 Kohlenstoffatomen steht;
R¹¹ außerdem für jeweils gegebenenfalls im Arylteil einfach oder mehrfach, gleich oder verschieden substituiertes Arylalkyl oder Aryl mit jeweils 6 bis 10 Kohlenstoffatomen im Arylteil und gegebenenfalls 1 bis 4 Kohlenstoffatomen im geradkettigen oder verzweigten Alkylteil steht, wobei als Arylsubstituenten jeweils infrage kommen:
Halogen, Cyano, Nitro, Amino, N-Acetylamino, jeweils geradkettiges oder verzweigtes Alkyl, Alkoxy, Alkylthio, Alkylsulfinyl oder Alkylsulfonyl mit jeweils 1 bis 6 Kohlenstoffatomen, jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen, jeweils geradkettiges oder verzweigtes Alkoxycarbonyl oder Alkoximinoalkyl mit jeweils 1 bis 6 Kohlenstoffatomen in den einzelnen Alkylteilen sowie gegebenenfalls einfach oder mehrfach, gleich oder verschieden durch Halogen und/oder geradkettiges oder verzweigtes Alkyl oder Alkoxy mit jeweils 1 bis 6 Kohlenstoffatomen und/oder geradkettiges oder verzweigtes Halogenalkyl oder Halogenalkoxy mit jeweils 1 bis 6 Kohlenstoffatomen und 1 bis 13 gleichen oder verschiedenen Halogenatomen substituiertes Phenyl.

## Claims

1. Substituted 1-aryltriazolinones of the general formula (I), 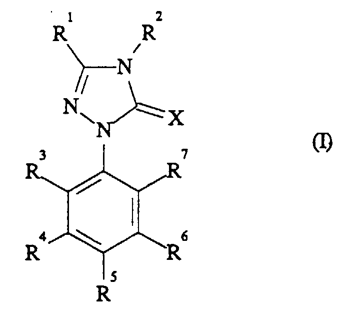 in which
R¹ represents hydrogen or represents in each case straight-chain or branched alkyl, alkoxy, alkylthio or alkylsulphonyl having in each case from 1 to 8 carbon atoms, furthermore represents straight-chain or branched halogenoalkyl having from 1 to 8 carbon atoms and from 1 to 17 identical or different halogen atoms, or represents cycloalkyl having from 3 to 8 carbon atoms,
R² represents a radical of the formula -NR⁸R⁹,
R³, R⁶ in each case represent hydrogen,
R⁷ represents hydrogen, halogen, amino or nitro,
R⁴ represents hydrogen, fluorine, chlorine, bromine, iodine, cyano or nitro, or represents one of the radicals -R¹⁰, -O-R¹⁰, -S-R¹⁰, -S(O)-R¹⁰, -SO₂-R¹⁰, -SO₂-OR¹⁰, -SO₂-NR¹¹R¹⁰, -CO-OR¹⁰, -CO-NR¹¹R¹⁰, -O-SO₂-R¹⁰, -N(R¹¹)-SO₂-R¹⁰, -NR¹¹R¹⁰, -NH-P(O)(R¹¹)(OR¹⁰) or -NH-P(O)(OR¹¹)(OR¹⁰),
R⁵ represents cyan and
X represents oxygen or sulphur, where
R⁸ represents hydrogen, straight-chain or branched alkyl having from 1 to 8 carbon atoms or straight-chain or branched halogenoalkyl having from 1 to 8 carbon atoms and from 1 to 17 identical or different halogen atoms, and furthermore represents a radical of the formula -CO-R¹² or a radical of the formula -S(O)ₙ-R¹²,
R⁹ represents straight-chain or branched alkyl having from 1 to 8 carbon atoms or straight-chain or branched halogenoalkyl having from 1 to 8 carbon atoms and from 1 to 17 identical or different halogen atoms, and furthermore represents a radical of the formula -CO-R¹² or a radical of the formula -S(O)ₙ-R¹²,
R¹⁰ represents hydrogen;
R¹⁰ furthermore represents straight-chain or branched alkyl having from 1 to 14 carbon atoms which is optionally substituted once or more than once by identical or different substituents, possible substituents being:
halogen - in particular fluorine, chlorine, bromine and/or iodine - cyano, carboxyl, carbamoyl, in each case straight-chain or branched alkoxy, alkoxyalkoxy, alkylthio, alkylsulphinyl, alkylsulphonyl, alkoxycarbonyl, N-alkylaminocarbonyl, N,N-dialkylaminocarbonyl or alkylsulphonylaminocarbonyl having in each case from 1 to 8 carbon atoms in the individual alkyl moieties, or heterocyclyl, the heterocyclyl radical being a five- to seven-membered, optionally benzo-fused, saturated or unsaturated heterocycle having from 1 to 3 identical or different hetero atoms - in particular nitrogen, oxygen and/or sulphur;
R¹⁰ furthermore represents alkenyl or alkinyl having in each case from 2 to 8 carbon atoms, which are optionally substituted once or more than once by identical or different halogens - in particular fluorine, chlorine, bromine and/or iodine;
R¹⁰ furthermore represents cycloalkyl having from 3 to 7 carbon atoms which is optionally substituted once or more than once by identical or different substituents comprising halogen - in particular fluorine, chlorine, bromine and/or iodine - and/or straight-chain or branched alkyl having from 1 to 4 carbon atoms;
R¹⁰ furthermore represents arylalkyl or aryl having in each case from 6 to 10 carbon atoms in the aryl moiety and optionally from 1 to 4 carbon atoms in the straight-chain or branched alkyl moiety, which are in each case optionally substituted in the aryl moiety once or more than once by identical or different substituents, or represents a saturated or unsaturated, five- to seven-membered heterocyclyl radical having from 1 to 3 identical or different hetero atoms - in particular nitrogen, oxygen and/or sulphur - which is optionally substituted once or more than once by identical or different substituents and/or is benzo-fused, possible substituents of the aryl and/or heterocyclyl being in each case:
halogen, cyano, nitro, amino, N-acetylamino, in each case straight-chain or branched alkyl, alkoxy, alkylthio, alkylsulphinyl or alkylsulphonyl having in each case from 1 to 6 carbon atoms, in each case straight-chain or branched halogenoalkyl, halogenoalkoxy, halogenoalkylthio, halogenoalkylsulphinyl or halogenoalkylsulphonyl having in each case from 1 to 6 carbon atoms and from 1 to 13 identical or different halogen atoms, in each case straight-chain or branched alkoxycarbonyl or alkoximinoalkyl having in each case from 1 to 6 carbon atoms in the individual alkyl moieties, and phenyl which is optionally substituted once or more than once by identical or different substituents comprising halogen and/or straight-chain or branched alkyl or alkoxy having in each case from 1 to 6 carbon atoms and/or straight-chain or branched halogenoalkyl or halogenoalkoxy having in each case from 1 to 6 carbon atoms and from 1 to 13 identical or different halogen atoms;
R¹¹ represents hydrogen;
R¹¹ furthermore represents straight-chain or branched alkyl having from 1 to 14 carbon atoms which is optionally substituted once or more than once by identical or different substituents, possible substituents being:
halogen - in particular fluorine, chlorine, bromine and/or iodine - cyano, carboxyl, carbamoyl, in each case straight-chain or branched alkoxy, alkoxyalkoxy, alkylthio, alkylsulphinyl, alkylsulphonyl, alkoxycarbonyl, N-alkylaminocarbonyl, N,N-dialkylaminocarbonyl or alkylsulphonylaminocarbonyl having in each case from 1 to 8 carbon atoms in the individual alkyl moieties, or heterocyclyl, the heterocyclyl radical being a five- to seven-membered, optionally benzo-fused, saturated or unsaturated heterocycle having from 1 to 3 identical or different hetero atoms - in particular nitrogen, oxygen and/or sulphur;
R¹¹ furthermore represents alkenyl or alkinyl having in each case from 2 to 8 carbon atoms, which are optionally substituted once or more than once by identical or different halogens - in particular fluorine, chlorine, bromine and/or iodine;
R¹¹ furthermore represents cycloalkyl having from 3 to 7 carbon atoms which is optionally substituted once or more than once by identical or different substituents comprising halogen - in particular fluorine, chlorine, bromine and/or iodine - and/or straight-chain or branched alkyl having from 1 to 4 carbon atoms;
R¹¹ furthermore represents arylalkyl or aryl having in each case from 6 to 10 carbon atoms in the aryl moiety and optionally from 1 to 4 carbon atoms in the straight-chain or branched alkyl moiety, which are in each case optionally substituted in the aryl moiety once or more than once by identical or different substituents, possible substituents of the aryl being in each case:
halogen, cyano, nitro, amino, N-acetylamino, in each case straight-chain or branched alkyl, alkoxy, alkylthio, alkylsulphinyl or alkylsulphonyl having in each case from 1 to 6 carbon atoms, in each case straight-chain or branched halogenoalkyl, halogenoalkoxy, halogenoalkylthio, halogenoalkylsulphinyl or halogenoalkylsulphonyl having in each case from 1 to 6 carbon atoms and from 1 to 13 identical or different halogen atoms, in each case straight-chain or branched alkoxycarbonyl or alkoximinoalkyl having in each case from 1 to 6 carbon atoms in the individual alkyl moieties, and phenyl which is optionally substituted once or more than once by identical or different substituents comprising halogen and/or straight-chain or branched alkyl or alkoxy having in each case from 1 to 6 carbon atoms and/or straight-chain or branched halogenoalkyl or halogenoalkoxy having in each case from 1 to 6 carbon atoms and from 1 to 13 identical or different halogen atoms;
R¹² represents straight-chain or branched alkyl having from 1 to 8 carbon atoms which is optionally substituted once or more than once by identical or different substituents, possible substituents being:
halogen - in particular fluorine, chlorine, bromine and/or iodine - cycloalkyl having from 3 to 8 carbon atoms or heterocyclyl, the heterocyclyl radical being a five- to seven-membered optionally benzo-fused, saturated or unsaturated heterocycle having from 1 to 3 identical or different hetero atoms - in particular nitrogen, oxygen and/or sulphur;
R¹² furthermore represents cycloalkyl having from 3 to 7 carbon atoms which is optionally substituted once or more than once by identical or different substituents comprising halogen - in particular fluorine, chlorine, bromine and/or iodine - and/or straight-chain or branched alkyl having from 1 to 4 carbon atoms;
R¹² furthermore represents arylalkyl or aryl having in each case from 6 to 10 carbon atoms in the aryl moiety and optionally from 1 to 4 carbon atoms in the straight-chain or branched alkyl moiety, which are in each case optionally substituted in the aryl moiety once or more than once by identical or different substituents, or represents a saturated or unsaturated, five- to seven-membered heterocyclyl radical having from 1 to 3 identical or different hetero atoms - in particular nitrogen, oxygen and/or sulphur - which is optionally substituted once or more than once by identical or different substituents, possible substituents of aryl or heterocyclyl being in each case:
halogen, cyano, nitro, amino, N-acetylamino, in each case straight-chain or branched alkyl, alkoxy, alkylthio, alkylsulphinyl or alkylsulphonyl having in each case from 1 to 6 carbon atoms, in each case straight-chain or branched halogenoalkyl, halogenoalkoxy, halogenoalkylthio, halogenoalkylsulphinyl or halogenoalkylsulphonyl having in each case from 1 to 6 carbon atoms and from 1 to 13 identical or different halogen atoms, in each case straight-chain or branched alkoxycarbonyl or alkoximinoalkyl having in each case from 1 to 6 carbon atoms in the individual alkyl moieties, and phenyl which is optionally substituted once or more than once by identical or different substituents comprising halogen and/or straight-chain or branched alkyl or alkoxy having in each case from 1 to 6 carbon atoms and/or straight-chain or branched halogenoalkyl or halogenoalkoxy having in each case from 1 to 6 carbon atoms and from 1 to 13 identical or different halogen atoms and
n represents a number 0, 1 or 2.

2. Substituted 1-aryltriazolinones of the general formula (I) according to Claim 1, characterized in that
R¹ represents hydrogen or in each case straight-chain or branched alkyl, alkoxy, alkylthio or alkylsulphonyl having in each case from 1 to 6 carbon atoms, or furthermore represents straight-chain or branched halogenoalkyl having from 1 to 6 carbon atoms and from 1 to 13 identical or different halogen atoms - in particular fluorine, chlorine or bromine - or represents cycloalkyl having from 3 to 7 carbon atoms,
R² represents a radical of the formula -NR⁸R⁹,
R³, R⁶ in each case represent hydrogen, nitro,
R⁷ represents hydrogen, halogen, amino or
R⁴ represents hydrogen, fluorine, chlorine, bromine, cyano or nitro, or represents one of the radicals -R¹⁰, -O-R¹⁰, -S-R¹⁰, -S(O)-R¹⁰, -SO₂-R¹⁰, -SO₂-OR¹⁰, -SO₂-NR¹¹R¹⁰, -CO-OR¹⁰, -CO-NR¹¹R¹⁰, -O-SO₂-R¹⁰, -N(R¹¹)-SO₂-R¹⁰, -NR¹¹R¹⁰, -NH-P(O)(R¹¹)(OR¹⁰) or -NH-P(O)(OR¹¹)(OR¹⁰),
R⁵ represents cyano and
X represents oxygen or sulphur, where
R⁸ represents hydrogen, straight-chain or branched alkyl having from 1 to 6 carbon atoms or straight-chain or branched halogenoalkyl having from 1 to 6 carbon atoms and from 1 to 13 identical or different halogen atoms - in particular fluorine, chlorine or bromine - and furthermore represents a radical of the formula -CO-R¹² or a radical of the formula -S(O)ₙ-R¹²,
R⁹ represents straight-chain or branched alkyl having from 1 to 6 carbon atoms or straight-chain or branched halogenoalkyl having from 1 to 6 carbon atoms and from 1 to 13 identical or different halogen atoms - in particular fluorine, chlorine or bromine - and furthermore represents a radical of the formula -CO-R¹² or a radical of the formula -S(O)ₙ-R¹²,
R¹⁰ represents hydrogen;
R¹⁰ furthermore represents straight-chain or branched alkyl having from 1 to 12 carbon atoms which is optionally substituted once or twice by identical or different substituents, possible substituents being:
cyano, carboxyl, carbamoyl, in each case straight-chain or branched alkoxy, alkoxy-alkoxy, alkylthio, alkylsulphinyl, alkylsulphonyl, alkoxycarbonyl, N-alkylaminocarbonyl, N,N-dialkylaminocarbonyl or alkylsulphonylaminocarbonyl having in each case from 1 to 6 carbon atoms in the individual alkyl moieties, or heterocyclyl, the heterocyclyl radical being a five- to seven-membered, optionally benzo-fused, saturated or unsaturated heterocycle having from 1 to 3 identical or different hetero atoms - in particular nitrogen, oxygen and/or sulphur;
R¹⁰ furthermore represents straight-chain or branched halogenoalkyl having from 1 to 6 carbon atoms and from 1 to 13 identical or different halogen atoms -in particular fluorine, chlorine and/or bromine,
R¹⁰ furthermore represents alkenyl or alkinyl having in each case from 2 to 6 carbon atoms, which are in each case optionally substituted once to three times by identical or different halogens - in particular fluorine, chlorine and/or bromine;
R¹⁰ furthermore represents cycloalkyl having from 3 to 7 carbon atoms which is optionally substituted once to three times by identical or different substituents comprising halogen - in particular fluorine, chlorine and/or bromine - and/or straight-chain or branched alkyl having from 1 to 3 carbon atoms;
R¹⁰ furthermore represents phenylalkyl or phenyl having optionally from 1 to 3 carbon atoms in the straight-chain or branched alkyl moiety, which are in each case optionally substituted in the phenyl moiety once to five times by identical or different substituents, or represents a saturated or unsaturated, five- to seven-membered heterocyclyl radical having from 1 to 3 identical or different hetero atoms - in particular nitrogen, oxygen and/or sulphur - which is optionally substituted once to three times by identical or different substituents and/or is benzo-fused, possible substituents of phenyl or heterocyclyl being in each case:
fluorine, chlorine, bromine, cyano, nitro, amino, N-acetylamino, in each case straight-chain or branched alkyl, alkoxy, alkylthio, alkylsulphinyl or alkylsulphonyl having in each case from 1 to 4 carbon atoms, in each case straight-chain or branched halogenoalkyl, halogenoalkoxy, halogenoalkylthio, halogenoalkylsulphinyl or halogenoalkylsulphonyl having in each case from 1 to 4 carbon atoms and from 1 to 9 identical or different halogen atoms, in each case straight-chain or branched alkoxycarbonyl or alkoximinoalkyl having in each case from 1 to 4 carbon atoms in the individual alkyl moieties, and phenyl which is optionally substituted once to five times by identical or different substituents comprising fluorine, chlorine, bromine and/or straight-chain or branched alkyl or alkoxy having in each case from 1 to 4 carbon atoms and/or straight-chain or branched halogenoalkyl or halogenoalkoxy having in each case from 1 to 4 carbon atoms and from 1 to 9 identical or different halogen atoms;
^{R11} represents hydrogen;
R¹¹ furthermore represents straight-chain or branched alkyl having from 1 to 12 carbon atoms which is optionally substituted once or twice by identical or different substituents, possible substituents being:
cyano, carboxyl, carbamoyl, in each case straight-chain or branched alkoxy, alkoxyalkoxy, alkylthio, alkylsulphinyl, alkylsulphonyl, alkoxycarbonyl, N-alkylaminocarbonyl, N,N-dialkylaminocarbonyl or alkylsulphonylaminocarbonyl having in each case from 1 to 6 carbon atoms in the individual alkyl moieties, or heterocyclyl, the heterocyclyl radical being a five- to seven-membered, optionally benzo-fused, saturated or unsaturated heterocycle having from 1 to 3 identical or different hetero atoms - in particular nitrogen, oxygen and/or sulphur;
R¹¹ furthermore represents straight-chain or branched halogenoalkyl having from 1 to 6 carbon atoms and from 1 to 13 identical or different halogen atoms -in particular fluorine, chlorine and/or bromine,
R¹¹ furthermore represents alkenyl or alkinyl having in each case from 2 to 6 carbon atoms, which are in each case optionally substituted once to three times by identical or different halogens - in particular fluorine, chlorine and/or bromine;
R¹¹ furthermore represents cycloalkyl having from 3 to 7 carbon atoms which is optionally substituted once to three times by identical or different substituents comprising halogen - in particular fluorine, chlorine and/or bromine - and/or straight-chain or branched alkyl having from 1 to 3 carbon atoms;
R¹¹ furthermore represents phenylalkyl or phenyl having optionally from 1 to 3 carbon atoms in the straight-chain or branched alkyl moiety, which are in each case optionally substituted in the phenyl moiety once to five times by identical or different substituents, possible substituents of phenyl being in each case:
fluorine, chlorine, bromine, cyano, nitro, amino, N-acetylamino, in each case straight-chain or branched alkyl, alkoxy, alkylthio, alkylsulphinyl or alkylsulphonyl having in each case from 1 to 4 carbon atoms, in each case straight-chain or branched halogenoalkyl, halogenoalkoxy, halogenoalkylthio, halogenoalkylsulphinyl or halogenoalkylsulphonyl having in each case from 1 to 4 carbon atoms and from 1 to 9 identical or different halogen atoms, in each case straight-chain or branched alkoxycarbonyl or alkoximinoalkyl having in each case from 1 to 4 carbon atoms in the individual alkyl moieties, and phenyl which is optionally substituted once to five times by identical or different substituents comprising fluorine, chlorine, bromine and/or straight-chain or branched alkyl or alkoxy having in each case from 1 to 4 carbon atoms and/or straight-chain or branched halogenoalkyl or halogenoalkoxy having in each case from 1 to 4 carbon atoms and from 1 to 9 identical or different halogen atoms;
R¹² represents straight-chain or branched alkyl having from 1 to 12 carbon atoms which is optionally substituted once or twice by identical or different substituents, possible substituents being:
cycloalkyl having from 3 to 7 carbon atoms or heterocyclyl, the heterocyclyl radical being a five- to seven-membered, optionally benzo-fused, saturated or unsaturated heterocycle having from 1 to 3 identical or different hetero atoms - in particular nitrogen, oxygen and/or sulphur;
R¹² furthermore represents halogenoalkyl having from 1 to 6 carbon atoms and from 1 to 13 identical or different halogen atoms - in particular fluorine, chlorine and/or bromine,
R¹² furthermore represents cycloalkyl having from 3 to 7 carbon atoms which is optionally substituted once to three times by identical or different substituents comprising halogen - in particular fluorine, chlorine and/or bromine - and/or straight-chain or branched alkyl having from 1 to 3 carbon atoms;
R¹² furthermore represents phenylalkyl or phenyl having optionally from 1 to 3 carbon atoms in the straight-chain or branched alkyl moiety, which are in each case optionally substituted in the phenyl moiety once to five times by identical or different substituents, or represents a saturated or unsaturated, five- to seven-membered heterocyclyl radical having from 1 to 3 identical or different hetero atoms - in particular nitrogen, oxygen and/or sulphur - which is optionally substituted once to three times by identical or different substituents and/or is benzo-fused, possible substituents of phenyl or heterocyclyl being in each case:
fluorine, chlorine, bromine, cyano, nitro, amino, N-acetylamino, in each case straight-chain or branched alkyl, alkoxy, alkylthio, alkylsulphinyl or alkylsulphonyl having in each case from 1 to 4 carbon atoms, in each case straight-chain or branched halogenoalkyl, halogenoalkoxy, halogenoalkylthio, halogenoalkylsulphinyl or halogenoalkylsulphonyl having in each case from 1 to 4 carbon atoms and from 1 to 9 identical or different halogen atoms, in each case straight-chain or branched alkoxycarbonyl or alkoximinoalkyl having in each case from 1 to 4 carbon atoms in the individual alkyl moieties, and phenyl which is optionally substituted once to five times by identical or different substituents comprising fluorine, chlorine, bromine and/or straight-chain or branched alkyl or alkoxy having in each case from 1 to 4 carbon atoms and/or straight-chain or branched halogenoalkyl or halogenoalkoxy having in each case from 1 to 4 carbon atoms and from 1 to 9 identical or different halogen atoms and
n represents a number 0, 1 or 2.

3. Process for the preparation of substituted 1-aryltriazolinones of the general formula (I), 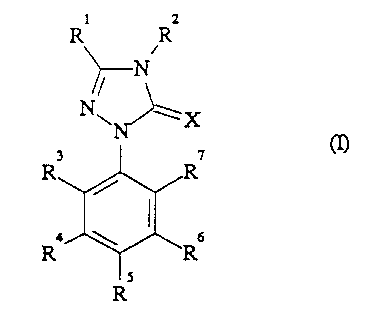 in which
R¹, R², R³, R⁴, R⁵, R⁶, R⁷ and X have the meanings given in Claim 1,
characterized in that
a) 1H-triazolinones of the formula (II), 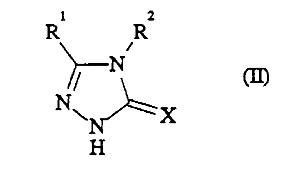 in which
R¹, R² and X have the meaning given above,
are reacted with halogenobenzene derivatives of the formula (III), 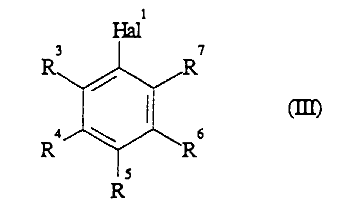 in which
R³, R⁴, R⁵, R⁶ and R⁷ have the meanings given above and
Hal1 represents halogen,
optionally in the presence of a diluent and optionally in the presence of a reaction auxiliary, or in that
b) substituted 1-aryltriazolinones of the formula (Ia), 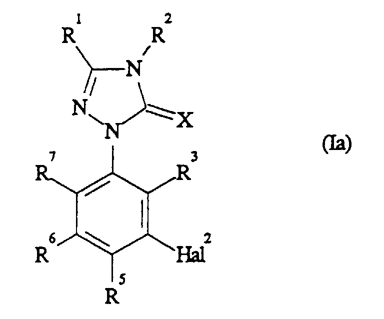 in which
R¹, R², R³, R⁶, R⁵, R⁷ and X have the meanings given above and
Hal² represents halogen,
are reacted with nucleophiles of the formula (IV),
H-R¹³ (IV)
in which
R¹³ represents a radical of the formula -O-R¹⁰, -S-R¹⁰ or -NR¹¹R¹⁰, where R¹⁰ and R¹¹ have the meanings given above,
optionally in the presence of a diluent and optionally in the presence of a reaction auxiliary, or in that
c) substituted triazolinones of the formula (V), 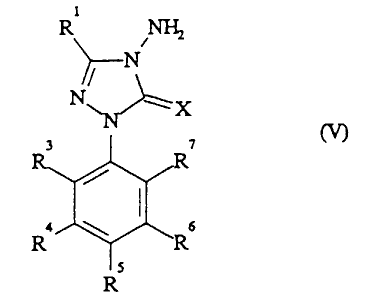 in which
R¹, R³, R⁴, R⁵, R⁶, R⁷ and X have the meanings given above
are reacted with alkylating, acylating or sulphonylating agents of the formula (VI),
R⁹-E (VI)
in which
R⁹ has the meaning given above and
E represents an electron-attracting leaving group,
optionally in the presence of a diluent and optionally in the presence of a reaction auxiliary, or in that
d) 4-alkylideneimino-triazolinones of the formula (VII), 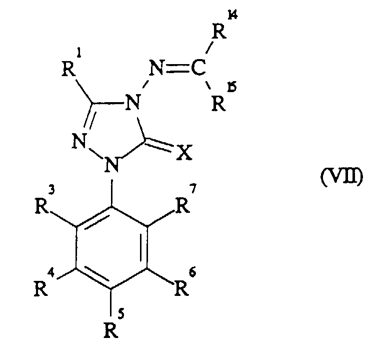 in which
R¹, R³, R⁴, R⁵, R⁶, R⁷ and X have the meanings given above,
R¹⁴ represents hydrogen or alkyl and
R¹⁵ represents alkyl or alkoxy,
are reacted with a reducing agent, optionally in the presence of a diluent and optionally in the presence of a reaction auxiliary.

4. Herbicide, characterized by a content of at least one substituted 1-aryltriazolinone of the formula (I) according to Claims 1 to 3.

5. Method of combating unwanted plants, characterized in that substituted 1-aryltriazolinones of the general formula (I) according to Claims 1 to 3 are allowed to act on plants and/or their habitat.

6. Use of substituted 1-aryltriazolinones of the general formula (I) according to Claims 1 to 3 for combating unwanted plants.

7. Process for the production of herbicides, characterized in that substituted 1-aryltriazolinones of the general formula (I) according to Claims 1 to 3 are mixed with extenders and/or surface-active substances.

8. Substituted 1-aryltriazolinones of the formula (Ia) 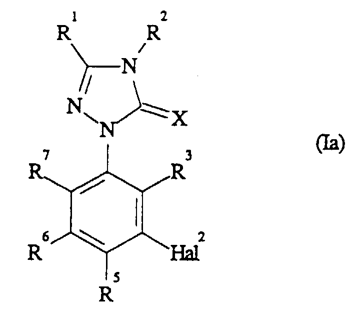 in which
R¹ represents hydrogen or represents in each case straight-chain or branched alkyl, alkoxy, alkylthio or alkylsulphonyl having in each case from 1 to 8 carbon atoms, furthermore represents straight-chain or branched halogenoalkyl having from 1 to 8 carbon atoms and from 1 to 17 identical or different halogen atoms, or represents cycloalkyl having from 3 to 8 carbon atoms,
R² represents a radical of the formula -NR⁸R⁹,
R³, R⁶ in each case represent hydrogen,
R⁷ represents hydrogen, halogen, amino or nitro,
Hal² represents halogen,
R⁵ represents cyan and
X represents oxygen or sulphur, where
R⁸ represents hydrogen, straight-chain or branched alkyl having from 1 to 8 carbon atoms or straight-chain or branched halogenoalkyl having from 1 to 8 carbon atoms and from 1 to 17 identical or different halogen atoms, and furthermore represents a radical of the formula -CO-R¹² or a radical of the formula -S(O)ₙ-R¹²,
R⁹ represents straight-chain or branched alkyl having from 1 to 8 carbon atoms or straight-chain or branched halogenoalkyl having from 1 to 8 carbon atoms and from 1 to 17 identical or different halogen atoms, and furthermore represents a radical of the formula -CO-R¹² or a radical of the formula -S(O)ₙ-R¹²,
R¹² represents straight-chain or branched alkyl having from 1 to 8 carbon atoms which is optionally substituted once or more than once by identical or different substituents, possible substituents being:
halogen - in particular fluorine, chlorine, bromine and/or iodine - cycloalkyl having from 3 to 8 carbon atoms or heterocyclyl, the heterocyclyl radical being a five- to seven-membered optionally benzo-fused, saturated or unsaturated heterocycle having from 1 to 3 identical or different hetero atoms - in particular nitrogen, oxygen and/or sulphur;
R¹² furthermore represents cycloalkyl having from 3 to 7 carbon atoms which is optionally substituted once or more than once by identical or different substituents comprising halogen - in particular fluorine, chlorine, bromine and/or iodine - and/or straight-chain or branched alkyl having from 1 to 4 carbon atoms;
R¹² furthermore represents arylalkyl or aryl having in each case from 6 to 10 carbon atoms in the aryl moiety and optionally from 1 to 4 carbon atoms in the straight-chain or branched alkyl moiety, which are in each case optionally substituted in the aryl moiety once or more than once by identical or different substituents, or represents a saturated or unsaturated, five- to seven-membered heterocyclyl radical having from 1 to 3 identical or different hetero atoms - in particular nitrogen, oxygen and/or sulphur - which is optionally substituted once or more than once by identical or different substituents, possible substituents of aryl or heterocyclyl being in each case:
halogen, cyano, nitro, amino, N-acetylamino, in each case straight-chain or branched alkyl, alkoxy, alkylthio, alkylsulphinyl or alkylsulphonyl having in each case from 1 to 6 carbon atoms, in each case straight-chain or branched halogenoalkyl, halogenoalkoxy, halogenoalkylthio, halogenoalkylsulphinyl or halogenoalkylsulphonyl having in each case from 1 to 6 carbon atoms and from 1 to 13 identical or different halogen atoms, in each case straight-chain or branched alkoxycarbonyl or alkoximinoalkyl having in each case from 1 to 6 carbon atoms in the individual alkyl moieties, and phenyl which is optionally substituted once or more than once by identical or different substituents comprising halogen and/or straight-chain or branched alkyl or alkoxy having in each case from 1 to 6 carbon atoms and/or straight-chain or branched halogenoalkyl or halogenoalkoxy having in each case from 1 to 6 carbon atoms and from 1 to 13 identical or different halogen atoms and
n represents a number 0, 1 or 2.

9. Substituted triazolinones of the formula (V) 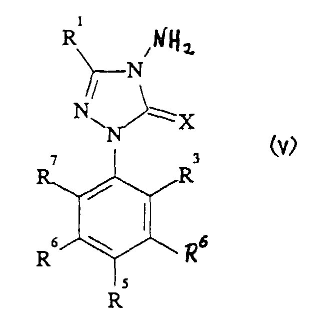 in which
R¹ represents hydrogen or represents in each case straight-chain or branched alkyl, alkoxy, alkylthio or alkylsulphonyl having in each case from 1 to 8 carbon atoms, furthermore represents straight-chain or branched halogenoalkyl having from 1 to 8 carbon atoms and from 1 to 17 identical or different halogen atoms, or represents cycloalkyl having from 3 to 8 carbon atoms,
R³, R⁶ in each case represent hydrogen,
R⁷ represents hydrogen, halogen, amino or nitro,
R⁴ represents hydrogen, fluorine, chlorine, bromine, iodine, cyano or nitro, or represents one of the radicals -R¹⁰, -O-R¹⁰, -S-R¹⁰, -S(O)-R¹⁰, -SO₂-R¹⁰, -SO₂-OR¹⁰, -SO₂-NR¹¹R¹⁰, -CO-OR¹⁰, -CO-NR¹¹R¹⁰, -O-SO₂-R¹⁰, -N(R¹¹)-SO₂-R¹⁰, -NR¹¹R¹⁰, -NH-P(O)(R¹¹)(OR¹⁰) or -NH-P(O)(OR¹¹)(OR¹⁰),
R⁵ represents cyan and
X represents oxygen or sulphur, where
R¹⁰ represents hydrogen;
R¹⁰ furthermore represents straight-chain or branched alkyl having from 1 to 14 carbon atoms which is optionally substituted once or more than once by identical or different substituents, possible substituents being:
halogen - in particular fluorine, chlorine, bromine and/or iodine - cyano, carboxyl, carbamoyl, in each case straight-chain or branched alkoxy, alkoxyalkoxy, alkylthio, alkylsulphinyl, alkylsulphonyl, alkoxycarbonyl, N-alkylaminocarbonyl, N,N-dialkylaminocarbonyl or alkylsulphonylaminocarbonyl having in each case from 1 to 8 carbon atoms in the individual alkyl moieties, or heterocyclyl, the heterocyclyl radical being a five- to seven-membered, optionally benzo-fused, saturated or unsaturated heterocycle having from 1 to 3 identical or different hetero atoms - in particular nitrogen, oxygen and/or sulphur;
R¹⁰ furthermore represents alkenyl or alkinyl having in each case from 2 to 8 carbon atoms, which are optionally substituted once or more than once by identical or different halogens - in particular fluorine, chlorine, bromine and/or iodine;
R¹⁰ furthermore represents cycloalkyl having from 3 to 7 carbon atoms which is optionally substituted once or more than once by identical or different substituents comprising halogen - in particular fluorine, chlorine, bromine and/or iodine - and/or straight-chain or branched alkyl having from 1 to 4 carbon atoms;
R¹⁰ furthermore represents arylalkyl or aryl having in each case from 6 to 10 carbon atoms in the aryl moiety and optionally from 1 to 4 carbon atoms in the straight-chain or branched alkyl moiety, which are in each case optionally substituted in the aryl moiety once or more than once by identical or different substituents, or represents a saturated or unsaturated, five- to seven-membered heterocyclyl radical having from 1 to 3 identical or different hetero atoms - in particular nitrogen, oxygen and/or sulphur - which is optionally substituted once or more than once by identical or different substituents and/or is benzo-fused, possible substituents of the aryl and/or heterocyclyl being in each case:
halogen, cyano, nitro, amino, N-acetylamino, in each case straight-chain or branched alkyl, alkoxy, alkylthio, alkylsulphinyl or alkylsulphonyl having in each case from 1 to 6 carbon atoms, in each case straight-chain or branched halogenoalkyl, halogenoalkoxy, halogenoalkylthio, halogenoalkylsulphinyl or halogenoalkylsulphonyl having in each case from 1 to 6 carbon atoms and from 1 to 13 identical or different halogen atoms, in each case straight-chain or branched alkoxycarbonyl or alkoximinoalkyl having in each case from 1 to 6 carbon atoms in the individual alkyl moieties, and phenyl which is optionally substituted once or more than once by identical or different substituents comprising halogen and/or straight-chain or branched alkyl or alkoxy having in each case from 1 to 6 carbon atoms and/or straight-chain or branched halogenoalkyl or halogenoalkoxy having in each case from 1 to 6 carbon atoms and from 1 to 13 identical or different halogen atoms;
R¹¹ represents hydrogen;
R¹¹ furthermore represents straight-chain or branched alkyl having from 1 to 14 carbon atoms which is optionally substituted once or more than once by identical or different substituents, possible substituents being:
halogen - in particular fluorine, chlorine, bromine and/or iodine - cyano, carboxyl, carbamoyl, in each case straight-chain or branched alkoxy, alkoxyalkoxy, alkylthio, alkylsulphinyl, alkylsulphonyl, alkoxycarbonyl, N-alkylaminocarbonyl, N,N-dialkylaminocarbonyl or alkylsulphonylaminocarbonyl having in each case from 1 to 8 carbon atoms in the individual alkyl moieties, or heterocyclyl, the heterocyclyl radical being a five- to seven-membered, optionally benzo-fused, saturated or unsaturated heterocycle having from 1 to 3 identical or different hetero atoms - in particular nitrogen, oxygen and/or sulphur;
R¹¹ furthermore represents alkenyl or alkinyl having in each case from 2 to 8 carbon atoms, which are optionally substituted once or more than once by identical or different halogens - in particular fluorine, chlorine, bromine and/or iodine;
R¹¹ furthermore represents cycloalkyl having from 3 to 7 carbon atoms which is optionally substituted once or more than once by identical or different substituents comprising halogen - in particular fluorine, chlorine, bromine and/or iodine - and/or straight-chain or branched alkyl having from 1 to 4 carbon atoms;
R¹¹ furthermore represents arylalkyl or aryl having in each case from 6 to 10 carbon atoms in the aryl moiety and optionally from 1 to 4 carbon atoms in the straight-chain or branched alkyl moiety, which are in each case optionally substituted in the aryl moiety once or more than once by identical or different substituents, possible substituents of the aryl being in each case:
halogen, cyano, nitro, amino, N-acetylamino, in each case straight-chain or branched alkyl, alkoxy, alkylthio, alkylsulphinyl or alkylsulphonyl having in each case from 1 to 6 carbon atoms, in each case straight-chain or branched halogenoalkyl, halogenoalkoxy, halogenoalkylthio, halogenoalkylsulphinyl or halogenoalkylsulphonyl having in each case from 1 to 6 carbon atoms and from 1 to 13 identical or different halogen atoms, in each case straight-chain or branched alkoxycarbonyl or alkoximinoalkyl having in each case from 1 to 6 carbon atoms in the individual alkyl moieties, and phenyl which is optionally substituted once or more than once by identical or different substituents comprising halogen and/or straight-chain or branched alkyl or alkoxy having in each case from 1 to 6 carbon atoms and/or straight-chain or branched halogenoalkyl or halogenoalkoxy having in each case from 1 to 6 carbon atoms and from 1 to 13 identical or different halogen atoms.

## Revendications

1. 1-aryltriazolinones substituées de formule générale (I) 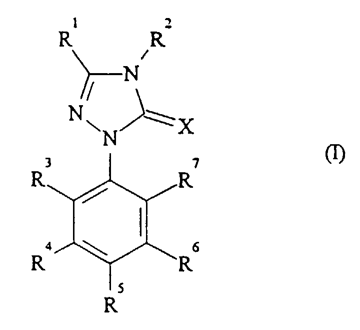 dans laquelle
R¹ est de l'hydrogène ou représente un groupe alkyle, alkoxy, alkylthio ou alkylsulfonyle étant chacun linéaire ou ramifié et ayant chacun 1 à 8 atomes de carbone, en outre un groupe halogénalkyle linéaire ou ramifié ayant 1 à 8 atomes de carbone et 1 à 17 atomes d'halogènes identiques ou différents, ou un groupe cycloalkyle ayant 3 à 8 atomes de carbone,
R² est un reste de formule -NR⁸R⁹,
R³, R⁶ représentent chacun de l'hydrogène,
R⁷ est de l'hydrogène, un halogène, un groupe amino ou nitro,
R⁴ est de l'hydrogène, du fluor, du chlore, du brome, de l'iode, un groupe cyano, nitro, ou l'un des restes -R¹⁰, -O-R¹⁰, -S-R¹⁰, -S(O)-R¹⁰, -SO₂R¹⁰, -SO₂-OR¹⁰, -SO₂NR¹¹R¹⁰, -CO-OR¹⁰, -CO-NR¹¹R¹⁰, -O-SO₂-R¹⁰, -N(R¹¹)-SO₂-R¹⁰, -NR¹¹R¹⁰, -NH-P(O)(R¹¹)OR¹⁰) ou -NH-P(O)OR¹¹)(OR¹⁰),
R⁵ est un groupe cyano et
X est de l'oxygène ou du soufre,
R⁸ est de l'hydrogène, un groupe alkyle linéaire ou ramifié ayant 1 à 8 atomes de carbone ou un groupe halogénalkyle linéaire ou ramifié ayant 1 à 8 atomes de carbone et 1 à 17 atomes d'halogènes identiques ou différents et, en outre, un reste de formule -CO-R¹² ou un reste de formule -S(O)ₙ-R¹²,
R⁹ représente un groupe alkyle linéaire ou ramifié ayant 1 à 8 atomes de carbone ou un groupe halogénalkyle linéaire ou ramifié ayant 1 à 8 atomes de carbone et 1 à 17 atomes d'halogènes identiques ou différents et, en outre, un reste de formule -CO-R¹² ou un reste de formule -S(O)ₙ-R¹²,
R¹⁰ est de l'hydrogène,
R¹⁰ représente en outre le cas échéant un groupe alkyle linéaire ou ramifié ayant 1 à 14 atomes de carbone, portant le cas échéant un ou plusieurs substituants identiques ou différents, avec comme substituants : un halogène - en particulier le fluor, le chlore, le brome et/ou l'iode -, un groupe cyano, carboxyle, carbamoyle, un groupe alkoxy, alkoxyalkoxy, alkylthio, alkylsulfinyle, alkylsulfonyle, alkoxycarbonyle, N-alkylaminocarbonyle, N,N-dialkylaminocarbonyle ou alkylsulfonylaminocarbonyle étant chacun linéaire ou ramifié et ayant chacun 1 à 8 atomes de carbone dans les parties alkyle individuelles ou un reste hétérocyclyle, ce reste hétérocyclyle étant un hétérocycle pentagonal à heptagonal saturé ou non saturé, éventuellement condensé au benzène, ayant 1 à 3 hétéroatomes - notamment azote, oxygène, et/ou soufre - identiques ou différents ;
R¹⁰ représente en outre un groupe alcényle ou un groupe alcynyle ayant chacun 2 à 8 atomes de carbone et portant chacun le cas échéant un à plusieurs substituants halogéno - notamment fluoro, chloro, bromo et/ou iodo - identiques ou différents ;
R¹⁰ représente en outre un groupe cycloalkyle de 3 à 7 atomes de carbone éventuellement substitué une ou plusieurs fois identiques ou différentes par un halogène - notamment fluor, chlore, brome et/ou iode - et/ou par un groupe alkyle linéaire ou ramifié ayant 1 à 4 atomes de carbone ;
R¹⁰ représente en outre un groupe arylalkyle ou un groupe aryle ayant chacun 6 à 10 atomes de carbone dans la partie aryle et le cas échéant 1 à 4 atomes de carbone dans la partie alkyle linéaire ou ramifiée et portant chacun le cas échéant dans la partie aryle un ou plusieurs substituants identiques ou différents, ou un reste hétérocyclyle pentagonal à heptagonal, saturé ou non saturé, portant le cas échéant un ou plusieurs substituants identiques ou différents et/ou condensé au benzène, ayant 1 à 3 hétéroatomes - notamment azote, oxygène et/ou soufre - identiques ou différents, en considérant dans chaque cas comme substituants des restes aryle et hétérocyclyle :
un halogène, un groupe cyano, nitro, amino, N-acétylamino, un groupe alkyle, alkoxy, alkylthio, alkylsulfinyle ou alkylsulfonyle étant chacun linéaire ou ramifié et ayant chacun 1 à 6 atomes de carbone, un groupe halogénalkyle, halogénalkoxy, halogénalkylthio, halogénalkylsulfinyle ou halogénalkylsulfonyle étant chacun linéaire ou ramifié et ayant chacun 1 à 6 atomes de carbone et 1 à 13 atomes d'halogènes identiques ou différents, un groupe alkoxycarbonyle ou alkoximinoalkyle étant chacun linéaire ou ramifié et ayant chacun 1 à 6 atomes de carbone dans les parties alkyle individuelles, ainsi qu'un groupe phényle éventuellement substitué une ou plusieurs fois identiques ou différentes par un halogène et/ou un radical alkyle ou alkoxy linéaire ou ramifié ayant chacun 1 à 6 atomes de carbone et/ou un radical halogénalkyle ou halogénalkoxy linéaire ou ramifié ayant chacun 1 à 6 atomes de carbone et 1 à 13 atomes d'halogènes identiques ou différents ;
R¹¹ est de l'hydrogène ;
R¹¹ représente en outre un groupe alkyle linéaire ou ramifié ayant 1 à 14 atomes de carbone, portant le cas échéant un ou plusieurs substituants identiques ou différents, en considérant comme substituants :
un halogène - notamment le fluor, le chlore, le brome et/ou l'iode -, un groupe cyano, carboxyle, carbamoyle, un groupe alkoxy, alkoxyalkoxy, alkylthio, alkylsulfinyle, alkylsulfonyle, alkoxycarbonyle, N-alkylaminocarbonyle, N,N-dialkylaminocarbonyle ou alkylsulfonylaminocarbonyle étant chacun linéaire ou ramifié et ayant chacun 1 à 8 atomes de carbone dans les parties alkyle individuelles ou un reste hétérocyclyle, le reste hétérocyclyle étant un hétérocycle pentagonal à heptagonal, saturé ou non saturé, éventuellement condensé au benzène, ayant 1 à 3 hétéroatomes - notamment azote, oxygène et/ou soufre - identiques ou différents ;
R¹¹ représente en outre un groupe alcényle ou alcynyle portant chacun le cas échéant un ou plusieurs substituants halogéno - notamment fluoro, chloro, bromo et/ou iodo - identiques ou différents et ayant chacun 2 à 8 atomes de carbone ;
R¹¹ représente en outre un groupe cycloalkyle de 3 à 7 atomes de carbone portant le cas échéant un ou plusieurs substituants, identiques ou différents, halogéno - notamment fluoro, chloro, bromo et/ou iodo - et/ou alkyle linéaire ou ramifié ayant 1 à 4 atomes de carbone ;
R¹¹ représente en outre un groupe arylalkyle ou aryle ayant chacun 6 à 10 atomes de carbone dans la partie aryle et le cas échéant 1 à 4 atomes de carbone dans la partie alkyle linéaire ou ramifiée et portant chacun le cas échéant dans la partie aryle un ou plusieurs substituants identiques ou différents, en considérant dans chaque cas comme substituants de la partie aryle :
un halogène, un groupe cyano, nitro, amino, N-acétylamino, un groupe alkyle, alkoxy, alkylthio, alkylsulfinyle ou alkylsulfonyle étant chacun linéaire ou ramifié et ayant chacun 1 à 6 atomes de carbone, un groupe halogénalkyle, halogénalkoxy, halogénalkylthio, halogénalkylsulfinyle ou halogénalkylsulfonyle étant chacun linéaire ou ramifié et ayant chacun 1 à 6 atomes de carbone et 1 à 13 atomes d'halogènes identiques ou différents, un groupe alkoxycarbonyle ou alkoximinoalkyle étant chacun linéaire ou ramifié et ayant chacun 1 à 6 atomes de carbone dans les parties alkyle individuelles, ainsi qu'un groupe phényle portant le cas échéant un ou plusieurs substituants, identiques ou différents, halogéno et/ou alkyle ou alkoxy linéaire ou ramifié ayant chacun 1 à 6 atomes de carbone et/ou halogénalkyle ou halogénalkoxy linéaire ou ramifié ayant chacun 1 à 6 atomes de carbone et 1 à 13 atomes d'halogènes identiques ou différents ;
R¹² représente un groupe alkyle linéaire ou ramifié ayant 1 à 8 atomes de carbone, portant le cas échéant un ou plusieurs substituants identiques ou différents, en considérant comme substituants :
un halogène - notamment le fluor, le chlore, le brome et/ou l'iode -, un groupe cycloalkyle ayant 3 à 8 atomes de carbone ou un reste hétérocyclyle, ce reste hétérocyclyle étant un hétérocycle saturé ou non saturé, pentagonal à heptagonal, éventuellement condensé au benzène, ayant 1 à 3 hétéroatomes - notamment azote, oxygène et/ou soufre - identiques ou différents ;
R¹² représente en outre un groupe cycloalkyle de 3 à 7 atomes de carbone portant éventuellement un ou plusieurs substituants, identiques ou différents, halogéno - notamment fluoro, chloro, bromo et/ou iodo - et/ou alkyle linéaire ou ramifié ayant 1 à 4 atomes de carbone,
R¹² représente en outre un groupe arylalkyle ou aryle ayant chacun 6 à 10 atomes de carbone dans la partie aryle et le cas échéant 1 à 4 atomes de carbone dans la partie alkyle linéaire ou ramifié et portant chacun le cas échéant dans la partie aryle un ou plusieurs substituants identiques ou différents, ou un reste hétérocyclyle pentagonal à heptagonal, saturé ou non saturé, portant le cas échéant un ou plusieurs substituants identiques ou différents et/ou condensé au benzène, ayant 1 à 3 hétéroatomes - notamment azote, oxygène et/ou soufre - identiques ou différents, en considérant comme substituants des restes aryle et hétérocyclyle :
un halogène, un groupe cyano, nitro, amino, N-acétylamino, un groupe alkyle, alkoxy, alkylthio, alkylsulfinyle ou alkylsulfonyle étant chacun linéaire ou ramifié et ayant chacun 1 à 6 atomes de carbone, un groupe halogénalkyle, halogénalkoxy, halogénalkylthio, halogénalkylsulfinyle ou halogénalkylsulfonyle étant chacun linéaire ou ramifié et ayant chacun 1 à 6 atomes de carbone et 1 à 13 atomes d'halogènes identiques ou différents, un groupe alkoxycarbonyle ou alkoximinoalkyle étant chacun linéaire ou ramifié et ayant chacun 1 à 6 atomes de carbone dans les parties alkyle individuelles, ainsi qu'un groupe phényle portant le cas échéant un ou plusieurs substituants, identiques ou différents, halogéno et/ou alkyle ou alkoxy linéaire ou ramifié ayant chacun 1 à 6 atomes de carbone et/ou halogénalkyle ou halogénalkoxy linéaire ou ramifié ayant chacun 1 à 6 atomes de carbone et 1 à 13 atomes d'halogènes identiques ou différents, et
n représente le nombre 0, 1 ou 2.

2. 1-aryltriazolinones substituées de formule générale (I) suivant la revendication 1, caractérisées en ce que
R¹ est de l'hydrogène ou un groupe alkyle, alkoxy, alkylthio ou alkylsulfonyle étant chacun linéaire ou ramifié et ayant chacun 1 à 6 atomes de carbone, en outre un groupe halogénalkyle linéaire ou ramifié ayant 1 à 6 atomes de carbone et 1 à 13 atomes d'halogènes - notamment fluor, chlore ou brome - identiques ou différents ou un groupe cycloalkyle ayant 3 à 7 atomes de carbone,
R² est un reste de formule -NR⁸R⁹,
R³, R⁶ représentent chacun de l'hydrogène,
R⁷ est de l'hydrogène, un halogène, un groupe amino ou nitro,
R⁴ est de l'hydrogène, du fluor, du chlore, du brome, un groupe cyano, nitro ou l'un des restes -R¹⁰, -O-R¹⁰, -S-R¹⁰, -S(O)-R¹⁰, -SO₂R¹⁰, -SO₂-OR¹⁰, -SO₂NR¹¹R¹⁰, -CO-OR¹⁰, -CO-N¹¹R¹⁰, -O-SO₂-R¹⁰, -N(R¹¹)-SO₂R¹⁰, -NR¹¹R¹⁰, -NH-P(O)(R¹¹)(OR¹⁰) ou -NH-P(O)(OR¹¹)(OR^{10),}
R⁵ est un groupe cyano et
X est de l'oxygène ou du soufre,
R⁸ représente de l'hydrogène, un groupe alkyle linéaire ou ramifié ayant 1 à 6 atomes de carbone ou un groupe halogénalkyle linéaire ou ramifié ayant 1 à 6 atomes de carbone et 1 à 13 atomes d'halogènes - notamment fluor, chlore ou brome - identiques ou différents, et en outre un reste de formule -CO-R¹² ou un reste de formule -S(O)ₙ-R¹²,
R⁹ est un groupe alkyle linéaire ou ramifié ayant 1 à 6 atomes de carbone ou un groupe halogénalkyle linéaire ou ramifié ayant 1 à 6 atomes de carbone et 1 à 13 atomes d'halogènes - notamment fluor, chlore ou brome - identiques ou différents et représentent en outre un reste de formule -CO-R¹² ou un reste de formule -S(O)ₙ-R¹²,
R¹⁰ est de l'hydrogène ;
R¹⁰ représente en outre un groupe alkyle linéaire ou ramifié ayant 1 à 12 atomes de carbone, portant le cas échéant un ou deux substituants identiques ou différents, en considérant comme substituants :
un groupe cyano, carboxyle, carbamoyle, un groupe alkoxy, alkoxyalkoxy, alkylthio, alkylsulfinyle, alkylsulfonyle, alkoxycarbonyle, N-alkylaminocarbonyle, N,N-dialkylaminocarbonyle ou alkylsulfonylaminocarbonyle étant chacun linéaire ou ramifié et ayant chacun 1 à 6 atomes de carbone dans les parties alkyle individuelles ou un reste hétérocyclyle, ce reste hétérocyclyle étant un hétérocycle saturé ou non saturé, pentagonal à heptagonal, éventuellement condensé au benzène, ayant 1 à 3 hétéroatomes - notamment azote, oxygène et/ou soufre - identiques ou différents ;
R¹⁰ représente en outre un groupe halogénalkyle linéaire ou ramifié ayant 1 à 6 atomes de carbone et 1 à 13 atomes d'halogènes - notamment fluor, chlore et/ou brome - identiques ou différents,
R¹⁰ est en outre un groupe alcényle ou un groupe alcynyle ayant chacun 2 à 6 atomes de carbone et portant chacun le cas échéant un à trois substituants halogéno - notamment fluoro, chloro et/ou bromo - identiques ou différents ;
R¹⁰ représente en outre un groupe cycloalkyle de 3 à 7 atomes de carbone portant éventuellement un à trois substituants, identiques ou différents, halogéno - notamment fluoro, chloro et/ou bromo- et/ou alkyle linéaire ou ramifié ayant 1 à 3 atomes de carbone ;
R¹⁰ représente en outre un groupe phénylalkyle ou phényle portant chacun le cas échéant un à cinq substituants identiques ou différents dans la partie phényle et ayant éventuellement un 1 à 3 atomes de carbone dans la partie alkyle linéaire ou ramifiée, ou un reste hétérocyclyle pentagonal à heptagonal, saturé ou non saturé, portant le cas échéant 1 à 3 substituants identiques ou différents et/ou éventuellement condensé au benzène, avec 1 à 3 hétéroatomes - notamment azote, oxygène et/ou soufre - identiques ou différents, en considérant dans chaque cas comme substituants des restes phényle et hétérocyclyle :
le fluor, le chlore, le brome, un groupe cyano, nitro, amino, N-acétylamino, un groupe alkyle, alkoxy, alkylthio, alkylsulfinyle ou alkylsulfonyle étant chacun linéaire ou ramifié et ayant chacun 1 à 4 atomes de carbone, un groupe halogénalkyle, halogénalkoxy, halogénalkylthio, halogénalkylsulfinyle ou halogénalkylsulfonyle étant chacun linéaire ou ramifié et ayant chacun 1 à 4 atomes de carbone et 1 à 9 atomes d'halogènes identiques ou différents, un groupe alkoxycarbonyle ou alkoximinoalkyle étant chacun linéaire ou ramifié et ayant chacun 1 à 4 atomes de carbone dans les parties alkyle individuelles, ainsi qu'un groupe phényle portant le cas échéant 1 à 5 substituants, identiques ou différents, fluoro, chloro, bromo et/ou alkyle ou alkoxy linéaire ou ramifié ayant chacun 1 à 4 atomes de carbone et/ou halogénalkyle ou halogénalkoxy linéaire ou ramifié ayant chacun 1 à 4 atomes de carbone et 1 à 9 atomes d'halogènes identiques ou différents ;
R¹¹ représente de l'hydrogène ;
R¹¹ est en outre un groupe alkyle linéaire ou ramifié de 1 à 12 atomes de carbone, portant le cas échéant un ou deux substituants identiques ou différents, en considérant comme substituants :
un groupe cyano, carboxyle, carbamoyle, un groupe alkoxy, alkoxyalkoxy, alkylthio, alkylsulfinyle, alkylsulfonyle, alkoxycarbonyle, N-alkylaminocarbonyle, N,N-dialkylaminocarbonyle ou alkylsulfonylaminocarbonyle étant chacun linéaire ou ramifié et ayant chacun 1 à 6 atomes de carbone dans les parties alkyle individuelles, ou un reste hétérocyclyle, ce reste hétérocyclyle étant un hétérocycle pentagonal à heptagonal, saturé ou non saturé, éventuellement condensé au benzène, ayant 1 à 3 hétéroatomes - notamment azote, oxygène et/ou soufre - identiques ou différents ;
R¹¹ représente en outre un groupe halogénalkyle linéaire ou ramifié ayant 1 à 6 atomes de carbone et 1 à 13 atomes d'halogènes - notamment fluor, chlore et/ou brome - identiques ou différents,
R¹¹ représente en outre un groupe alcényle ou un groupe alcynyle ayant chacun 2 à 6 atomes de carbone et portant chacun le cas échéant 1 à 3 substituants halogéno - notamment fluoro, chloro et/ou bromo - identiques ou différents ;
R¹¹ représente en outre un groupe cycloalkyle de 3 à 7 atomes de carbone portant le cas échéant 1 à 3 substituants, identiques ou différents, halogéno - notamment fluoro, chloro et/ou bromo - et/ou alkyle linéaire ou ramifié ayant 1 à 3 atomes de carbone ;
R¹¹ représente en outre un groupe phénylalkyle ou un groupe phényle portant chacun le cas échéant dans la partie phényle 1 à 5 substituants identiques ou différents, avec le cas échéant 1 à 3 atomes de carbone dans la partie alkyle linéaire ou ramifié, en considérant dans chaque cas comme substituants de la partie phényle :
le fluor, le chlore, le brome, un groupe cyano, nitro, amino, N-acétylamino, un groupe alkyle, alkoxy, alkylthio, alkylsulfinyle ou alkylsulfonyle étant chacun linéaire ou ramifié et ayant chacun 1 à 4 atomes de carbone, un groupe halogénalkyle, halogénalkoxy, halogénalkylthio, halogénalkylsulfinyle ou halogénalkylsulfonyle étant chacun linéaire ou ramifié et ayant chacun 1 à 4 atomes de carbone et 1 à 9 atomes d'halogènes identiques ou différents, un groupe alkoxycarbonyle ou alkoximinoalkyle étant chacun linéaire ou ramifié et ayant chacun 1 à 4 atomes de carbone dans les parties alkyle individuelles, ainsi qu'un groupe phényle portant le cas échéant 1 à 5 substituants, identiques ou différents, fluoro, chloro, bromo et/ou alkyle ou alkoxy linéaire ou ramifié ayant chacun 1 à 4 atomes de carbone et/ou halogénalkyle ou halogénalkoxy linéaire ou ramifié ayant 1 à 4 atomes de carbone et 1 à 9 atomes d'halogènes identiques ou différents ;
R¹² est un groupe alkyle linéaire ou ramifié ayant 1 à 12 atomes de carbone, portant le cas échéant un ou deux substituants identiques ou différents, en considérant comme substituants :
un groupe cycloalkyle ayant 3 à 7 atomes de carbone ou un reste hétérocyclyle, ce reste hétérocyclyle étant un hétérocycle saturé ou non saturé, pentagonal à heptagonal, éventuellement condensé au benzène, ayant 1 à 3 hétéroatomes, notamment azote, oxygène et/ou soufre - identiques ou différents ;
R¹² représente en outre un groupe halogénalkyle linéaire ou ramifié ayant 1 à 6 atomes de carbone et 1 à 13 atomes d'halogènes - notamment fluor, chlore et/ou brome - identiques ou différents,
R¹² représente en outre un groupe cycloalkyle de 3 à 7 atomes de carbone portant le cas échéant 1 à 3 substituants, identiques ou différents, halogéno - notamment fluoro, chloro et/ou bromo - et/ou alkyle linéaire ou ramifié ayant 1 à 3 atomes de carbone ;
R¹² représente en outre un groupe phénylalkyle ou un groupe phényle portant chacun le cas échéant dans la partie phényle 1 à 5 substituants identiques ou différents avec le cas échéant 1 à 3 atomes de carbone dans la partie alkyle linéaire ou ramifiée, ou un reste hétérocyclyle pentagonal à heptagonal, saturé ou non saturé, éventuellement porteur de 1 à 3 substituants identiques ou différents et/ou condensé au benzène, avec 1 à 3 hétéroatomes - notamment azote, oxygène et/ou soufre - identiques ou différents, en considérant dans chaque cas comme substituants des restes phényle et hétérocyclyle :
le fluor, le chlore, le brome, un groupe cyano, nitro, amino, N-acétylamino, un groupe alkyle, alkoxy, alkylthio, alkylsulfinyle ou alkylsulfonyle étant chacun linéaire ou ramifié et ayant chacun 1 à 4 atomes de carbone, un groupe halogénalkyle, halogénalkoxy, halogénalkylthio, halogénalkylsulfinyle ou halogénalkylsulfonyle étant chacun linéaire ou ramifié et ayant chacun 1 à 4 atomes de carbone et 1 à 9 atomes d'halogènes identiques ou différents, un groupe alkoxycarbonyle ou alkoximinoalkyle étant chacun linéaire ou ramifié et ayant chacun 1 à 4 atomes de carbone dans les parties alkyle individuelles ainsi qu'un groupe phényle portant le cas échéant 1 à 5 substituants, identiques ou différents, fluoro, chloro, bromo et/ou alkyle ou alkoxy linéaire ou ramifié ayant chacun 1 à 4 atomes de carbone et/ou halogénalkyle ou halogénalkoxy linéaire ou ramifié ayant chacun 1 à 4 atomes de carbone et 1 à 9 atomes d'halogènes identiques ou différents, et
n représente le nombre 0, 1 ou 2.

3. Procédé de production de 1-aryltriazolinones substituées de formule générale (I) 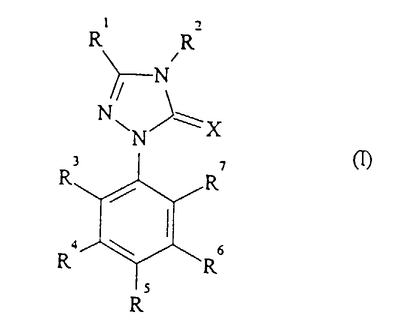 dans laquelle
R¹, R², R³, R⁴, R⁵, R⁶, R⁷ et X ont les définitions indiquées dans la revendication 1,
caractérisé en ce que :
a) on fait réagir des 1H-triazolinones de formule (II) , 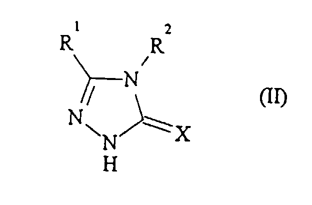 dans laquelle
R¹, R² et X ont la définition indiquée ci-dessus,
avec des dérivés halogénobenzéniques de formule (III), 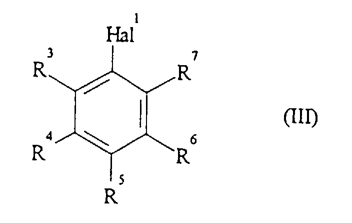 dans laquelle
R³, R⁴, R⁵, R⁶ et R⁷ ont les définitions indiquées ci-dessus et Hal1 représente un halogène,
le cas échéant en présence d'un diluant et en la présence éventuelle d'un auxiliaire de réaction, ou en ce que :
b) on fait réagir des 1-aryltriazolinones substituées de formule (Ia), 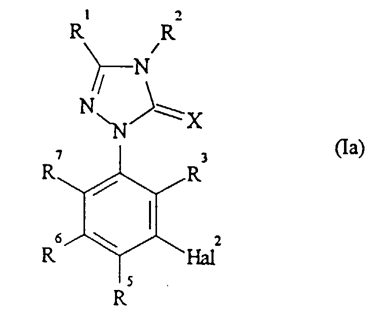 dans laquelle
R¹, R², R³, R⁶, R⁵, R⁷ et X ont les définitions indiquées ci-dessus et Hal² est un halogène,
avec des nucléophiles de formule (IV)
H-R¹³ (IV)
dans laquelle
R¹³ représente un reste de formule -O-R¹⁰, -S-R¹⁰ ou -NR¹¹R¹⁰, où R¹⁰ et R¹¹ ont les définitions indiquées ci-dessus,
le cas échéant en présence d'un diluant et en la présence éventuelle d'un auxiliaire de réaction, ou en ce que
c) on fait réagir des triazolinones substituées de formule (V), 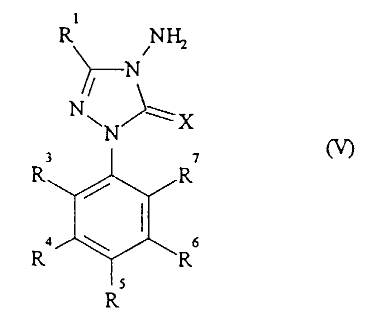 dans laquelle
R¹, R³, R⁴, R⁵, R⁶, R⁷ et X ont les définitions indiquées ci-dessus,
avec des agents d'alkylation, d'acylation ou de sulfonylation de formule (VI)
R⁹-E (VI)
dans laquelle
R⁹ a la définition indiquée ci-dessus et
E est un groupe partant attirant les électrons,
le cas échéant en présence d'un diluant et en la présence éventuelle d'un auxiliaire de réaction, ou en ce que :
d) on fait réagir des 4-alkylidène-iminotriazolinones de formule (VII), 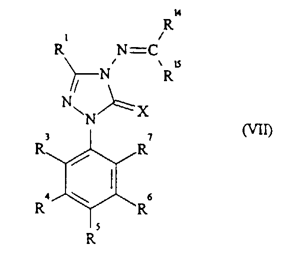 dans laquelle
R¹, R³, R⁴, R⁵, R⁶, R⁷ et X ont les définitions indiquées ci-dessus,
R¹⁴ est de l'hydrogène ou un groupe alkyle et
R¹⁵ représente un groupe alkyle ou alkoxy,
avec un agent réducteur, éventuellement en présence d'un diluant et, le cas échéant, en présence d'un auxiliaire de réaction.

4. Compositions herbicides, caractérisées par une teneur en au moins une 1-aryltriazolinone substituée de formule (I) suivant les revendications 1 à 3.

5. Procédé pour combattre des plantes indésirables, caractérisé en ce qu'on fait agir des 1-aryltriazolinones substituées de formule générale (I) suivant les revendications 1 à 3 sur les plantes et/ou sur leur milieu.

6. Utilisation de 1-aryltriazolinones substituées de formule générale (I) suivant les revendications 1 à 3 pour combattre des plantes indésirables.

7. Procédé de préparation de compositions herbicides, caractérisé en ce qu'on mélange des 1-aryltriazolinones substituées de formule générale (I) suivant les revendications 1 à 3 avec des diluants et/ou des agents tensio-actifs.

8. 1-aryltriazolinones substituées de formule (Ia) 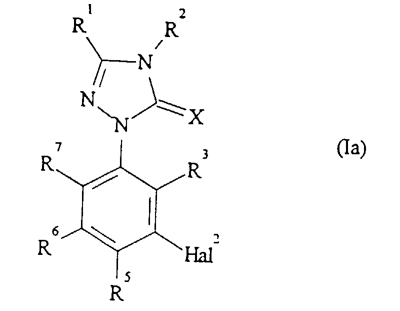 dans laquelle
R¹ représente de l'hydrogène ou un groupe alkyle, alkoxy, alkylthio ou alkylsulfonyle étant chacun linéaire ou ramifié et ayant chacun 1 à 8 atomes de carbone, en outre un groupe halogénalkyle linéaire ou ramifié ayant 1 à 8 atomes de carbone et 1 à 17 atomes d'halogènes identiques ou différents ou un groupe cycloalkyle ayant 3 à 8 atomes de carbone,
R² est un reste de formule -NR⁸R⁹,
R³, R⁶ représentent chacun de l'hydrogène,
R⁷ est de l'hydrogène, un halogène, un groupe amino ou nitro,
Hal² est un halogène,
R⁵ est un groupe cyano et
X est de l'oxygène ou du soufre,
R⁸ est de l'hydrogène, un groupe alkyle linéaire ou ramifié ayant 1 à 8 atomes de carbone ou un groupe halogénalkyle linéaire ou ramifié ayant 1 à 8 atomes de carbone et 1 à 17 atomes d'halogènes identiques ou différents et, en outre, un reste de formule -CO-R¹² ou un reste de formule -S(O)ₙ-R¹²,
R⁹ est un groupe alkyle linéaire ou ramifié ayant 1 à 8 atomes de carbone ou un groupe halogénalkyle linéaire ou ramifié ayant 1 à 8 atomes de carbone et 1 à 17 atomes d'halogènes identiques ou différents et, en outre, un reste de formule -CO-R¹² ou un reste de formule -S(O)ₙ-R¹²,
R¹² est un groupe alkyle linéaire ou ramifié ayant 1 à 8 atomes de carbone, portant le cas échéant un ou plusieurs substituants identiques ou différents, en considérant comme substituants :
un halogène - notamment le fluor, le chlore, le brome et/ou l'iode -, un groupe cycloalkyle ayant 3 à 8 atomes de carbone ou un reste hétérocyclyle, ce reste hétérocyclyle étant un hétérocycle saturé ou non saturé, pentagonal à heptagonal, éventuellement condensé au benzène, ayant 1 à 3 hétéroatomes - notamment azote, oxygène et/ou soufre - identiques ou différents ;
R¹² représente en outre un groupe cycloalkyle de 3 à 7 atomes de carbone portant le cas échéant un ou plusieurs substituants, identiques ou différents, halogéno - notamment fluoro, chloro, bromo et/ou iodo - et/ou alkyle linéaire ou ramifié ayant 1 à 4 atomes de carbone ;
R¹² représente en outre un groupe arylalkyle ou un groupe aryle portant chacun le cas échéant dans la partie aryle un ou plusieurs substituants identiques ou différents et ayant chacun 6 à 10 atomes de carbone dans la partie aryle et le cas échéant 1 à 4 atomes de carbone dans la partie alkyle linéaire linéaire ou ramifié, ou un reste hétérocyclyle pentagonal à heptagonal, saturé ou non saturé, éventuellement porteur d'un ou plusieurs substituants identiques ou différents et/ou condensé au benzène, ayant 1 à 3 hétéroatomes - notamment azote, oxygène et/ou soufre - identiques ou différents, en considérant dans chaque cas comme substituants des restes aryle et hétérocyclyle :
un halogène, un groupe cyano, nitro, amino, N-acétylamino, un groupe alkyle, alkoxy, alkylthio, alkylsulfinyle ou alkylsulfonyle étant chacun linéaire ou ramifié et ayant chacun 1 à 6 atomes de carbone, un groupe halogénalkyle, halogénalkoxy, halogénalkylthio, halogénalkylsulfinyle ou halogénalkylsulfonyle étant chacun linéaire ou ramifié et ayant chacun 1 à 6 atomes de carbone et 1 à 13 atomes d'halogènes identiques ou différents, un groupe alkoxycarbonyle ou alkoximinoalkyle étant chacun linéaire ou ramifié et ayant chacun 1 à 6 atomes de carbone dans les parties alkyle individuelles ainsi qu'un groupe phényle portant le cas échéant un ou plusieurs substituants, identiques ou différents, halogéno et/ou alkyle ou alkoxy linéaire ou ramifié ayant chacun 1 à 6 atomes de carbone et/ou halogénalkyle ou halogénalkoxy linéaire ou ramifié ayant chacun 1 à 6 atomes de carbone et 1 à 13 atomes d'halogènes identiques ou différents, et
n représente le nombre 0, 1 ou 2.

9. Triazolinones substituées de formule (V) 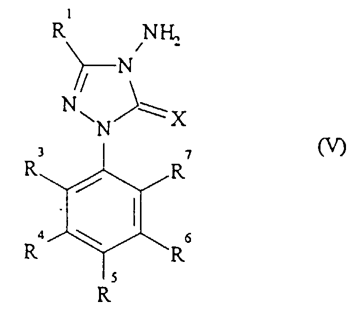 dans laquelle
R¹ représente de l'hydrogène ou un groupe alkyle, alkoxy, alkylthio ou alkylsulfonyle étant chacun linéaire ou ramifié et ayant chacun 1 à 8 atomes de carbone, en outre un groupe halogénalkyle linéaire ou ramifié ayant 1 à 8 atomes de carbone et 1 à 17 atomes d'halogènes identiques ou différents ou un groupe cycloalkyle ayant 3 à 8 atomes de carbone,
R³, R⁶ représentent chacun de l'hydrogène,
R⁷ est de l'hydrogène, un halogène, un groupe amino ou nitro,
R⁴ est de l'hydrogène, du fluor, du chlore, du brome, de l'iode, un groupe cyano, nitro ou l'un des restes -R¹⁰, -O-R¹⁰, -S-R¹⁰, -S(O)-R¹⁰, -SO₂R¹⁰, -SO₂-OR¹⁰, -SO₂NR¹¹R¹⁰, -CO-OR¹⁰, -CO-NR¹¹R¹⁰, -O-SO₂-R¹⁰, -N(R¹¹)-SO₂-R¹⁰, -NR¹¹R¹⁰, -NH-P(O)(R¹¹)(OR¹⁰) ou -NH-P(O)(OR¹¹)(OR¹⁰),
R⁵ est un groupe cyano et
X est de l'oxygène ou du soufre,
R¹⁰ est de l'hydrogène ;
R¹⁰ représente en outre un groupe alkyle linéaire ou ramifié ayant 1 à 14 atomes de carbone et portant le cas échéant un ou plusieurs substituants identiques ou différents, en considérant comme substituants : un halogène - notamment le fluor, le chlore, le brome et/ou l'iode -, un groupe cyano, carboxyle, carbamoyle, un groupe alkoxy, alkoxyalkoxy, alkylthio, alkylsulfinyle, alkylsulfonyle, alkoxycarbonyle, N-alkylaminocarbonyle, N,N-dialkylaminocarbonyle ou alkylsulfonylaminocarbonyle étant chacun linéaire ou ramifié et ayant chacun 1 à 8 atomes de carbone dans les parties alkyle individuelles ou un reste hétérocyclyle, ce reste hétérocyclyle étant un hétérocycle pentagonal à heptagonal, saturé ou non saturé, éventuellement condensé au benzène, ayant 1 à 3 hétéroatomes - notamment azote, oxygène et/ou soufre - identiques ou différents ;
R¹⁰ représente en outre un groupe alcényle ou un groupe alcynyle ayant chacun 2 à 8 atomes de carbone et portant chacun le cas échéant un ou plusieurs subtituants halogéno - notamment fluoro, chloro, bromo et/ou iodo - identiques ou différents ;
R¹⁰ représente en outre un groupe cycloalkyle de 3 à 7 atomes de carbone portant le cas échéant un ou plusieurs substituants, identiques ou différents, halogéno - notamment fluoro, chloro, bromo et/ou iodo - et/ou alkyle linéaire ou ramifié ayant 1 à 4 atomes de carbone,
R¹⁰ représente en outre un groupe arylalkyle ou aryle portant chacun le cas échéant dans la partie aryle un ou plusieurs substituants identiques ou différents et ayant chacun 6 à 10 atomes de carbone dans la partie aryle et le cas échéant 1 à 4 atomes de carbone dans la partie alkyle linéaire ou ramifiée, ou un reste hétérocyclyle pentagonal à heptagonal, saturé ou non saturé, éventuellement porteur d'un ou plusieurs substituants identiques ou différents et/ou condensé au benzène, ayant 1 à 3 hétéroatomes - notamment azote, oxygène et/ou soufre - identiques ou différents, en considérant dans chaque cas comme substituants des restes aryle et hétérocyclyle :
un halogène, un groupe cyano, nitro, amino, N-acétylamino, un groupe alkyle, alkoxy, alkylthio, alkylsulfinyle ou alkylsulfonyle étant chacun linéaire ou ramifié et ayant chacun 1 à 6 atomes de carbone, un groupe halogénalkyle, halogénalkoxy, halogénalkylthio, halogénalkylsulfinyle ou halogénalkylsulfonyle étant chacun linéaire ou ramifié et ayant chacun 1 à 6 atomes de carbone et 1 à 13 atomes d'halogènes identiques ou différents, un groupe alkoxycarbonyle ou alkoximinoalkyle étant chacun linéaire ou ramifié et ayant chacun 1 à 6 atomes de carbone dans les parties alkyle individuelles ainsi qu'un groupe phényle éventuellement substitué une ou plusieurs fois identiques ou différentes par un halogène et/ou un groupe alkyle ou alkoxy linéaire ou ramifié ayant chacun 1 à 6 atomes de carbone et/ou un groupe halogénalkyle ou halogénalkoxy linéaire ou ramifié ayant chacun 1 à 6 atomes de carbone et 1 à 13 d'halogènes identiques ou différents ;
R¹¹ représente de l'hydrogène ;
R¹¹ représente en outre un groupe alkyle linéaire ou ramifié ayant 1 à 14 atomes de carbone, portant le cas échéant un ou plusieurs substituants identiques ou différents, en considérant comme substituants :
un halogène - notamment fluor, chlore, brome et/ou iode - un groupe cyano, carboxyle, carbamoyle, un groupe alkoxy, alkoxyalkoxy, alkylthio, alkylsulfinyle, alkylsulfonyle, alkoxycarbonyle, N-alkylaminocarbonyle, N,N-dialkylaminocarbonyle ou alkylsulfonylaminocarbonyle étant chacun linéaire ou ramifié et ayant chacun 1 à 8 atomes de carbone dans les parties alkyle individuelles ou un reste hétérocyclyle, ce reste hétérocyclyle étant un hétérocycle pentagonal à heptagonal, saturé ou non saturé, éventuellement condensé au benzène, avec 1 à 3 hétéroatomes - notamment azote, oxygène et/ou soufre - identiques ou différents ;
R¹¹ représente en outre un groupe alcényle ou un groupe alcynyle ayant chacun 2 à 8 atomes de carbone et portant chacun le cas échéant un ou plusieurs substituants halogéno - notamment fluoro, chloro, bromo et/ou iodo - identiques ou différents ;
R¹¹ représente en outre un groupe cycloalkyle de 3 à 7 atomes de carbone portant le cas échéant un ou plusieurs substituants, identiques ou différents, halogéno - notamment fluoro, chloro, bromo et/ou iodo - et/ou alkyle linéaire ou ramifié ayant 1 à 4 atomes de carbone ;
R¹¹ représente en outre un groupe arylalkyle ou un groupe aryle portant chacun le cas échéant dans la partie aryle un ou plusieurs substituants identiques ou différents et ayant chacun 6 à 10 atomes de carbone dans la partie aryle et le cas échéant 1 à 4 atomes de carbone dans la partie alkyle linéaire ou ramifiée, en considérant dans chaque cas comme substituants de la partie aryle :
un halogène, un groupe cyano, nitro, amino, N-acétylamino, un groupe alkyle, alkoxy, alkylthio, alkylsulfinyle ou alkylsulfonyle étant chacun linéaire ou ramifié et ayant chacun 1 à 6 atomes de carbone, un groupe halogénalkyle, halogénalkoxy, halogénalkylthio, halogénalkylsulfinyle ou halogénalkylsulfonyle étant chacun linéaire ou ramifié et ayant chacun 1 à 6 atomes de carbone et 1 à 13 atomes d'halogènes identiques ou différents, un groupe alkoxycarbonyle ou alkoximinoalkyle étant chacun linéaire ou ramifié et ayant chacun 1 à 6 atomes de carbone dans les parties alkyle individuelles, ainsi qu'un groupe phényle portant le cas échéant un ou plusieurs substituants, identiques ou différents, halogéno et/ou alkyle ou alkoxy linéaire ou ramifié ayant chacun 1 à 6 atomes de carbone et/ou halogénalkyle ou halogénalkoxy linéaire ou ramifié ayant chacun 1 à 6 atomes de carbone et 1 à 13 atomes d'halogènes identiques ou différents.
